# EUROPEAN PATENT SPECIFICATION

(11) **EP 2 749 647 B1**
(45) Date of publication and mention of the grant of the patent: **04.07.2018**
(21) Application number: 12826199.7
(22) Date of filing: 17.02.2012
(51) Int. Cl.: C12N 15/09, C12N 7/00, C12Q 1/02, C12N 15/861

(54) **CONDITIONALLY REPLICATION-COMPETENT ADENOVIRUS**
BEDINGT REPLIKATIONSKOMPETENTES ADENOVIRUS
ADÉNOVIRUS APTE À SE RÉPLIQUER CONDITIONNELLEMENT

(30) Priority: 23.08.2011 JP 2011181414
(43) Date of publication of application: 02.07.2014
(73) Proprietor: National Institute of Biomedical Innovation, Osaka 567-0085 (JP)
(72) Inventor: MIZUGUCHI Hiroyuki, Ibaraki-shi Osaka 567-0085 (JP); SAKURAI Fuminori, Ibaraki-shi Osaka 567-0085 (JP)
(74) Representative: J A Kemp
(86) International application number: PCT/JP2012/053814
(87) International publication number: WO 2013/027427

(56) References cited:
- WO-A1-2005/121343
- WO-A1-2006/036004
- WO-A2-2006/026331
- JP-A- 2009 171 861
- US-A1- 2006 067 890
- K. SUGIO ET AL: "Enhanced Safety Profiles of the Telomerase-Specific Replication-Competent Adenovirus by Incorporation of Normal Cell-Specific microRNA-Targeted Sequences", CLINICAL CANCER RESEARCH, vol. 17, no. 9, 1 May 2011 (2011-05-01), pages 2807-2818, XP55006983, ISSN: 1078-0432, DOI: 10.1158/1078-0432.CCR-10-2008
- M. MARTTILA ET AL: "CD46 Is a Cellular Receptor for All Species B Adenoviruses except Types 3 and 7", JOURNAL OF VIROLOGY, vol. 79, no. 22, 27 October 2005 (2005-10-27), pages 14429-14436, XP55170385, ISSN: 0022-538X, DOI: 10.1128/JVI.79.22.14429-14436.2005
- SUGIO K. ET AL.: 'Enhanced safety profiles of the telomerase-specific replication-competent adenovirus by incorporation of normal cell-specific microRNA-targeted sequences' CLIN. CANCER RES. vol. 17, no. 9, February 2011, pages 2807 - 2818, XP055006983
- SUZUKI T. ET AL.: 'miR-122a-regulated expression of a suicide gene prevents hepatotoxicity without altering antitumor effects in suicide gene therapy' MOL. THER. vol. 16, no. 10, 2008, pages 1719 - 1726, XP055006986
- FUMINORI SAKURAI ET AL.: 'Development of recombinant viruses containing microRNA- regulated gene expression system' DRUG DELIV. SYS. vol. 24, no. 6, 2009, pages 572 - 581, XP055144917
- FUMINORI SAKURAI ET AL.: 'Development of recombinant adenovirus carrying microRNA- regulated gene expression system' JOURNAL OF THE PHARMACEUTICAL SOCIETY OF JAPAN vol. 130, no. 11, 2010, pages 1497 - 1504, XP055144918
- SAKURAI F. ET AL.: 'MicroRNA-regulated transgene expression systems for gene therapy and virotherapy' FRONT. BIOSCI. vol. 16, June 2011, pages 2389 - 2401, XP055144923
- MIZUGUCHI H. ET AL.: 'Adenovirus vectors containing chimeric type 5 and type 35 fiber proteins exhibit altered and expanded tropism and increase the size limit of foreign genes' GENE vol. 285, no. 1-2, 2002, pages 69 - 77, XP027353633
- YU L. ET AL.: 'Increased infectivity of adenovirus type 5 bearing type 11 or type 35 fibers to human esophageal and oral carcinoma cells' ONCOL. REP. vol. 14, no. 4, 2005, pages 831 - 835, XP003021236

## Description

### TECHNICAL FIELD

The present invention relates to a novel conditionally replicating adenovirus and a reagent comprising the same for cancer cell detection or for cancer diagnosis.

### BACKGROUND ART

Techniques currently used for cancer diagnosis mainly include (i) those using large-sized testing instruments (e.g., MRI) and (ii) those for measuring tumor markers or the like in blood, and expectations are now focused on (ii) which are simple techniques with less burden on patients. In particular, cancer cells circulating in the peripheral blood of cancer patients (i.e., circulating tumor cells (CTCs)) show a close relationship with clinical symptoms because these cells increase the risk of systemic metastasis and because the prognosis of patients with CTCs is significantly poor. Thus, it has been expected to develop a technique for simple and highly sensitive detection of CTCs as a predictive factor or surrogate marker for prognosis.

Techniques used for CTC detection include detection with a cancer-related antigen such as EpCAM (epithelial cell adhesion molecule) or cytokeratin-8 (e.g., CellSearch system) and detection by means of RT-PCR, etc. However, these cancer-related antigens are also expressed on normal epithelial cells and hence are highly likely to cause false positive detection, while cell morphology characteristic of cancer cells cannot be observed at the same time in the case of PCR detection. For these reasons, there has been a demand for a new technique in terms of sensitivity, simplicity, accuracy and costs.

On the other hand, the inventors of the present invention have already developed a conditionally replicating adenovirus which grows specifically in cancer cells and expresses GFP (GFP-expressing conditionally replicating adenovirus: GFP-CRAd) (which is referred to as TelomeScan®, OBP-401 or Telomelysin-GFP) (Patent Document 1: WO2006/036004). Moreover, the inventors of the present invention have also developed a simple technique for CTC detection using this TelomeScan (Non-patent Document 1: Kojima T., et al, J. Clin. Invest., 119; 3172, 2009).

However, since TelomeScan has the fiber protein of adenovirus type 5 and infects via coxsackievirus and adenovirus receptor (CAR) in target cells, TelomeScan may not infect cells which do not express CAR. In particular, it is known that CAR expression is reduced in highly malignant cancer cells which are highly invasive, metastatic and proliferative (Non-patent Document 2: Okegawa T., et al, Cancer Res., 61: 6592-6600, 2001); and hence TelomeScan may not detect these highly malignant cancer cells. Moreover, although less likely, TelomeScan may give false positive results by infecting and growing in normal blood cells (e.g., leukocytes) to cause GFP expression.

For these reasons, there has been a demand for a reagent for cancer cell detection and a reagent for cancer diagnosis, each of which detects almost all cancer cells including CAR-negative ones and does not give any false positive results in normal blood cells.

### Prior Art Documents

### Patent Documents

Patent Document 1: WO2006/036004

Non-patent Document 1: Kojima T., et al, J. Clin. Invest., 119: 3172, 2009
Non-patent Document 2: Okegawa T., et al, Cancer Res., 61: 6592-6600, 2001

### SUMMARY OF THE INVENTION

### PROBLEM TO BE SOLVED BY THE INVENTION

The present invention has been made under these circumstances, and the problem to be solved by the present invention is to provide a reagent for cancer cell detection and a reagent for cancer diagnosis, each of which detects almost all cancer cells including CAR-negative ones and does not give any false positive results in blood cells, as well as to provide a conditionally replicating recombinant adenovirus which is useful as such a reagent.

### MEANS TO SOLVE THE PROBLEM

As a result of extensive and intensive efforts made to solve the above problem, the inventors of the present invention have found that not only CAR-positive cells, but also CAR-negative cells can be detected when the fiber of adenovirus type 5 in TelomeScan is replaced with another adenovirus fiber binding to CD46, which is highly expressed on almost all human cells, particularly cancer cells in general. Moreover, the inventors of the present invention have succeeded in avoiding any false positive results in blood cells by integration of a microRNA (miRNA)-mediated gene regulatory system into TelomeScan, which led to the completion of the present invention.

Namely, the present invention is as follows.
(1) A recombinant adenovirus, which comprises a replication cassette comprising a polynucleotide, which comprises human telomerase reverse transcriptase promoter, E1A gene, IRES sequence and E1B gene in this order and which comprises a target sequence of a first microRNA wherein the replication cassette is integrated into the E1 region of the adenovirus genome and wherein the first microRNA is expressed in blood cells; a labelling cassette comprising a reporter gene, a promoter capable of regulating the expression of the gene and a target sequence of a second microRNA, wherein the labelling cassette is integrated into the E3 region of the adenovirus genome and the second microRNA is expressed in blood cells; and a gene encoding a CD46-binding fiber protein.
(2) The recombinant adenovirus according to (1), wherein the first microRNA is at least one selected from the group consisting of miR-142, miR-15, miR-16, miR-21, miR-126, miR-181, miR-223 and miR-296.
(3) The recombinant adenovirus according to (1) or (2), wherein the second microRNA is at least one selected from the group consisting of miR-142, miR-15, miR-16, miR-21, miR-126, miR-181, miR-223 and miR-296.
(4) The recombinant adenovirus according to any one of (1) to (3), wherein the reporter gene is a gene encoding a protein which emits fluorescence or a gene encoding an enzyme protein which generates a luminophore or a chromophore upon enzymatic reaction.
(5) The recombinant adenovirus according to any one of (1) to (4), wherein the promoter is human telomerase reverse transcriptase promoter or cytomegalovirus promoter.
(6) The recombinant adenovirus according to any one of (1) to (5), wherein the CD46-binding fiber protein comprises at least the fiber knob region in the fiber protein of adenovirus type 34 or 35.
(7) A reagent for cancer cell detection, which comprises the recombinant adenovirus according to any one of claim (1) to (6).
(8) The reagent according to (7), wherein the cancer cells are present in a biological sample taken from a subject, optionally wherein the biological sample is blood and optionally wherein the cancer cells are circulating tumor cells.
(9) The reagent according to (7) or (8), wherein:
   (a) the cancer cells are drug-resistant cancer cells; and/or
   (b) the cancer cells are cancer stem cells; and/or
   (c) the cancer cells are cancer cells having undergone epithelial-mesenchymal transition or mesenchymal-epithelial transition.
(10) A reagent for cancer diagnosis, which comprises the recombinant adenovirus according to any one of (1) to (6).
(11) A method for cancer cell detection, which comprises contacting cancer cells with the recombinant adenovirus according to (4) and detecting the fluorescence or color produced by the cancer cells.
(12) The method according to (11), wherein the cancer cells are present in a biological sample taken from a subject.
(13) The method according to (12), wherein the biological sample is blood.
(14) The method according to (13), wherein the cancer cells in the blood sample are circulating tumor cells.
(15) The method according to any one of (11) to (14), wherein:
   (a) the cancer cells are drug-resistant cancer cells; and/or
   (b) the cancer cells are cancer stem cells; and/or
   (c) the cancer cells are cancer cells having undergone epithelial-mesenchymal transition or mesenchymal-epithelial transition.

### EFFECTS OF THE INVENTION

The present invention enables simple and highly sensitive detection of CAR-negative cancer cells without detection of normal blood cells (e.g., leukocytes).

### BRIEF DESCRIPTION OF THE DRAWINGS

Figure 1 is a schematic view showing an example of the structure of the recombinant adenovirus of the present invention.
Figure 2 shows the results measured for activity of recombinant adenoviruses by flow cytometry.
Figure 3 shows the results detected for H1299 cells contained in blood samples.
Figure 4 shows the results detected for A549 cells contained in blood samples.
Figure 5 shows the results measured for activity of the recombinant adenovirus of the present invention in various types of cancer cells.
Figure 6 shows the results detected for cancer cells having undergone epithelial-mesenchymal transition (EMT).
Figure 7 shows the results detected for cancer stem cells.
Figure 8 shows the results detected for HI299 and T24 cells contained in blood samples by using a red fluorescent protein.

### DESCRIPTION OF EMBODIMENTS

### 1. Summary

TelomeScan (i.e., a conditionally replicating adenovirus comprising hTERT promoter, E1A gene, IRES sequence and E1B gene integrated in this order into the E1-deficient region of adenovirus type 5 and comprising cytomegalovirus (CMV) promoter and GFP integrated in this order into the E3-deficient region of adenovirus type 5), which has been previously developed by the inventors of the present invention, has problems in that: (i) TelomeScan may not detect highly malignant cancer cells where CAR expression is reduced; and (ii) TelomeScan may detect normal blood cells as false positive. As a result of extensive and intensive efforts made to solve these problems, the inventors of the present invention have found that highly malignant CAR-negative cancer cells can be detected when the fiber of adenovirus type 5 in TelomeScan is replaced with another adenovirus fiber binding to CD46, which is highly expressed on almost all human cells, particularly cancer cells in general. Moreover, the inventors of the present invention have also found that when a target sequence of miR-142-3p, which is miRNA, is integrated into each of the replication and labeling cassettes in TelomeScan, virus growth and labeling protein expression can be prevented in normal blood cells to thereby prevent the occurrence of false positive results in normal blood cells.

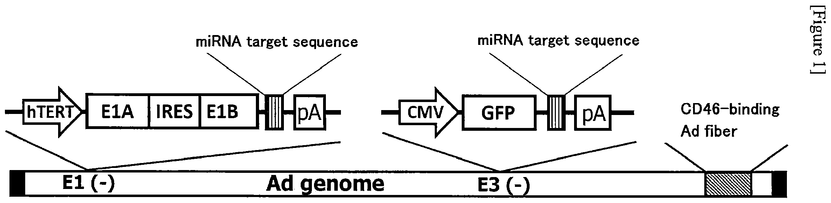

The recombinant adenovirus of the present invention is a recombinant adenovirus, in which a replication cassette comprising hTERT promoter, E1A gene, IRES sequence, E1B gene and a target sequence of microRNA is integrated into the E1 region of the adenovirus genome and a labeling cassette comprising a reporter gene, a promoter capable of regulating the expression of the gene and a target sequence of microRNA is integrated into the E3 region of the adenovirus genome, and which comprises a gene encoding a CD46-binding adenovirus fiber protein (Figure 1). This recombinant adenovirus has the following features.
(i) Because of comprising a gene encoding a CD46-binding adenovirus fiber protein, this recombinant adenovirus is able to infect almost all cells including CAR-negative cells.
(ii) Because of comprising hTERT promoter, this recombinant adenovirus grows specifically in hTERT-expressing cancer cells and also increases reporter gene expression upon growth, whereby the production of a labeling protein, a chromophore or the like can be increased to detectable levels.
(iii) Because of comprising a target sequence of miRNA, this recombinant adenovirus can prevent the occurrence of false positive results even when the virus infects normal cells having hTERT promoter activity, because expression of this miRNA prevents not only growth of the virus but also expression of the reporter gene. In particular, because of comprising a target sequence of miRNA which is expressed specifically in blood cells, this recombinant adenovirus can prevent the occurrence of false positive results even when the virus infects normal blood cells having hTERT promoter activity, because expression of this miRNA prevents not only growth of the virus in blood cells but also expression of the reporter gene.

The present invention has been completed on the basis of these findings.

### 2. Recombinant adenovirus

### (1) Replication cassette

The present invention relates to a polynucleotide, which comprises human telomerase reverse transcriptase (hTERT) promoter, E1A gene, IRES sequence and E1B gene in this order and which comprises a target sequence of microRNA. In addition, the present invention relates to a recombinant adenovirus, which comprises a replication cassette comprising the above polynucleotide, wherein the replication cassette is integrated into the E1 region of the adenovirus genome.

By the action of the above polynucleotide (or a replication cassette comprising the same), the recombinant adenovirus of the present invention can grow specifically in cancer cells and can also be prevented from growing in cells which express the desired miRNA. For example, since the target sequence of miRNA contained in the replication cassette of the present invention is a target sequence of miRNA which is expressed specifically in blood cells, the recombinant adenovirus of the present invention grows specifically in hTERT-expressing cancer cells and is prevented from growing in blood cells.

Human telomerase reverse transcriptase (hTERT) promoter is a promoter for reverse transcriptase which is an element of human telomerase. Although human telomerase activity will be increased by splicing of hTERT mRNA, post-translational modification of hTERT protein and other events, enhanced hTERT gene expression, i.e., increased hTERT promoter activity is thought to be the most important molecular mechanism. Human telomerase has been confirmed to show increased activity in 85% or more of human cancers, whereas it shows no activity in most normal cells. Thus, the use of hTERT promoter allows a gene downstream thereof to be expressed specifically in cancer cells. In the present invention, the hTERT promoter is located upstream of E1A gene, IRES sequence and E1B gene, whereby the virus can grow specifically in hTERT-expressing cancer cells.

hTERT has been confirmed to have many transcription factor binding sequences in a 1.4 kbp region upstream of its 5'-terminal end, and this region is regarded as hTERT promoter. In particular, a 181 bp sequence upstream of the translation initiation site is a core region important for expression of its downstream genes. In the present invention, although any sequence may be used as long as it includes this core region, an upstream sequence of approximately 378 bp which covers this core region in its entirety is preferred for use as the hTERT promoter. This sequence of approximately 378 bp has been confirmed to have the same efficiency of gene expression as the 181 bp core region alone. The nucleotide sequence of a 455 bp long hTERT promoter is shown in SEQ ID NO: 1.

In addition to the sequence shown in SEQ ID NO: 1, the nucleotide sequence of hTERT promoter includes the nucleotide sequences of polynucleotides which are hybridizable under stringent conditions with DNA consisting of a nucleotide sequence complementary to DNA consisting of SEQ ID NO: 1 and which have hTERT promoter activity. Such polynucleotides may be obtained from cDNA and genomic libraries by known hybridization techniques (e.g., colony hybridization, plaque hybridization, Southern blotting) using a polynucleotide which consists of the nucleotide sequence shown in SEQ ID NO: 1 or a fragment thereof as a probe.

For preparation of cDNA libraries, reference may be made to "Molecular Cloning, A Laboratory Manual 2nd ed." (Cold Spring Harbor Press (1989)). Alternatively, commercially available cDNA and genomic libraries may also be used for this purpose.

Stringent conditions in the above hybridization include, for example, conditions of 1 × SSC to 2 × SSC, 0.1% to 0.5% SDS and 42°C to 68°C, more specifically prehybridization at 60°C to 68°C for 30 minutes or longer and the subsequent 4 to 6 washings in 2 × SSC, 0.1% SDS at room temperature for 5 to 15 minutes.

As to detailed procedures for hybridization, reference may be made to "Molecular Cloning, A Laboratory Manual 2nd ed." (Cold Spring Harbor Press (1989); particularly Section 9.47-9.58), etc.

E1A and E1B genes are both included in the E1 gene of adenovirus. This E1 gene refers to one of the early genes among the virus early (E) and late (L) genes related to DNA replication, and it encodes a protein related to the regulation of viral genome transcription. E1A protein encoded by the E1A gene of adenovirus activates the transcription of a group of genes (e.g., E1B, E2, E4) required for infectious virus production. E1B protein encoded by the E1B gene of adenovirus assists late gene (L gene) mRNAs to accumulate into the cytoplasm of infected host cells and inhibits protein synthesis in the host cells, thereby facilitating virus replication. The nucleotide sequences of the E1A and E1B genes are shown in SEQ ID NO: 2 and SEQ ID NO: 3, respectively. In addition to the sequences shown in SEQ ID NO: 2 and SEQ ID NO: 3, the nucleotide sequences of the E1A and E1B genes include nucleotide sequences which are hybridizable under stringent conditions with DNA consisting of a nucleotide sequence complementary to DNA consisting of SEQ ID NO: 2 or SEQ ID NO: 3 and which encode a protein having E1A or E1B activity. Procedures and stringent conditions for hybridization are the same as those described above for the hTERT promoter.

IRES (internal ribosome entry site) sequence is a protein synthesis initiation signal specific to the picornavirus family and is considered to serve as a ribosomal binding site because of having a sequence complementary to the 3'-terminal end of 18S ribosomal RNA. It is known that translation of mRNAs derived from viruses of the picornavirus family is mediated by this sequence. The efficiency of translation from the IRES sequence is high and protein synthesis occurs even from the middle of mRNA in a manner not dependent on the cap structure. Thus, in the virus of the present invention, the E1A gene and the E1B gene, which is located downstream of the IRES sequence, are both translated independently by the action of hTERT promoter. With the use of the IRES sequence, hTERT promoter-mediated expression regulation occurs independently in both the E1A gene and the E1B gene, and hence virus growth can be more strictly limited to cells having telomerase activity when compared to the case where any one of the E1A gene or the E1B gene is regulated by the hTERT promoter. Moreover, the IRES sequence inserted between the E1A gene and the E1B gene can increase the growth capacity of the virus in host cells. The nucleotide sequence of the IRES sequence is shown in SEQ ID NO: 4. In addition to the sequence shown in SEQ ID NO: 4, the nucleotide sequence of the IRES sequence includes nucleotide sequences which are hybridizable under stringent conditions with DNA consisting of a nucleotide sequence complementary to DNA consisting of SEQ ID NO: 4 and which encode a protein having IRES activity. Procedures and stringent conditions for hybridization are the same as those described above for the hTERT promoter.

miRNA generally refers to short single-stranded RNA of approximately 15 to 25 nucleotides and is considered to regulate the translation of various genes upon binding to its target sequence present in mRNA. Thus, for example, when miRNA-expressing cells are infected with a recombinant adenovirus comprising a desired gene and a target sequence of the miRNA, the desired gene is prevented from being expressed in these cells. Such a target sequence of miRNA may be inserted into any site as long as a desired gene is prevented from being expressed, but it preferably inserted into an untranslated region of the desired gene, more preferably downstream of the desired gene.

The target sequence of miRNA to be used in the present invention includes target sequences of miRNAs which are expressed in non-cancer cells. Non-cancer cells are intended to mean cells that are not malignant tumor cells, and examples include normal cells, benign tumor cells and so on. Normal cells include, for example, normal blood cells, normal endothelial cells, normal fibroblasts, normal stem cells and so on. On the other hand, circulating tumor cells are regarded as cells originating from malignant tumors, and hence they fall within malignant tumor cells in the present invention.

The target sequence of miRNA to be used in the present invention are target sequences of miRNAs which are expressed specifically in blood cells. In the present invention, "blood cells" may include not only normal blood cells, but also cancerous blood cells. Namely, in the present invention, "miRNA which is expressed specifically in blood cells" may be expressed specifically in normal blood cells or may be expressed specifically in both normal blood cells and cancerous blood cells. Even when expressed specifically in both normal blood cells and cancerous blood cells, miRNA can also reduce false positive cases of normal blood cells during detection of circulating tumor cells and thereby ensures accurate detection of circulating tumor cells released from solid cancers. In the present invention, "miRNA which is expressed specifically in blood cells" is more preferably miRNA which is expressed in normal blood cells but is not expressed in cancerous blood cells.

In the present invention, blood cells include leukocytes (i.e., neutrophils, eosinophils, basophils, lymphocytes (T cells and B cells), monocytes, dendritic cells), CD34-positive cells, hematopoietic cells, hematopoietic stem cells, hematopoietic progenitor cells, peripheral blood mononuclear cells (PBMCs) and so on. Likewise, cancerous blood cells include leukemia cells, lymphoma cells and so on. In the present invention, being "expressed specifically" in certain cells is intended to mean not only that expression is limited only to the intended cells, but also that expression levels are higher in the intended cells than in other cells. For example, being "expressed specifically in blood cells" is intended to mean not only that expression is limited only to blood cells, but also that expression levels are higher in blood cells than in any cells other than blood cells.
miRNA which is expressed specifically in blood cells includes, for example, miR-142, miR-15, miR-16, miR-21, miR-126, miR-181, miR-223, miR-296 and so on, with miR-142, miR-15 and miR-16 being preferred.

Although miRNA is single-stranded RNA, it is possible to use a target sequence of either strand of premature double-stranded RNA as long as a desired gene can be prevented from being expressed. For example, there are miR-142-3p and miR-142-5p for miR-142, and a target sequence of either miRNA may be used in the present invention. Namely, in the present invention, "miR-142" includes both miR-142-3p and miR-142-5p, with miR-142-3p being preferred. Likewise, in the present invention, "miR-15" includes the sense strand (referred to as "miR-15S") and antisense strand (referred to as "miR-15 AS") of premature double-stranded RNA. The same applies to other miRNAs.
miR-142-3p gene is located at a site where translocation occurs in B cell leukemia (aggressive B cell leukemia), and is known to be expressed in hematopoietic tissues (e.g., bone marrow, spleen, thymus), but not expressed in other tissues. Moreover, miR-142-3p has been observed to be expressed in mouse fetal liver (fetal hematopoietic tissue) and hence is considered to be involved in differentiation of the hematopoietic system (Chang-Zheng Chen, et al.. Science, 2004).

In this embodiment, gene expression is regulated in two stages in a selective manner, because specific gene expression is caused in cancer cells by the action of hTERT promoter and gene expression in blood cells is regulated by the action of miRNA.

Also described herein is a target sequence of miRNA whose expression is suppressed in cancer cells. miRNA whose expression is suppressed in cancer cells includes, for example, miR-125, miR-143, miR-145, miR-199, let-7 and so on. In this case, specific gene expression in cancer cells is doubly regulated by the action of hTERT promoter and miRNA.

Although miRNA molecules have been initially found in nematodes, yeast and other organisms, there are currently found several hundreds of miRNAs in humans and mice. The sequences of these miRNAs are known, and sequence information and so on can be obtained by access to public DBs (e.g., miRBase sequence database (http://microma.sanger.ac.uk/sequences/index.shtml, http://www.mirbase.org/)).

The sequences of miR-142, miRNA-15, miRNA-16, miR-21, miR-126, miR-181, miR-223, miR-296, miR-125, miR-143, miR-145, miR-199 and let-7 are shown below.

| | |
|---|---|
| miR-142-3p | : 5'-UGUAGUGUUUCCUACUUUAUGGA (SEQ ID NO: 5) |
| miR-142-5p | : 5'-CAUAAAGUAGAAAGCACUACU (SEQ ID NO: 6) |
| miR-15S | : 5'-UAGCAGCACAUAAUGGUUUGUG (SEQ ID NO: 7) |
| miR-15AS | : 5'-CAGGCCAUAUUGUGCUGCCUCA (SEQ ID NO: 8) |
| miR-16S | : 5'-UAGCAGCACGUAAAUAUUGGCG (SEQ ID NO: 9) |
| miR-16AS | : 5'-CCAGUAUUAACUGUGCUGCUGA (SEQ ID NO: 10) |
| miR-21S | : 5'-UAGCUUAUCAGACUGAUGUUGA (SEQ ID NO: 11) |
| miR-21AS | : 5'-CAACACCAGUCGAUGGGCUGU (SEQ ID NO: 12) |
| miR-126S | : 5'-UCGUACCGUGAGUAAUAAUGCG (SEQ ID NO: 13) |
| miR-126AS | : 5'-CAUUAUUACUUUUGGUACGCG (SEQ ID NO: 14) |
| miR-181 | : 5'-AACAUUCAACGCUGUCGGUGAGU (SEQ ID NO: 15) |
| miR-223S | : 5'-UGUCAGUUUGUCAAAUACCCCA (SEQ ID NO: 16) |
| miR-223AS | : 5'-CGUGUAUUUGACAAGCUGAGUU (SEQ ID NO: 17) |
| miR-296-3p | : 5'-GAGGGUUGGGUGGAGGCUCUCC (SEQ ID NO: 18) |
| miR-296-5p | : 5'-AGGGCCCCCCCUCAAUCCUGU (SEQ ID NO: 19) |
| miR-125 | : 5'-UCCCUGAGACCCUUUAACCUGUGA (SEQ ID NO: 20) |
| miR-143S | : 5'-UGAGAUGAAGCACUGUAGCUC (SEQ ID NO: 21) |
| miR-143AS | : 5'-GGUGCAGUGCUGCAUCUCUGGU (SEQ ID NO: 22) |
| miR-145S | : 5'-GUCCAGUUUUCCCAGGAAUCCCU (SEQ ID NO: 23) |
| miR-145 AS | : 5'-GGAUUCCUGGAAAUACUGUUCU (SEQ ID NO: 24) |
| miR-199 | : 5'-CCCAGUGUUCAGACUACCUGUUC (SEQ ID NO: 25) |
| let-7 | : 5'-UGAGGUAGUAGGUUGUAUAGUU (SEQ ID NO: 26) |

In the present invention, a single unit of a target sequence of miRNA is composed of a sequence complementary to the whole or part of the miRNA, and has a nucleotide length of 7 to 30 nucleotides, preferably 19 to 25 nucleotides, more preferably 21 to 23 nucleotides. In the present invention, a single unit of a target sequence of miRNA is intended to mean a nucleotide sequence having the minimum length required for serving as a target of certain miRNA. More specifically, it is intended to mean an oligonucleotide of at least 7 nucleotides in length selected from complementary sequences of the nucleotide sequences shown in SEQ ID NOs: 5 to 19, and such an oligonucleotide may comprise substitution, deletion, addition or removal of one or several nucleotides at any site(s).

The target sequence as a whole to be integrated into the polynucleotide or recombinant adenovirus of the present invention may comprise several copies of a single unit of target sequence in order to ensure effective interaction between miRNA and the target sequence. The target sequence as a whole to be integrated into the recombinant adenovirus may be of any length as long as it can be integrated into the viral genome. For example, it may comprise 1 to 10 copies, preferably 2 to 6 copies, and more preferably 2 or 4 copies of a single unit of target sequence (John G. Doench, et al., Genes Dev. 2003 17:438-442). An oligonucleotide of appropriate length may be inserted between single units of target sequence contained in the target sequence as a whole. The length of such an oligonucleotide of appropriate length is not limited in any way as long as the target sequence as a whole can be integrated into the recombinant adenovirus genome. For example, such an oligonucleotide may be of 0 to 8 nucleotides in length. Moreover, in the case of comprising several units of a target sequence of miRNA, the target sequences in the respective units may be those toward the same miRNA or those toward different miRNAs. Furthermore, in the case of comprising target sequences toward the same miRNA, the target sequences in the respective units may have different lengths and/or different nucleotide sequences.

The target sequence of miRNA to be contained in the polynucleotide of the present invention (or a replication cassette comprising the same) can also be referred to as a "target sequence of a first microRNA" in order that the polynucleotide, when integrated into the recombinant adenovirus, should be distinguished from other miRNA target sequences present in the recombinant adenovirus.

When miR-142-3p is used as miRNA in the present invention, a target sequence thereof may be exemplified by sequences comprising the following sequences, by way of example.
(i) Sequence comprising two units of a target sequence of miR-142-3p:
   5'-gcggcctccataaagtaggaaacactacacagctccataaagtaggaaacactacattataagcggtac
   (SEQ ID NO: 27, each underline represents a single unit of a target sequence of miR-142-3p)
(ii) Sequence comprising four units of a target sequence of miR-142-3p: (SEQ ID NO: 28, each underline represents a single unit of a target sequence of miR-142-3p)

In the present invention, a target sequence of miRNA is placed downstream of the construct of hTERT promoter-E1A gene-IRES sequence-ElB gene, and the resulting polynucleotide comprising the hTERT promoter, the E1A gene, the IRES sequence, the E1B gene and the target sequence of miRNA in this order (which polynucleotide is referred to as a replication cassette) is integrated into the adenovirus genome, whereby E1 gene expression and virus growth can be prevented in cells expressing the miRNA.

In the present invention, a target sequence of miRNA is integrated downstream of the E1B gene or the reporter gene described later, whereby a gene located upstream thereof is prevented from being expressed. Although the details of this mechanism are not clear, a possible mechanism is as follows. First, miRNA-RISC (RNA-induced silencing complex) cleaves a target sequence on mRNA to thereby remove polyA from the mRNA. This would reduce the stability of the mRNA to cause degradation of the mRNA and hence prevention of gene expression. Alternatively, miRNA-RISC would recruit polyA ribonuclease, as in the case of normal miRNA, to cause polyA degradation, as a result of which the stability of mRNA would be reduced and gene expression would be prevented.

It should be noted that there are previous reports showing that the miRNA-induced inhibitory effect against gene expression was not obtained for the expression (translation) of a gene inserted downstream of the IRES sequence (Ramesh S. Pillai et al., Science 309, 1573(2005); Geraldine Mathonnet, et al., Science 317, 1764 (2007)). However, when the inventors of the present invention confirmed gene expression for the recombinant adenovirus of the present invention comprising hTERT promoter, E1A gene, IRES sequence, E1B gene and a target sequence of miRNA in this order, the miRNA was found to sufficiently prevent the expression of the E1B gene inserted downstream of the IRES sequence. This is a new finding in the present invention.

The genes to be contained in the replication cassette of the present invention can be obtained by standard genetic engineering techniques. For example, it is possible to use nucleic acid synthesis with a DNA synthesizer, which is commonly used as a genetic engineering technique. Alternatively, it is also possible to use PCR techniques in which gene sequences serving as templates are isolated or synthesized, and primers specific to each gene are then designed to amplify the gene sequence with a PCR system (Current Protocols in Molecular Biology, John Wiley & Sons (1987) Section 6.1-6.4) or gene amplification techniques using a cloning vector. The above techniques can be easily accomplished by those skilled in the art in accordance with Molecular cloning 2nd Edt. Cold Spring Harbor Laboratory Press (1989), etc. For purification of the resulting PCR product, known techniques can be used. If necessary, conventionally used sequencing techniques may be used to confirm whether the intended gene has been obtained, as expected. For example, dideoxynucleotide chain termination sequencing (Sanger et al. (1977) Proc. Natl. Acad. Sci. USA 74: 5463) or the like may be used for this purpose. Alternatively, an appropriate DNA sequencer (e.g., ABI PRISM (Applied Biosystems)) may also be used for sequence analysis.

In the present invention, the target sequence of miRNA can be obtained by being designed and synthesized such that each single unit of target sequence is complementary to the whole or part of the nucleotide sequence of the miRNA. For example, a target sequence of miR-142-3p can be obtained by synthesizing DNA such that it is complementary to the nucleotide sequence of miR-142-3p.

Then, the respective genes obtained as above are ligated in a given order. First, the above genes are each cleaved with known restriction enzymes or the like, and the cleaved DNA fragment of each gene is inserted into and ligated to a known vector in accordance with known procedures. As a known vector, pIRES vector may be used, by way of example. The pIRES vector comprises the IRES (internal ribosome entry site) sequence of encephalomyocarditis virus (ECMV) and is capable of translating two open reading frames (ORFs) from one mRNA. With the use of the pIRES vector, it is possible to prepare a "polynucleotide which comprises hTERT promoter, E1A gene, IRES sequence and E1B gene in this order and which comprises a target sequence of microRNA" by sequentially inserting the required genes into a multicloning site. Such a target sequence of miRNA may be inserted into any site, but it is preferably inserted downstream of the hTERT promoter-ElA-IRES-ElB construct. For DNA ligation, DNA ligase may be used. Alternatively, CMV promoter contained in a known vector (e.g., pShuttle) may be removed with known restriction enzymes and a sequence cleaved from the hTERT promoter-ElA-IRES-ElB-miRNA target sequence with appropriate restriction enzymes may then be inserted into this site, if necessary. Once the E1 gene required for adenovirus growth is allowed to be expressed under the control of the hTERT promoter, the virus can be grown specifically in cancer cells.

### (2) Labeling cassette

The present invention relates to a recombinant adenovirus in which the above replication cassette is integrated into the E1 region of the adenovirus genome and a labeling cassette is further integrated into the E3 region of the adenovirus genome. Such a labeling cassette comprises a reporter gene, a promoter capable of regulating the expression of the gene, and a target sequence of miRNA.

The adenovirus E3 region contains 11.6 kDa ADP (adenovirus death protein), and ADP has the function of promoting cell damage and virus diffusion. The recombinant adenovirus of the present invention is designed to eliminate any viral genome region like the E3 region containing ADP, which encodes a protein having the function of promoting cell damage and virus diffusion, so that the timing of cell death is delayed to facilitate identification of cancer tissues by production (emission, expression) of fluorescence (e.g., GFP). This is also effective in that circulating tumor cells (CTCs) described later can be detected alive over a long period of time.

The reporter gene to be contained in the labeling cassette in the recombinant adenovirus of the present invention is not limited in any way, and examples include a gene encoding a protein which emits fluorescence, a gene encoding an enzyme protein which generates a luminophore or a chromophore upon enzymatic reaction, a gene encoding an antibiotic, a gene encoding a tag-fused protein, a gene encoding a protein which is expressed on the cell surface and binds to a specific antibody, a gene encoding a membrane transport protein, and so on. Examples of a protein which emits fluorescence (i.e., a labeling protein) include a green fluorescent protein (GFP) derived from luminous jellyfish such as ***Aequorea victorea**,* its variants EGFP (enhanced-humanized GFP) and rsGFP (red-shift GFP), a yellow fluorescent protein (YFP), a cyan fluorescent protein (CFP), a blue fluorescent protein (BFP), GFP derived from ***Renilla reniformis*** and so on, and genes encoding these proteins can be used in the present invention. The above protein which emits fluorescence is preferably GFP or EGFP.

Likewise, examples of an enzyme protein which generates a luminophore or a chromophore upon enzymatic reaction include β-galactosidase, luciferase and so on. β-Galactosidase generates a blue chromophore from 5-bromo-4-chloro-3-indolyl-β-D-galactopyranoside (X-gal) upon enzymatic reaction. On the other hand, luciferase generates a luminophore upon enzymatic reaction with luciferin. Firefly luciferase, bacterial luciferase, Renilla luciferase and so on are known as members of luciferase, and those skilled in the art would be able to select an appropriate enzyme from known luciferase members.

Moreover, the promoter capable of regulating the expression of the above gene is not limited in any way as long as it is a suitable promoter compatible with the virus used for the expression of the above desired gene. Examples include CMV promoter, hTERT promoter, SV40 late promoter, MMTV LTR promoter, RSV LTR promoter, SRα promoter, β-actin promoter, PGK promoter, EF-la promoter and so on. Preferably, CMV promoter or hTERT promoter can be used for this purpose.

The target sequence of miRNA to be integrated into the labeling cassette may be either the same or different from the target sequence of miRNA to be integrated into the replication cassette.

In the present invention, the target sequence of miRNA is placed within the untranslated region of the reporter gene, preferably downstream of this gene, whereby the reporter gene can be prevented from being expressed. Namely, in the present invention, the labeling cassette preferably comprises a promoter capable of regulating the reporter gene, the reporter gene and the target sequence of microRNA in this order. The target sequence of miRNA to be integrated into the labeling cassette is referred to as a "target sequence of a second microRNA" in order that it should be distinguished from the target sequence of miRNA to be contained in the replication cassette. Other explanations on miRNA are the same as described above.

Details on how to obtain, purify and sequence the recombinant genes to be contained in the labeling cassette of the present invention are the same as described above for the replication cassette.

### (3) Cell death-inducing cassette

Also described herein is a recombinant adenovirus in which the above replication cassette is integrated into the E1 region of the adenovirus genome and a cell death-inducing cassette is integrated into the E3 region of the adenovirus genome. Such a cell death-inducing cassette comprises a gene encoding a cell death induction-related protein and a promoter capable of regulating the expression of the gene, and may further comprise a target sequence of microRNA.

The cell death-inducing cassette used in the recombinant adenovirus comprises a gene encoding a cell death induction-related protein and a promoter capable of regulating the expression of the gene. Thus, for example, when the recombinant adenovirus of the is infected into cancer cells, the virus grows specifically in the cancer cells to thereby increase the intracellular expression level of the cell death induction-related protein and induce cell death only in the cancer cells without damaging other normal cells.

Such a gene encoding a cell death induction-related protein is intended to mean a gene encoding a protein related to the induction of cell death in specific cells. Examples of a cell death induction-related protein include immunological proteins such as PA28. PA28 is a protein which activates intracellular proteasomes and which elicits immune reactions and also induces cell death when overexpressed. Moreover, TRAIL can also be exemplified as an apoptosis-inducing protein. TRAIL refers to a molecule which induces apoptotic cell death upon binding to its receptor on the cell surface.

Moreover, another example of the gene encoding a cell death induction-related protein is a tumor suppressor gene, which has the function of suppressing the growth of cancer cells. Examples of such a tumor suppressor gene include the following genes used in conventional gene therapy. SEQ ID NO (nucleotide sequence) and GenBank Accession No. are shown below for each gene.
p53 (SEQ ID NO: 29; Accession No. M14694): multiple types of cancer
pl5 (SEQ ID NO: 30; Accession No. L36844): multiple types of cancer
pl6 (SEQ ID NO: 31; Accession No. L27211): multiple types of cancer
APC (SEQ ID NO: 32; Accession No. M74088): colorectal cancer, gastric cancer, pancreatic cancer
BRCA-1 (SEQ ID NO: 33; Accession No. U14680): ovarian cancer, breast cancer
DPC-4 (SEQ ID NO: 34; Accession No. U44378): colorectal cancer, pancreatic cancer
FHIT (SEQ ID NO: 35; Accession No. NM 112012): gastric cancer, lung cancer, uterine cancer
p73 (SEQ ID NO: 36; Accession No. Y11416): neuroblastoma
PATCHED (SEQ ID NO: 37; Accession No. U59464): basal cell carcinoma
Rbp110 (SEQ ID NO: 38; Accession No. M15400): lung cancer, osteosarcoma
DCC (SEQ ID NO: 39; Accession No. X76132): colorectal cancer
NF1 (SEQ ID NO: 40; Accession No. NM 000267): neurofibroma type 1
NF2 (SEQ ID NO: 41; Accession No. L11353): neurofibroma type 2
WT-1 (SEQ ID NO: 42; Accession No. NM 000378): Wilms tumor

The target sequence of miRNA to be contained in the cell death-inducing cassette may be either the same or different from the target sequence of miRNA to be integrated into the replication cassette. The target sequence of miRNA may be placed within the untranslated region of the gene encoding a cell death induction-related protein, preferably downstream of this gene, whereby the cell death induction-related protein can be prevented from being expressed. Namely, the cell death-inducing cassette preferably comprises a promoter capable of regulating the gene encoding a cell death induction-related protein, the gene encoding a cell death induction-related protein and the target sequence of microRNA in this order. Other explanations on miRNA are the same as described above.

Details on how to obtain, purify and sequence the recombinant genes to be contained in the cell death-inducing cassette of the present invention are the same as described above for the replication cassette.

To determine whether or not cell death has been induced, morphological observation described below may be conducted for this purpose. Namely, once cells adhered onto the bottom surface of a culture vessel have been infected with the recombinant virus of the present invention and incubated for a given period, the cells will be rounded and detached from the bottom surface and then will float as shiny cells in the culture solution, as observed under an inverted microscope. At this stage, the cells have lost their vital mechanism and hence a determination can be made that cell death has been induced. Alternatively, cell death can also be confirmed with a commercially available kit for living cell assay which uses a tetrazolium salt (e.g., MTT, XTT).

### (4) CD46-binding fiber protein

The recombinant adenovirus of the present invention comprises a gene encoding a CD46-binding adenovirus fiber protein.

Adenovirus vectors which are now commonly used are prepared structurally based on adenovirus type 5 (or type 2) belonging to Subgroup C among 51 serotypes of human adenovirus. Although adenovirus type 5 is widely used because of its excellent gene transfer properties, adenovirus of this type has a problem of being difficult to infect cells with low expression of coxsackievirus and adenovirus receptor (CAR) because its infection is mediated by binding to CAR on target cells. In particular, CAR expression is reduced in highly malignant cancer cells which are highly invasive,
metastatic and proliferative, and hence an adenovirus having the fiber protein of adenovirus type 5 may not infect such highly malignant cancer cells.

In contrast, CD46 is expressed on almost all cells except for erythrocytes in humans and is also expressed on highly malignant cancer cells. Thus, a recombinant adenovirus comprising a gene encoding a CD46-binding adenovirus fiber protein can also infect CAR-negative and highly malignant cancer cells. For example, adenovirus types 34 and 35 bind to CD46 as their receptor and thereby infect cells (Marko Marttila, et al., J. Virol. 2005, 79(22):14429-36). As described above, CD46 is expressed on almost all cells except for erythrocytes in humans, and hence adenovirus types 34 and 35 are able to infect a wide range of cells including CAR-negative cells. Moreover, the fiber of adenovirus consists of a knob region, a shaft region and a tail region, and adenovirus infects cells through binding of its fiber knob region to the receptor. Thus, at least the fiber knob region in the fiber protein is replaced from adenovirus type 5 origin to adenovirus type 34 or 35 origin, whereby the virus will be able to infect CAR-negative cells via CD46.

Because of comprising a gene encoding a CD46-binding adenovirus fiber protein, the recombinant adenovirus of the present invention is able to infect almost all cells except for erythrocytes and thus able to infect highly malignant CAR-negative cancer cells which are highly invasive, metastatic and proliferative. In the present invention, "CAR-negative" cells are intended to mean cells where CAR expression is low or cells where CAR is not expressed at all.

57 serotypes have now been identified for human adenovirus, and these serotypes are classified into six groups, i.e., Groups A to F. Among them, adenovirus types belonging to Group B have been reported to bind to CD46. Adenovirus types belonging to Group B include adenovirus types 34 and 35, as well as adenovirus types 3, 7, 11, 16, 21 and 50, by way of example.

For use as a CD46-binding adenovirus fiber protein in the present invention, preferred is the fiber protein of adenovirus belonging to Group B, more preferred is the fiber protein of adenovirus type 3, 7, 34, 35, 11, 16, 21 or 50, and even more preferred is the fiber protein of adenovirus type 34 or 35.

The nucleotide sequence of a gene encoding the fiber protein of adenovirus type 34, 35, 3, 7, 11, 16, 21 or 50 is available from a known gene information database, e.g., the GenBank of NCBI (The National Center for Biotechnology Information). Moreover, in the present invention, the nucleotide sequence of a gene encoding the fiber protein of adenovirus type 34, 35, 3, 7, 11, 16, 21 or 50 includes not only the nucleotide sequence of each gene available from a database as described above, but also nucleotide sequences which are hybridizable under stringent conditions with DNA consisting of a nucleotide sequence complementary to DNA consisting of each nucleotide sequence available from a database and which encode a protein with binding activity to CD46.

The binding activity to CD46 can be evaluated when a recombinant adenovirus having DNA comprising the nucleotide sequence is measured for its infectivity to CD46-expressing cells. The infectivity of such a recombinant adenovirus may be measured in a known manner, for example, by detecting GFP expressed by the virus infected into CD46-expressing cells under a fluorescence microscope or by flow cytometry, etc. Procedures and stringent conditions for hybridization are the same as described above.

The recombinant adenovirus of the present invention may comprise the entire or partial region of a CD46-binding adenovirus fiber protein, such that at least the fiber knob region in the fiber protein binds to CD46. Namely, in the present invention, the CD46-binding adenovirus fiber protein may comprise at least the fiber knob region in the fiber protein of adenovirus belonging to Group B, more preferably at least the fiber knob region in the fiber protein of adenovirus of any type selected from the group consisting of type 34, type 35, type 3, type 7, type 11, type 16, type 21 and type 50, and even more preferably at least the fiber knob region in the fiber protein of adenovirus type 34 or 35. Moreover, the technical idea of the present invention is not limited to these fiber proteins as long as the intended protein binds to CD46, and it also covers various proteins capable of binding to CD46 as well as proteins having a motif capable of binding to CD46.

Alternatively, in the present invention, the CD46-binding fiber protein may comprise a region consisting of the fiber knob region and the fiber shaft region in the fiber protein of adenovirus belonging to Group B, more preferably a region consisting of the fiber knob region and the fiber shaft region in the fiber protein of adenovirus of any type selected from the group consisting of type 34, type 35, type 3, type 7, type 11, type 16, type 21 and type 50, and even more preferably a region consisting of the fiber knob region and the fiber shaft region in the fiber protein of adenovirus type 34 or 35.

In the present invention, the CD46-binding fiber protein may comprise the fiber shaft region or the fiber tail region in the fiber protein of adenovirus of any type (e.g., type 2, type 5) other than the above types, as long as it comprises at least the fiber knob region in the fiber protein of adenovirus belonging to Group B.

Examples of such a fiber protein include fiber proteins which comprise a region consisting of not only the fiber knob region and the fiber shaft region in the fiber protein of adenovirus of any type selected from the group consisting of type 34, type 35, type 3, type 7, type 11, type 16, type 21 and type 50, but also the fiber tail region in the fiber protein of adenovirus type 5.

The nucleotide sequences of a gene encoding the fiber knob region in the fiber protein of adenovirus type 34, a gene encoding the fiber shaft region in the fiber protein of adenovirus type 34 and a gene encoding a region consisting of the fiber knob region and the fiber shaft region in the fiber protein of adenovirus type 34 are shown in SEQ ID NOs: 47, 48 and 49, respectively.

Likewise, the nucleotide sequence of a gene encoding a region consisting of not only the fiber knob region and the fiber shaft region in the fiber protein of adenovirus type 34, but also the fiber tail region in the fiber protein of adenovirus type 5 is shown in SEQ ID NO: 50. In the present invention, the nucleotide sequence of such a gene includes not only the nucleotide sequence shown in SEQ ID NO: 50, but also nucleotide sequences which are hybridizable under stringent conditions with DNA consisting of a nucleotide sequence complementary to DNA consisting of the nucleotide sequence shown in SEQ ID NO: 50 and which encode a protein with binding activity to CD46. Procedures for evaluation of the binding activity to CD46, procedures and stringent conditions for hybridization are the same as described above.

To prepare the recombinant adenovirus, a polynucleotide comprising the replication cassette, the labeling cassette and/or the cell death-inducing cassette may be excised with appropriate restriction enzymes and inserted into an appropriate virus expression vector. A preferred virus expression vector is an adenovirus vector, more preferably an adenovirus type 5 vector, and particularly preferably an adenovirus type 5 vector which comprises a gene encoding a CD46-binding adenovirus fiber protein (e.g., the fiber protein of adenovirus type 34 or 35).

As shown in Example 2 described later, GFP expression in blood cells was sufficiently suppressed in both cases where a miRNA target sequence was inserted downstream of the replication cassette and where a miRNA target sequence was inserted downstream of the labeling cassette, whereas GFP expression in blood cells was unexpectedly significantly suppressed in a case where miRNA target sequences were simultaneously inserted downstream of the replication cassette and downstream of the labeling cassette, respectively. This is a new finding in the present invention.

In the present invention, the recombinant adenovirus may be obtained in the following manner, by way of example.

First, pHMCMVS (Mizuguchi H. et al., Human Gene Therapy, 10; 2013-2017, 1999) is treated with restriction enzymes and a target sequence of miRNA is inserted to prepare a vector having the target sequence of miRNA. Next, pSh-hAIB comprising a construct of hTERT promoter-ElA-IRES-ElB (WO2006/036004) is treated with restriction enzymes and the resulting fragment comprising the hTERT promoter-ElA-IRES-ElB construct is inserted into the above vector having the target sequence of miRNA to obtain a vector comprising hTERT promoter-ElA-IRES-ElB-miRNA target sequence. On the other hand, pHMCMVGFP-1 (pHMCMVS comprising EGFP gene) is treated with restriction enzymes to obtain a fragment comprising CMV promoter and EGFP gene, and this fragment is inserted into the above vector having the target sequence of miRNA to obtain a vector comprising a construct of CMV-EGFP-miRNA target sequence. Then, the vector comprising hTERT promoter-ElA-IRES-ElB-miRNA target sequence and the vector comprising CMV-EGFP-miRNA target sequence are each treated with restriction enzymes and ligated together to obtain a vector in which hTERT promoter-E1A-IRES-ElB-miRNA target sequence is integrated into the E1-deficient region of the adenovirus genome and CMV-EGFP-miRNA target sequence is integrated into the E3-deficient region of the adenovirus genome. Alternatively, when a vector comprising a gene encoding a CD46-binding adenovirus fiber protein is used as a vector to be inserted with the DNA fragments comprising the respective constructs, it is possible to obtain a vector in which hTERT promoter-E1A-IRES-E1B-miRNA target sequence is integrated into the E1-deficient region of the adenovirus genome and CMV-EGFP-miRNA target sequence is integrated into the E3-deficient region of the adenovirus genome and which comprises a gene encoding a CD46-binding adenovirus fiber protein. Moreover, this vector may be linearized with a known restriction enzyme and then transfected into cultured cells (e.g., 293 cells) to thereby prepare an infectious recombinant adenovirus. It should be noted that those skilled in the art would be able to easily prepare all viruses falling within the present invention by making minor modifications to the above preparation procedures.

### 3. Reagent for cancer cell detection or reagent for cancer diagnosis

As described above, the recombinant adenovirus of the present invention has the following features.
(i) This recombinant adenovirus infects almost all cells except for erythrocytes, and is also able to infect highly malignant CAR-negative cancer cells.
(ii) This recombinant adenovirus grows specifically in hTERT-expressing cancer cells and also increases the expression level of a reporter gene upon growth, whereby the production of a labeling protein, a chromophore or the like can be increased to detectable levels.
(iii) This recombinant adenovirus can prevent the occurrence of false positive results even when the virus infects normal cells having hTERT promoter activity, because miRNA expression prevents not only growth of the virus, but also expression of a reporter gene. In particular, because of comprising a target sequence of miRNA which is expressed specifically in blood cells, this recombinant adenovirus can prevent the occurrence of false positive results even when the virus infects normal blood cells having hTERT promoter activity, because expression of this miRNA prevents not only growth of the virus in blood cells but also expression of a reporter gene.

Thus, the recombinant adenovirus of the present invention can be used as a reagent for cancer cell detection or as a reagent for cancer diagnosis. In particular, because of having the above features, the recombinant virus of the present invention is extremely effective for detection of circulating tumor cells (CTCs) present in blood.

On the other hand, since 2004 when CTCs, which are cancer cells present in blood, were reported to serve as a prognostic factor for post-operative breast cancer patients in the New England Journal of Medicine (Cristofanilli M. et al., The New England Journal of Medicine, 2004, 781-791), CTCs have been measured as a biomarker in many clinical trials conducted in Europe and North America. Particularly in breast cancer, prostate cancer and skin cancer, CTCs have been proven to be an independent factor which determines the prognosis of these cancers. Moreover, in Europe, in the clinical trial in adjuvant setting of prostate cancer (SUCCESS), the number of CTCs counted is added to the inclusion criteria and only patients in whom one or more cells have been detected are included. This trial is a large-scale clinical trial including 2000 cases or more, and attention is being given to the results. Moreover, there is also a clinical trial in which an increase or decrease per se in CTCs is one of the clinical endpoints (MDV3100).

In recent years, the FDA in the United States has issued guidelines for approval and authorization of molecular-targeted anticancer agents, and hence the CTC test has become more important in cancer diagnosis. The guidelines issued by the FDA define that genetic changes in molecular targets in tumors should be tested before selection of molecular-targeted anticancer agents. When attempting to achieve the guidelines by conventional techniques, there arises a need for surgical biopsy from tumor tissues in patients to conduct genetic testing, which will impose a very strong burden on the patients. To solve this problem, efforts are now made to conduct genetic testing on CTCs collected from blood, and this strategy is referred to as "liquid biopsy" in contrast to the conventional "biopsy." Once this strategy has been achieved, genetic testing of tumor tissues can be conducted simply by blood collection and the burden on patients can be reduced greatly. For these reasons, the CTC test is receiving great attention as a highly useful testing technique in the clinical setting.

The CellSearch System of Veridex LLC is the only CTC detection device currently approved by the FDA, and most of the CTC detection methods used in clinical trials are accomplished by this CellSearch System. The CellSearch System is based on techniques to detect cancer cells with EpCAM antibody and cytokeratin antibody.

However, CTC detection techniques are designed to detect several to several tens of cells from among a billion of blood cells, and it is therefore very difficult to improve their sensitivity and accuracy. Thus, some problems are also pointed out in CTC detection methods based on the CellSearch System. For example, it is pointed out that cancer cells which are negative in the CTC test based on the CellSearch System are detected as being positive in another test, and that there are great differences in sensitivity and accuracy, depending on the cancer type (Allard W.J. et al., Clinical Cancer Research, 2004, 6897-6904). Moreover, the CellSearch System is also pointed out to have a problem of low CTC detection rate for lung cancer in the clinical setting (ibid).

Likewise, the CellSearch System is also pointed out to have a problem of reduced CTC detection rate because the expression of cell surface antigens including EpCAM is reduced in cancer cells having undergone epithelial-mesenchymal transition (EMT) (Anieta M. et.al., J Natl Cancer Inst, 101, 2009, 61-66, Janice Lu et.al., Int J Cancer, 126(3), 2010, 669-683).

Further, to conduct the above "liquid biopsy," additional steps are required for concentration and phenotyping or genotyping of CTCs, which require more sensitive and more accurate CTC detection techniques than simply counting the number of CTCs.

In contrast to this, because of having the above features (i) to (iii), the recombinant adenovirus of the present invention allows simple, highly sensitive and highly accurate detection of CTCs in blood without detection of leukocytes and other normal blood cells. Further, the reagent of the present invention allows detection of CTCs alive, so that the source organ of the detected CTCs can be identified upon analyzing surface antigens or the like present on the cell surface of the CTCs. Thus, the recombinant adenovirus of the present invention is useful for CTC detection and cancer diagnosis.

Moreover, the recombinant adenovirus or reagent for cancer cell detection of the present invention can be used to detect cancer cells having undergone EMT or mesenchymal-epithelial transition (MET). EMT is a phenomenon in which cancer cells lose their properties as epithelium and acquire features as mesenchymal lineage cells tending to migrate into surrounding tissues, and EMT is also involved in invasion and/or metastasis of cancer cells. On the other hand, mesenchymal-epithelial transition (MET) is a phenomenon in which mesenchymally derived cells acquire features as epithelium. As described above, it is difficult to detect cancer cells having undergone EMT by known techniques including the CellSerch System. In contrast, the present invention allows detection of cancer cells having undergone EMT or MET. The recombinant adenovirus of the present invention is therefore useful for cancer cell detection and for cancer diagnosis.

Further, the recombinant adenovirus of the present invention can also be used to detect drug-resistant cancer cells. Drugs intended in the present invention are those used for cancer chemotherapy. Examples of such drugs include adriamycin, carboplatin, cisplatin, 5-fluorouracil, mitomycin, bleomycin, doxorubicin, daunorubicin, methotrexate, paclitaxel, docetaxel and actinomycin D, etc. Moreover, the recombinant virus of the present invention, can also be used to detect cancer stem cells. In the present invention, cancer stem cells refer to cells (stem cells) serving as the origin of cancer cells. Cancer stem cells also include those having drug resistance.

In the present invention, the type of cancer or tumor to be detected or diagnosed is not limited in any way, and cells of all cancer types can be used. Examples include solid cancers or blood tumors, more specifically brain tumor, cervical cancer, esophageal cancer, tongue cancer, lung cancer, breast cancer, pancreatic cancer, gastric cancer, small intestinal cancer, duodenal cancer, colorectal cancer, bladder cancer, kidney cancer, liver cancer, prostate cancer, uterine cancer, uterine cervical cancer, ovarian cancer, thyroid cancer, gallbladder cancer, pharyngeal cancer, sarcoma, melanoma, leukemia, lymphoma and multiple myeloma (MM). Most (85% or more) of the cancer cells derived from human tissues show increased telomerase activity, and the present invention allows detection of such telomerase-expressing cancer cells in general.

Moreover, in the present invention, CTCs are not limited in any way as long as they are cancer cells present in blood, and they include not only cancer cells released from solid cancers, but also blood tumor cells such as leukemia cells and lymphoma cells as mentioned above. However, in cases where CTCs are blood tumor cells, the miRNA target sequence contained in the adenovirus of the present invention is preferably a target sequence of miRNA which is expressed specifically in normal blood cells.

To prepare the reagent of the present invention, the recombinant adenovirus may be treated, e.g., by freezing for easy handling and then used directly or mixed with known pharmaceutically acceptable carriers (e.g., excipients, extenders, binders, lubricants) and/or known additives (including buffering agents, isotonizing agents, chelating agents, coloring agents, preservatives, aromatics, flavorings, sweeteners).

### 4. Method for cancer cell detection or method for cancer diagnosis

Furthermore, the recombinant adenovirus of the present invention can be used for cancer cell detection or cancer diagnosis by contacting the same with cancer cells and detecting the fluorescence or color produced by the cancer cells.

In the present invention, the term "contact(ing)" is intended to mean that cancer cells and the recombinant adenovirus of the present invention are allowed to exist in the same reaction system, for example, by adding the recombinant adenovirus of the present invention to a sample containing cancer cells, by mixing cancer cells with the recombinant adenovirus, by culturing cancer cells in the presence of the recombinant adenovirus, or by infecting the recombinant adenovirus into cancer cells. Moreover, in the present invention, "fluorescence or color" is not limited in any way as long as it is light or color produced from a protein expressed from a reporter gene, and examples include fluorescence emitted from a labeling protein (e.g., GFP), light emitted from a luminophore generated by luciferase-mediated enzymatic reaction, blue color produced from a chromophore generated by enzymatic reaction between β-galactosidase and X-gal, etc.

Cancer cells for use in the method for cancer cell detection or in the method for cancer diagnosis may be derived from a biological sample taken from a subject. Such a biological sample taken from a subject is not limited in any way as long as it is a tissue suspected to contain cancer cells, and examples include blood, tumor tissue, lymphoid tissue and so on. Alternatively, cancer cells may be circulating tumor cells (CTCs) in blood, and explanations on CTCs are the same as described above.

Cancer cell detection and cancer diagnosis using the reagent of the present invention may be accomplished as follows, by way of example.

In cases where the biological sample taken from a subject is blood, the blood sample is treated by addition of an erythrocyte lysis reagent to remove erythrocytes and the remaining cell suspension is mixed in a test tube with the reagent of the present invention at a given ratio (0.01 to 1000 MOI (multiplicity of infection), preferably 0.1 to 100 MOI, more preferably 1 to 10 MOI). The test tube is allowed to stand or rotated for culture at room temperature or 37°C for a given period of time (e.g., 4 to 96 hours, preferably 12 to 72 hours, more preferably 18 to 36 hours) to facilitate virus infection into cancer cells and virus growth. GFP fluorescence production in the cell fraction is quantitatively analyzed by flow cytometry. Alternatively, GFP-expressing cells are morphologically analyzed by being observed under a fluorescence microscope. This system allows highly sensitive detection of CTCs present in peripheral blood. This method can be used for detection of CTCs which are present in trace amounts in peripheral blood.

In cases where flow cytometry is used for CTC detection, CTCs may be detected by determining whether each cell is GFP-positive or GFP-negative, e.g., in accordance with the following criteria.

First, groups of cells in a sample which is not infected with any virus are analyzed to obtain a background fluorescence value. A threshold is set to the maximum fluorescence value. Subsequently, groups of cells in samples which have been infected with the virus of the present invention are analyzed and groups of cells in a sample showing a fluorescence value equal to or greater than the threshold are determined to be GFP-positive. In the case of using a blood sample taken from a subject, GFP-positive cells can be detected as CTCs. Further, these GFP-positive cells (CTCs) may be concentrated for phenotyping or genotyping.

In the present invention, examples of a subject include mammals such as humans, rabbits, guinea pigs, rats, mice, hamsters, cats, dogs, goats, pigs, sheep, cows, horses, monkeys and so on.

The amount of the reagent of the present invention to be used is selected as appropriate, depending on the state and amount of a biological sample to be used for detection and the type of detection method to be used, etc. For example, in the case of a blood sample, the reagent of the present invention can be used in an amount ranging from about 0.01 to 1000 MOI, preferably 0.1 to 100 MOI, and more preferably 1 to 10 MOI per 1 to 50 ml, preferably 3 to 25 ml, and more preferably 5 to 15 ml of the blood sample. MOI refers to the ratio between the amount of virus (infectious unit) and the number of cells when a given amount of cultured cells are infected with a given amount of virus particles, and is used as an index when viruses are infected into cells.

To infect the recombinant virus into cells, the following procedures may be used for this purpose. First, cells are seeded in a culture plate containing an appropriate culture medium and cultured at 37°C in the presence of carbon dioxide gas. The culture medium is selected from DMEM, MEM, RPMI-1640 and others commonly used for animal cell culture, and may be supplemented with serum, antibiotics, vitamins and so on, if necessary. The cultured cells are inoculated with a given amount of the virus, for example, at 0.1 to 10 MOI.

For confirmation of virus growth, the virus-infected cells are collected and treated to extract their DNA, followed by real-time PCR with primers targeting an appropriate gene possessed by the virus of the present invention, whereby virus growth can be quantitatively analyzed.

In cases where GFP gene is used as a reporter gene, labeled cells may be detected as follows: cells showing virus growth will emit a given fluorescence (e.g., a green fluorescence for GFP) upon irradiation with an excitation light, so that cancer cells can be visualized by the fluorescence. For example, when the virus-infected cells are observed under a fluorescence microscope, GFP fluorescence production can be seen in the cells. Moreover, to observe the virus-infected cells over time, GFP fluorescence production can be monitored over time with a CCD camera.

Moreover, the reagent of the present invention also allows real-time detection of cancer cells present *in vivo.* To label and detect cells *in vivo* in a real-time manner, the recombinant adenovirus of the present invention may be administered *in vivo.*

The reagent of the present invention may be applied directly to the affected area or may be introduced *in vivo* (into target cells or organs) in any known manner, e.g., by injection into vein, muscle, peritoneal cavity or subcutaneous tissue, inhalation from nasal cavity, oral cavity or lungs, oral administration, catheter-mediated intravascular administration and so on, as preferably exemplified by local injection into muscle, peritoneal cavity or elsewhere, injection into vein, etc.

When the reagent of the present invention is administered to a subject, the dose may be selected as appropriate, depending on the type of active ingredient, the route of administration, a target to be administered, the age, body weight, sex and/or symptoms of a patient, and other conditions. As a daily dose, the amount of the virus of the present invention serving as an active ingredient may usually be set to around 10⁶ to 10¹¹ PFU (plaque forming units), preferably around 10⁹ to 10¹¹ PFU, given once a day or in divided doses.

Real-time *in vivo* monitoring of fluorescence from cancer cells has the advantage of being used for *in vivo* diagnostic agents. This is useful for so-called navigation surgery and so on. Details on navigation surgery can be found in WO2006/036004.

Further, the reagent of the present invention is useful for detection of CTCs as a biomarker, and hence the reagent of the present invention can be used to determine prognosis.

For example, in cases where GFP is used as a labeling protein in the virus of the present invention, a biological sample taken from a cancer patient before being treated by any cancer therapy (e.g., chemotherapy, radiation therapy, surgical operation) and a biological sample taken at a time point after a certain period (e.g., 1 to 90 days) has passed from the treatment are each infected with the virus of the present invention. Next, GFP-positive cells contained in the sample taken before the treatment and GFP-positive cells contained in the sample taken at a certain time point after the treatment are compared for their number under the same conditions. As a result, if the number of GFP-positive cells after the treatment becomes smaller than the number of GFP-positive cells before the treatment, a determination can be made that prognosis has been improved.

The present invention will be further described in more detail by way of the following illustrative examples, which are not intended to limit the scope of the invention.

### Example 1

### Preparation of Ad34 fiber 142-3pT

### (1) Preparation of pHMCMV5-miR-142-3pT

pHMCMV5 (Mizuguchi H. et al., Human Gene Therapy, 10; 2013-2017, 1999) was treated with NotI/KpnI and the resulting fragment was ligated to a double-stranded oligo, which had been prepared by annealing the following synthetic oligo DNAs, to thereby prepare pHMCMV5-miR-142-3pT(pre).
miR-142-3pT-S1: (SEQ ID NO: 43, each underline represents a miR-142-3p target sequence)
miR-142-3pT-AS1: (SEQ ID NO: 44, each underline represents a miR-142-3p target sequence)

Then, pHMCMV5-miR-142-3pT(pre) was treated with PacI/KpnI and the resulting fragment was ligated to a double-stranded oligo, which had been prepared by annealing the following synthetic oligo DNAs, to thereby obtain pHMCMV5-miR-142-3pT having 4 repeats of a miR-142-3p target sequence.
miR-142-3pT-S2: (SEQ ID NO: 45, each underline represents a miR-142-3p target sequence)
miR-142-3pT-AS2: (SEQ ID NO: 46, each underline represents a miR-142-3p target sequence)

### (2) Preparation of E1 shuttle plasmid pHM5-hAIB-miR-142-3pT

pSh-hAIB (WO2006/036004) was digested with I-CeuI/PmeI and the digested product was electrophoresed on an agarose gel. A band of approximately 4.5 kbp (hAIB cassette) was excised from the gel and treated with GENECLEAN II (Q-Biogene) to purify and collect a DNA fragment. The purified DNA fragment (hAIB cassette) was ligated to a fragment which had been obtained from pHMCMV5-miR-142-3pT by being digested with NheI, treated with Klenow Fragment and further digested with I-CeuI, thereby obtaining pHM5-hAIB-miR-142-3pT having hTERT promoter, E1A gene, IRES (internal ribosomal entry site) sequence, E1B gene and a miR-142-3pT target sequence.

### (3) Preparation of E3 shuttle plasmid pHM13CMV-EGFP-miR-142-3pT

pEGFP-N1 (Clontech) was digested with Apal and NotI, and the resulting digested product was inserted into the ApaI/NotI site of pHMCMV5 to obtain pHMCMVGFP-1. pHMCMVGFP-1 was digested with PmeI/HindIII, and the digested product was electrophoresed on an agarose gel. A band of approximately 750 bp (EGFP) was excised from the gel and treated with GENECLEAN II to purify and collect a DNA fragment. The purified DNA fragment (EGFP) was ligated to a fragment which had been obtained from pBluescriptII KS+ by being digested with HincII/HindIII, thereby preparing pBSKS-EGFP. pBSKS-EGFP was digested with ApaI/XbaI, and the digested product was electrophoresed on an agarose gel. A band of approximately 750 bp (EGFP) was excised from the gel and treated with GENECLEAN II to purify and collect a DNA fragment. The purified DNA fragment (EGFP) was ligated to a fragment which had been obtained from pHMCMV5-miR-142-3pT by being digested with ApaI/XbaI, thereby obtaining pHMCMV5-EGFP-miR-142-3pT. pHMCMV5-EGFP-miR-142-3pT was digested with BglII, and the digested product was electrophoresed on an agarose gel. A band of approximately 2 kbp (CMV-EGFP-miR-142-3pT) was excised from the gel and treated with GENECLEAN II to purify and collect a DNA fragment. The purified DNA fragment (CMV-EGFP-miR-142-3pT) was ligated to a fragment which had been obtained from pHM13 (Mizuguchi et al., Biotechniques, 30; 1112-1116, 2001) by being digested with BamHI and treated with CIP (Alkaline Phosphatase, Calf Intest), thereby obtaining pHM13CMV-EGFP-miR-142-3pT.

### (4) Preparation of pAdHM49-hAIB142-3pT-CG142-3pT

pAdHM49 (Mizuguchi et al, J. Controlled Release 110; 202-211, 2005) was treated with I-CeuI/PI-SceI and the resulting fragment was ligated to pHM5-hAIB-miR-142-3pT which had also been treated with I-CeuI/PI-SceI, thereby preparing pAdHM49-hAIB142-3pT in which hTERT promoter, E1A gene, IRES sequence, E1B gene and a miR-142-3pT target sequence were integrated into the E1-deficient region of the Ad vector. pAdHM49 is a recombinant adenovirus in which a region covering genes encoding the fiber knob and fiber shaft of the adenovirus type 5 fiber is replaced with a region covering genes encoding the fiber knob and fiber shaft of the adenovirus type 34 fiber, and hence pAdHM49 comprises the nucleotide sequence (SEQ ID NO: 49) of a gene encoding a region consisting of the fiber knob region and the fiber shaft region in the fiber protein of adenovirus type 34. The nucleotide sequence of a gene encoding the pAdHM49 fiber protein (i.e., the fiber knob region and fiber shaft region of the adenovirus type 34 fiber and the fiber tail region of the adenovirus type 5 fiber) is shown in SEQ ID NO: 50. In the nucleotide sequence shown in SEQ ID NO: 50, the nucleotide sequence of a gene encoding the fiber tail region of the adenovirus type 5 fiber is located at nucleotides 1 to 132, the nucleotide sequence of a gene encoding the fiber shaft region of the adenovirus type 34 fiber is located at nucleotides 133 to 402, and the nucleotide sequence of a gene encoding the fiber knob region of the adenovirus type 34 fiber is located at nucleotides 403 to 975. Namely, in the nucleotide sequence shown in SEQ ID NO: 50, the nucleotide sequence of a region derived from the adenovirus type 5 fiber is located at nucleotides 1 to 132, while the nucleotide sequence of a region derived from the adenovirus type 34 fiber is located at nucleotides 133 to 975.

Then, pAdHM49-hAIB142-3pT was digested with Csp45I and the resulting fragment was ligated to a fragment which had been obtained from pHM13CMV-EGFP-miR-142-3pT by being digested with ClaI, thereby obtaining pAdHM49-hAIB142-3pT-CG142-3pT in which hTERT promoter, E1A gene, IRES sequence, E1B gene and a miR-142-3pT target sequence were integrated into the E1-deficient region of the adenovirus vector and CMV promoter, EGFP and a miR-142-3pT target sequence were integrated into the E3-deficient region of the adenovirus vector, and which further comprised a gene encoding the fiber protein of adenovirus type 34.

### (5) Preparation ofAd34 fiber 142-3pT(El,E3)

pAdHM49-hAIB142-3pT-CG142-3pT was linearized by being cleaved with a restriction enzyme PacI whose recognition site was present at each end of the adenovirus genome therein, and the linearized product was transfected into 293 cells seeded in a 60 mm culture dish by using Lipofectamine 2000 (Invitrogen). After about 2 weeks, a recombinant adenovirus Ad34 fiber 142-3pT(El,E3) was obtained (Figure 1).

### Example 2

### Activity measurement of Ad34 fiber 142-3pT(E1,E3)

### (1) Cells

HeLa (derived from human uterine cancer cells) and LN319 (derived from human glioma cells) were used as CAR-positive cells, while LNZ308 (derived from human glioma cells), LN444 (derived from human glioma cells) and K562 (derived from human myelogenous leukemia cells) were used as CAR-negative cells. K562 cells are expressing miR-142-3p. DMEM (10% FCS, supplemented with antibiotics) was used for HeLa, LN319, LNZ308 and LN444 cells, while RPMI-1640 medium (10% FCS, supplemented with antibiotics) was used for K562 cells. These cells were cultured at 37°C under saturated vapor pressure in the presence of 5% CO₂.

### (2) Activity measurement of Ad34 fiber 142-3pT(E1,E3) by flow cytometry

Cells of each line were seeded in a 24-well plate at 5 × 10⁴ cells/500 ul/well and treated with Ad34 fiber 142-3pT(E1,E3) at an MOI of 10. As a control, TelomeScan (i.e., a conditionally replicating adenovirus comprising hTERT promoter, E1A gene, IRES sequence and E1B gene integrated in this order into the E1-deficient site of adenovirus type 5 and comprising CMV promoter and GFP integrated in this order into the E3-deficient site of adenovirus type 5) was used. After culture for 24 hours, the cells were collected and the number of GFP-positive cells was measured using a flow cytometer MACSQuant (Miltenyi Biotec).

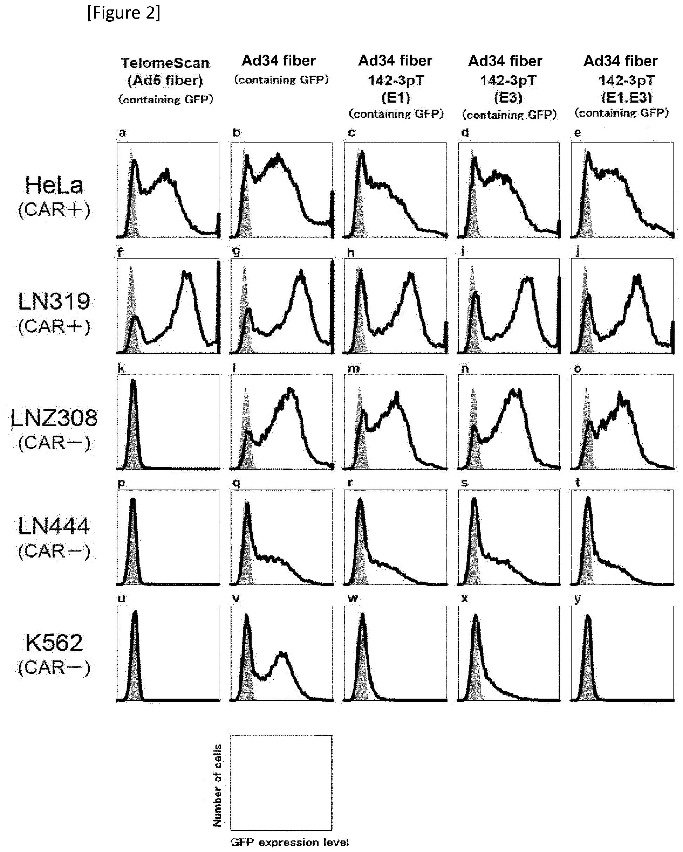

The results obtained are shown in Figure 2. In the specification and Figure 2, "TelomeScan (Ad5 fiber)" represents TelomeScan, while "Ad34 fiber" represents a recombinant adenovirus which comprises hTERT promoter, E1A gene, IRES sequence and E1B gene integrated in this order into the E1-deficient site of the adenovirus genome and also comprises CMV promoter and GFP integrated in this order into the E3-deficient site of the adenovirus genome and which comprises a gene encoding a fiber protein derived from adenovirus type 34. Likewise, "Ad34 fiber 142-3pT(El)" represents a recombinant adenovirus which further comprises a target sequence of miR-142-3p integrated into the E1-deficient region (downstream of the E1B gene) in the above Ad34 fiber, while "Ad34 fiber 142-3pT(E3)" represents a recombinant adenovirus which further comprises a target sequence of miR-142-3p integrated into the E3-deficient region (downstream of the GFP gene) in the above Ad34 fiber. Likewise, "Ad34 fiber 142-3pT(E1,E3)" represents a recombinant adenovirus which further comprises a target sequence of miR-142-3p integrated into each of the E1- and E3-deficient regions (downstream of the E1B gene and downstream of the GFP gene, respectively) in the above Ad34 fiber. Moreover, in Figure 2 and the subsequent figures, "(containing GFP)" is intended to mean that the GFP gene is inserted into each viral genome.

As a result of activity measurement, when LNZ308, LN444 and K562, which are CAR-negative cells, were infected with TelomeScan (Ad5 fiber), no GFP-positive cell was detected (Figure 2, panels k, p and u). In contrast, when these cells were infected with Ad34 fiber, GFP-positive cells were detected (85.5% positive in LNZ308, 58.4% positive in LN444, and 63.7% positive in K562) (panels 1, q and v).

This result indicated that the recombinant adenovirus of the present invention having a gene encoding the fiber protein of adenovirus type 34 allowed significant detection of CAR-negative cells.

Further, in the case of K562 cells which are CAR-negative and are expressing miR-142-3p, GFP-positive cells were 63.7% upon infection with Ad34 fiber (panel v), whereas GFP-positive cells were 12.2% upon infection with Ad34 fiber 142-3pT(E1) and 34.8% upon infection with Ad34 fiber 142-3pT(E3), and no GFP-positive cell was detected upon infection with Ad34 fiber 142-3pT(El,E3) (panels w, x and y). Namely, the detection rate of K562 cells was significantly reduced when using an adenovirus comprising a target sequence of miR-142-3p integrated into either the E1- or E3-deficient region of the adenovirus genome, and K562 cells were no longer detected when using an adenovirus comprising a target sequence of miR-142-3p integrated into each of the E1- and E3-deficient regions.

This result indicated that the recombinant virus of the present invention comprising a target sequence of miR-142-3p did not detect highly miR-142-3p-expressing cells, such as normal blood cells.

### Example 3

### Detection of cancer cells in blood samples using Ad34 fiber 142-3pT(E1,E3)

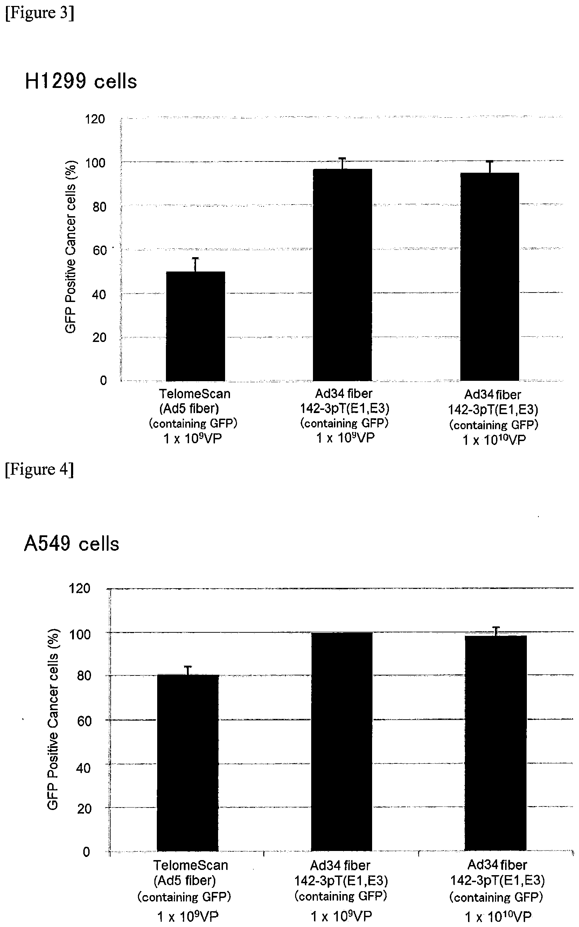

5 × 10⁴ H1299 cells (CAR-positive) were suspended in 5 mL blood and erythrocytes were lysed to collect PBMCs. To these PBMCs, a virus was added in an amount of 1 × 10⁹, 1 × 10¹⁰ or 1 × 10¹¹ VPs (virus particles) and infected at 37°C for 24 hours while rotating with a rotator. The cells were collected and immunostained with anti-CD45 antibody, and GFP-positive cells were observed under a fluorescence microscope. CD45 is known to be a surface antigen of blood cell lineage cells except for erythrocytes and platelets. "GFP Positive Cancer cells (%)" found in the vertical axis of Figures 3 and 4 represents the "number of GFP-positive and CD45-negative cells (%) among GFP-positive cells."

As a result, many false positive cells (GFP-positive and CD45-positive cells) were observed upon infection with TelomeScan (Ad5 fiber), whereas false positive cells were very few upon infection with Ad34 fiber 142-3pT(E1,E3), so that cancer cells were able to be specifically detected.

Moreover, as a result of quantitative analysis on the detection specificity of H1299 cells, many false positive cells were detected in the case of TelomeScan (Ad5 fiber) upon virus infection at 1 × 10⁹ VPs, whereas the detection specificity was 90% or higher and some samples showed 100% detection specificity in the case of Ad34 fiber 142-3pT(E1,E3) even when the amount of virus infection was increased (Figure 3). Likewise, quantitative analysis was also performed on A549 cells (CAR-positive cells) in the same manner, indicating that the detection specificity was 100% upon virus infection at 1 × 10⁹ VPs (Figure 4). These results indicated that the recombinant virus of the present invention allowed specific detection of cancer cells contained in the PBMC fraction.

In view of the foregoing, the detection reagent and diagnostic reagent of the present invention were demonstrated to allow detection of highly malignant CAR-negative cancer cells and, on the other hand, to ensure no false positive detection of highly miR-142-3p-expressing normal blood cells (e.g., leukocytes), etc.; and hence they were shown to be very effective for detection of circulating tumor cells (CTCs) in blood.

### Example 4

### Activity measurement of Ad34 fiber 142-3pT(E1,E3) in various human cancer cell lines

### (1) Cells

The cancer cells used in this example were human non-small cell lung cancer-derived H1299 cells, human lung cancer-derived A549 cells, human breast cancer-derived MCF7 cells, human breast cancer-derived MDA-MB-231 cells, human bladder cancer-derived KK47 cells, human gastric cancer-derived MKN45 cells, human colorectal cancer-derived SW620, human liver cancer-derived Huh7 cells, human pancreatic cancer-derived PancI cells, human glioma-derived LN319 cells, human bladder cancer-derived T24 cells, human glioma-derived LNZ308 cells, and human glioma-derived LN444 cells.

### (2) Activity measurement of Ad34 fiber 142-3pT(E1,E3) by flow cytometry

5 × 10⁴ cancer cells of each line were suspended in 500 µl medium, to which 100 µl of a conditionally replicating Ad suspension prepared at 5 × 10⁵ or 5 × 10⁶ pfu/ml was then added. The resulting mixture of the cells and the conditionally replicating Ad was seeded in a 24-well plate and cultured at 37°C for 24 hours. The cells were collected and centrifuged at 1500 rpm for 5 minutes. After removal of the medium, the cells were suspended in 300 µl of 2% FCS-containing PBS and measured for GFP-positive rate using a flow cytometer (MACS Quant Analyzer; Miltenyi Biotec). The data obtained were analyzed by FCS multi-color data analysis software (Flowjo).

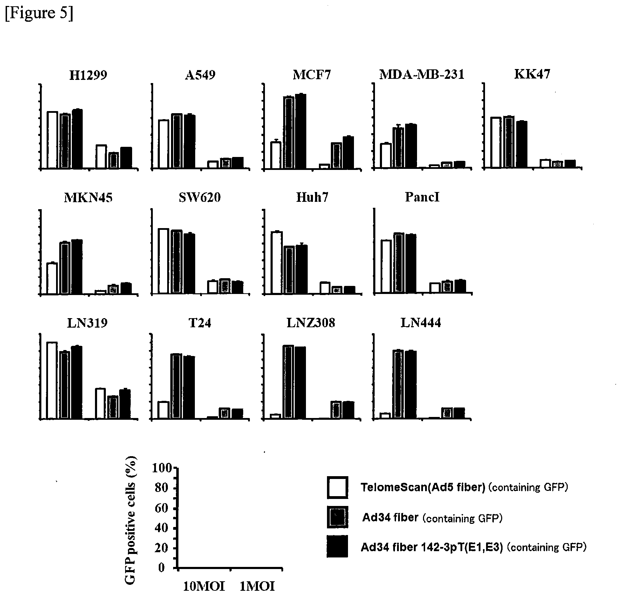

As a result, Ad34 fiber 142-3pT(E1,E3) was found to efficiently infect almost all cancer cells, and 60% or more of the cancer cells were GFP-positive. Particularly in the case of CAR-negative cells (T24, LNZ308, LN444), their GFP-positive rate was significantly improved when compared to conventionally used TelomeScan (Figure 5).

This result indicated that the recombinant virus of the present invention allowed efficient detection of not only CAR-positive cells but also CAR-negative cells.

### Example 5

### Detection of cancer cells having undergone epithelial-mesenchymal transition (EMT)

Human pancreatic cancer PancI cells were cultured for 6 days in the presence of 10 ng/mL recombinant TGF-β1 to thereby induce epithelial-mesenchymal transition (EMT). After induction of EMT, relative expression of mRNAs encoding E-cadherin, EpCAM, hTERT, N-cadherin, Slug and Snail was measured by real-time RT-PCR. In addition, CAR and CD46 expression in the Panc I cells was analyzed by flow cytometry. The virus of the present invention was infected into the cells in the same manner as shown in Example 4.

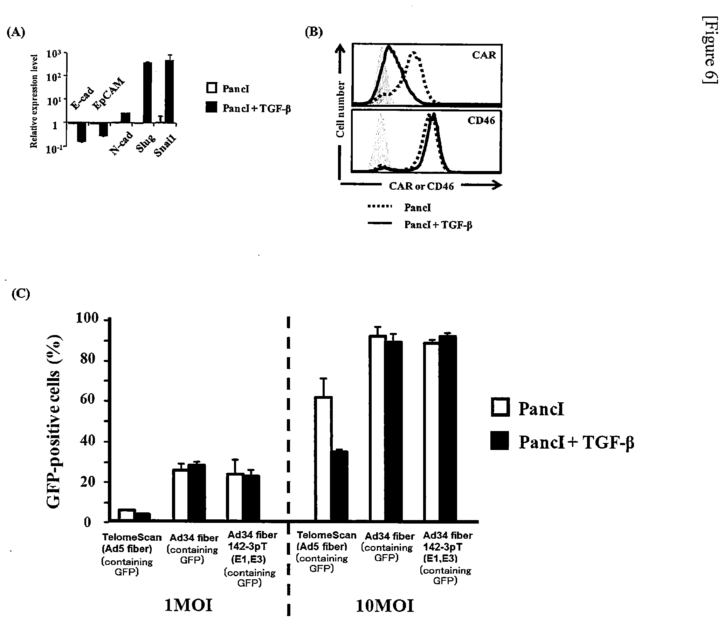

As a result, upon culture in a TGF-β-containing medium, the expression of EMT marker genes Slug, Snail and N-cadherin were increased, while the expression of epithelial markers E-cadherin and EpCAM was reduced, thus indicating that EMT has been induced (Figure 6A). Moreover, upon EMT induction, CAR expression was reduced whereas CD46 expression was not reduced at all (Figure 6B). Further, when conventionally used TelomeScan was used for PancI cells having undergone EMT, only about 35% of these cells were GFP-positive, whereas almost 90% or more of the cells were GFP-positive in the case of Ad34 fiber 142-3pT(El,E3) (Figure 6C).

These results indicated that the recombinant virus of the present invention allowed highly sensitive detection of cancer cells having undergone epithelial-mesenchymal transition (EMT).

### Example 6

### Detection of cancer stem cells

MCF7 cells and MCF7-ADR cells (cancer cells resistant to the anticancer agent adriamycin) were each seeded in a 96-well plate at 1 × 10³ cells/well, and on the following day, adriamycin was added thereto at 0.2, 1, 5, 25 or 125 µg/mL. After 24 hours from the addition of adriamycin, an alamarBlue® cell viability reagent was used to measure cell viability (value: mean ± S.D. (n = 6)).

MCF7 cells and MCF7-ADR cells were also analyzed by flow cytometry for expression of CAR, CD46, P-glycoprotein (MDR), CD24 and CD44. 5 × 10⁵ MCF7-ADR cells were suspended in 100 µl of 2% FCS-containing PBS, and FITC-labeled mouse anti-human CD24 antibody and PE-labeled mouse anti-human CD44 antibody were each added thereto in a volume of 1 µl, followed by reaction for 1 hour on ice under light-shielded conditions. After washing with 4 ml of 2% FCS-containing PBS, the suspension was centrifuged at 1500 rpm for 5 minutes to remove the supernatant by aspiration. The cells were suspended again in 100 µl of 2% FCS-containing PBS and subjected to a cell sorter (FACS Aria II cell sorter; BD Biosciences) to sort a CD24-negative and CD44-positive cell fraction. The data obtained were analyzed by FCS multi-color data analysis software (Flowjo). In human breast cancer cells, a fraction having the characteristics of CD24-negative and CD44-positive cells is known to be cancer stem cells (Al-Hajj M., et al., Proc Natl Acad Sci USA, 100; 3983-3988, (2003)). The virus of the present invention was infected into the cells in the same manner as shown in Example 4.

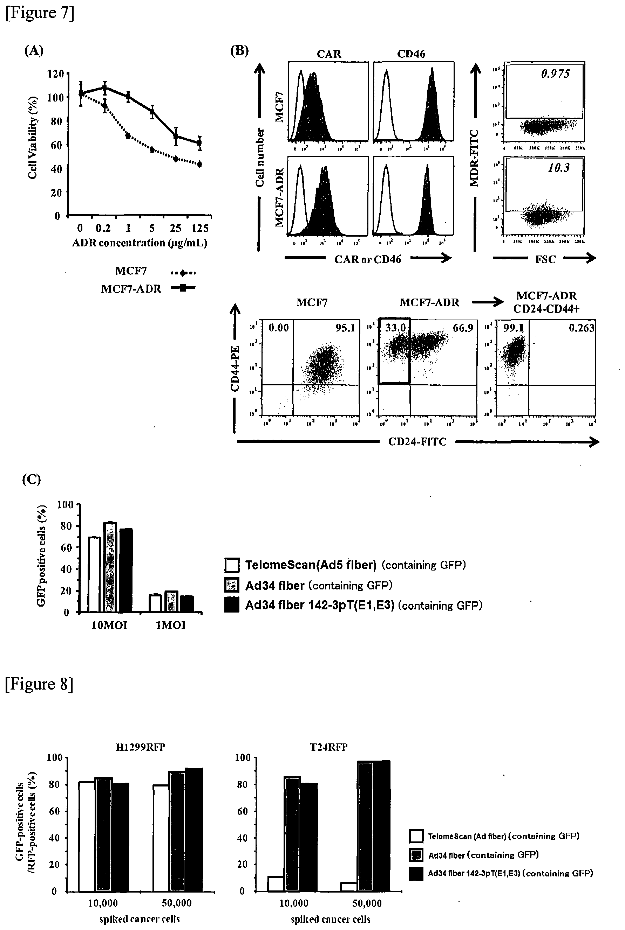

As a result, MCF7-ADR cells showed significantly high viability even in the presence of adriamycin when compared to MCF7 cells and hence were found to have drug resistance ability (Figure 7A). MCF7-ADR cells were also found to highly express CAR and CD46 as in the case of MCF7 cells. Moreover, MCF7-ADR cells were also found to highly express MDR, which is a membrane protein responsible for drug elimination ability (Figure 7B). Further, when Ad34 fiber 142-3pT(El,E3) was infected into CD24-negative and CD44-positive cells among MCF-ADR cells, 80% or more of the cells were GFP-positive. In contrast, about 70% of the cells were GFP-positive in the case of conventionally used TelomeScan (Figure 7C).

These results indicated that the recombinant virus of the present invention allowed detection of drug-resistant cancer cells. Moreover, it was also indicated that the recombinant virus of the present invention allowed detection of cancer stem cells.

### Example 7

### Detection of cancer cells in blood samples using Ad34 fiber 142-3pT(E1,E3)

H1299 cells or T24 cells were infected with a lentivirus vector expressing a red fluorescent protein (monomeric red fluorescent protein; RFP) at an MOI of 100 and cultured. To obtain cell clones, the cells were then seeded in a 96-well plate at 0.1 cells/well and cultured until colonies were formed. RFP-expressing cells were selected under a fluorescence microscope and subjected to extended culture, followed by flow cytometry to measure the intensity of RFP expression. Then, cells showing high intensity of RFP expression were identified as RFP-expressing cells.

Human peripheral blood mononuclear cells (hPBMCs) obtained from 1.0 mL of human peripheral blood were suspended in 800 µL of RPMI-1640 medium (10% FCS, supplemented with antibiotics). To the hPBMC suspension, cancer cells prepared at 1.0 × 10⁵ or 5.0 × 10⁵ cells/mL were added in a volume of 100 µL (in Figure 8, "spiked cancer cells" represents the number of cancer cells added to the hPBMC suspension). Further, a conditionally replicating Ad suspension prepared at 2 x 10⁸ pfu/mL was added in a volume of 100 µL to give a total volume of 1 mL, followed by culture at 37°C for 24 hours while slowly rotating with a rotator.

The cell suspension cultured for 24 hours after virus infection was centrifuged at 300 x g for 5 minutes to remove the supernatant. A cell fixative was added in a volume of 200 µL and reacted at 4°C under light-shielded conditions for 15 minutes. After addition of 1 mL PBS, the suspension was centrifuged at 300 x g for 5 minutes to remove the supernatant. The cells were suspended in 2% FCS-containing PBS and measured for GFP-positive rate using a flow cytometer (MACS Quant Analyzer; Miltenyi Biotec). The data obtained were analyzed by FCS multi-color data analysis software (Flowjo).

In this study, cancer cells labeled with RFP (red fluorescent protein) were mixed into hPBMCs to examine whether the cancer cells in hPBMCs were able to be detected. As a result, in the case of CAR-positive cancer cells (H1299), TelomeScan (Ad5 fiber) and Ad34 fiber 142-3pT(E1,E3) were both able to detect 80% or more of the cancer cells. On the other hand, in the case of CAR-negative cancer cells (T24), TelomeScan (Ad5 fiber) achieved very low detection efficiency (about 10% of the cells were detected as being GFP-positive), whereas Ad34 fiber 142-3pT(E1,E3) was able to detect 80% or more of the cancer cells (Figure 8).

This result indicated that the recombinant adenovirus of the present invention allowed efficient detection of not only CAR-positive cancer cells but also CAR negative cancer cells.

### INDUSTRIAL APPLICABILITY

Reagents comprising the recombinant adenovirus of the present invention enable simple and highly sensitive detection of CAR-negative cancer cells without detection of normal blood cells (e.g., leukocytes).

### Sequence Listing Free Text

SEQ ID NO: 4: synthetic DNA
SEQ ID NOs: 5 to 26: synthetic RNA
SEQ ID NOs: 27 to 28: synthetic DNA
SEQ ID NOs: 43 to 46: synthetic DNA
SEQ ID NO: 50: synthetic DNA

## Claims

1. A recombinant adenovirus, which comprises a replication cassette comprising a polynucleotide, which comprises human telomerase reverse transcriptase promoter, E1A gene, IRES sequence and E1B gene in this order and which comprises a target sequence of a first microRNA wherein the replication cassette is integrated into the E1 region of the adenovirus genome and wherein the first microRNA is expressed in blood cells; a labelling cassette comprising a reporter gene, a promoter capable of regulating the expression of the gene and a target sequence of a second microRNA, wherein the labelling cassette is integrated into the E3 region of the adenovirus genome and the second microRNA is expressed in blood cells; and a gene encoding a CD46-binding fiber protein.

2. The recombinant adenovirus according to claim 1, wherein the first microRNA is at least one selected from the group consisting of miR-142, miR-15, miR-16, miR-21, miR-126, miR-181, miR-223 and miR-296.

3. The recombinant adenovirus according to claim 1 or 2, wherein the second microRNA is at least one selected from the group consisting of miR-142, miR-15, miR-16, miR-21, miR-126, miR-181, miR-223 and miR-296.

4. The recombinant adenovirus according to any one of claims 1 to 3, wherein the reporter gene is a gene encoding a protein which emits fluorescence or a gene encoding an enzyme protein which generates a luminophore or a chromophore upon enzymatic reaction.

5. The recombinant adenovirus according to any one of the preceding claims, wherein the promoter is human telomerase reverse transcriptase promoter or cytomegalovirus promoter.

6. The recombinant adenovirus according to any one of the preceding claims, wherein the CD46-binding fiber protein comprises at least the fiber knob region in the fiber protein of adenovirus type 34 or 35.

7. A reagent for cancer cell detection, which comprises the recombinant adenovirus according to any one of claims 1 to 6.

8. The reagent according to claim 7, wherein the cancer cells are present in a biological sample taken from a subject, optionally wherein the biological sample is blood and optionally wherein the cancer cells are circulating tumor cells.

9. The reagent according to claim 7 or 8, wherein:
(a) the cancer cells are drug-resistant cancer cells; and/or
(b) the cancer cells are cancer stem cells; and/or
(c) the cancer cells are cancer cells having undergone epithelial-mesenchymal transition or mesenchymal-epithelial transition.

10. A reagent for cancer diagnosis, which comprises the recombinant adenovirus according to any one of claims 1 to 6.

11. A method for cancer cell detection, which comprises contacting cancer cells with the recombinant adenovirus according to claim 4 and detecting the fluorescence or color produced by the cancer cells.

12. The method according to claim 11, wherein the cancer cells are present in a biological sample taken from a subject.

13. The method according to claim 12, wherein the biological sample is blood.

14. The method according to claim 13, wherein the cancer cells in the blood sample are circulating tumor cells.

15. The method according to any one of claims 11 to 14, wherein:
(a) the cancer cells are drug-resistant cancer cells; and/or
(b) the cancer cells are cancer stem cells; and/or
(c) the cancer cells are cancer cells having undergone epithelial-mesenchymal transition or mesenchymal-epithelial transition.

## Patentansprüche

1. Rekombinantes Adenovirus, das eine Replikationskassette umfasst, umfassend ein Polynukleotid, das einen menschlichen Telomerase-Reverse-Transkriptase-Promotor, ein E1A-Gen, eine IRES-Sequenz und ein E1B-Gen in dieser Reihenfolge umfasst und das eine Zielsequenz einer ersten MikroRNA umfasst, wobei die Replikationskassette in die E1-Region des Adenovirus-Genoms integriert ist und wobei die erste MikroRNA in Blutzellen exprimiert ist; eine Markierungskassette, umfassend ein Reportergen, einen Promoter, der in der Lage ist, die Expression des Gens zu regulieren, und eine Zielsequenz einer zweiten MikroRNA, wobei die Markierungskassette in die E3-Region des Adenovirus-Genoms integriert ist und die zweite MikroRNA in Blutzellen exprimiert ist; und ein Gen, das ein CD46-bindendes Faserprotein kodiert.

2. Rekombinantes Adenovirus nach Anspruch 1, wobei die erste MikroRNA mindestens eine ist, die ausgewählt ist aus der Gruppe bestehend aus miR-142, miR-15, miR-16, miR-21, miR-126, miR-181, miR-223 und miR-296.

3. Rekombinantes Adenovirus nach Anspruch 1 oder 2, wobei die zweite MikroRNA mindestens eine ist, die ausgewählt ist aus der Gruppe bestehend aus miR-142, miR-15, miR-16, miR-21, miR-126, miR-181, miR-223 und miR-296.

4. Rekombinantes Adenovirus nach einem der Ansprüche 1 bis 3, wobei das Reportergen ein Gen ist, das ein Protein kodiert, das Fluoreszenz emittiert, oder ein Gen, das ein Enzymprotein kodiert, das ein Luminophor oder ein Chromophor nach einer enzymatischen Reaktion erzeugt.

5. Rekombinantes Adenovirus nach einem der vorstehenden Ansprüche, wobei der Promoter ein menschlicher Telomerase-Reverse-Transkriptase-Promoter oder ein Zytomegalovirus-Promoter ist.

6. Rekombinantes Adenovirus nach einem der vorstehenden Ansprüche, wobei das CD46-bindende Faserprotein mindestens die Faserkopfregion in dem Faserprotein von Adenovirustyp 34 oder 35 umfasst.

7. Reagenz zur Krebszellerkennung, das das rekombinante Adenovirus nach einem der Ansprüche 1 bis 6 umfasst.

8. Reagenz nach Anspruch 7, wobei die Krebszellen in einer biologischen Probe vorhanden sind, die einem Subjekt entnommen wurde, optional wobei die biologische Probe Blut ist und optional wobei die Krebszellen zirkulierende Krebszellen sind.

9. Reagenz nach Anspruch 7 oder 8, wobei:
(a) die Krebszellen arzneimittelresistente Krebszellen sind und/oder
(b) die Krebszellen Krebsstammzellen sind und/oder
(c) die Krebszellen Krebszellen sind, die eine epithelial-mesenchymale Transition oder eine mesenchymal-epitheliale Transition durchlaufen haben.

10. Reagenz zur Krebsdiagnose, das das rekombinante Adenovirus nach einem der Ansprüche 1 bis 6 umfasst.

11. Verfahren zur Krebszellerkennung, das ein Kontaktieren von Krebszellen mit dem rekombinanten Adenovirus nach Anspruch 4 und ein Erkennen der Fluoreszenz oder Farbe, die von den Krebszellen produziert wird, umfasst.

12. Verfahren nach Anspruch 11, wobei die Krebszellen in einer biologischen Probe vorhanden sind, die einem Subjekt entnommen wurde.

13. Verfahren nach Anspruch 12, wobei die biologische Probe Blut ist.

14. Verfahren nach Anspruch 13, wobei die Krebszellen in der Blutprobe zirkulierende Krebszellen sind.

15. Verfahren nach einem der Ansprüche 11 bis 14, wobei:
(a) die Krebszellen arzneimittelresistente Krebszellen sind und/oder
(b) die Krebszellen Krebsstammzellen sind und/oder
(c) die Krebszellen Krebszellen sind, die eine epithelial-mesenchymale Transition oder eine mesenchymal-epitheliale Transition durchlaufen haben.

## Revendications

1. Adénovirus recombiné, qui comprend une cassette de réplication comprenant un polynucléotide, qui comprend le promoteur de transcriptase inverse de télomérase humaine, le gène E1A, la séquence IRES et le gène E1B, dans cet ordre, et qui comprend une séquence cible d'un premier microARN, dans lequel la cassette de réplication est intégrée dans la région E1 du génome d'adénovirus et dans lequel le premier microARN est exprimé dans des cellules sanguines ; une cassette de marquage comprenant un gène rapporteur, un promoteur capable de réguler l'expression du gène et une séquence cible d'un second microARN, dans lequel la cassette de marquage est intégrée dans la région E3 du génome d'adénovirus et le second microARN est exprimé dans des cellules sanguines ; et un gène encodant une protéine fibreuse de liaison avec CD46.

2. Adénovirus recombiné selon la revendication 1, dans lequel le premier microARN est au moins l'un sélectionné parmi le groupe constitué de : miR-142, miR-15, miR-16, miR-21, miR-126, miR-181, miR-223 et miR-296.

3. Adénovirus recombiné selon la revendication 1 ou 2, dans lequel le second microARN est au moins l'un sélectionné parmi le groupe constitué de : miR-142, miR-15, miR-16, miR-21, miR-126, miR-181, miR-223 et miR-296.

4. Adénovirus recombiné selon l'une quelconque des revendications 1 à 3, dans lequel le gène rapporteur est un gène encodant une protéine qui émet une fluorescence ou un gène encodant une protéine enzymatique qui génère un luminophore ou un chromophore lors d'une réaction enzymatique.

5. Adénovirus recombiné selon l'une quelconque des revendications précédentes, dans lequel le promoteur est le promoteur de transcriptase inverse de télomérase humaine ou le promoteur de cytomégalovirus.

6. Adénovirus recombiné selon l'une quelconque des revendications précédentes, dans lequel
la protéine fibreuse de liaison avec CD46 comprend au moins la région de bouton de fibre dans la protéine fibreuse d'adénovirus type 34 ou 35.

7. Réactif pour détection de cellules cancéreuses, qui comprend l'adénovirus recombiné selon l'une quelconque des revendications 1 à 6.

8. Réactif selon la revendication 7, dans lequel les cellules cancéreuses sont présentes dans un échantillon biologique prélevé chez un sujet, optionnellement dans lequel l'échantillon biologique est du sang et optionnellement dans lequel les cellules cancéreuses sont des cellules tumorales circulantes.

9. Réactif selon la revendication 7 ou 8, dans lequel :
(a) les cellules cancéreuses sont des cellules cancéreuses pharmacorésistantes; et/ou
(b) les cellules cancéreuses sont des cellules souches cancéreuses; et/ou
(c) les cellules cancéreuses sont des cellules cancéreuses ayant subi une transition épithéliale-mésenchymale ou une transition mésenchymale-épithéliale.

10. Réactif pour diagnostic du cancer, qui comprend l'adénovirus recombiné selon l'une quelconque des revendications 1 à 6.

11. Procédé pour détection de cellules cancéreuses, qui comprend la mise en contact de cellules cancéreuses avec l'adénovirus recombiné selon la revendication 4 et la détection de la fluorescence ou couleur produite par les cellules cancéreuses.

12. Procédé selon la revendication 11, dans lequel les cellules cancéreuses sont présentes dans un échantillon biologique prélevé chez un sujet.

13. Procédé selon la revendication 12, dans lequel l'échantillon biologique est du sang.

14. Procédé selon la revendication 13, dans lequel les cellules cancéreuses dans l'échantillon sanguin sont des cellules tumorales circulantes.

15. Procédé selon l'une quelconque des revendications 11 à 14, dans lequel :
(a) les cellules cancéreuses sont des cellules cancéreuses pharmacorésistantes; et/ou
(b) les cellules cancéreuses sont des cellules souches cancéreuses; et/ou
(c) les cellules cancéreuses sont des cellules cancéreuses ayant subi une transition épithéliale-mésenchymale ou une transition mésenchymale-épithéliale.


### SEQUENCE LISTING

<110> National Institute of Biomedical Innovation
<120> Conditionally replicating adenovirus
<130> PCT12-0008
<150> JP2011-181414
   <151> 2011-08-23
<160> 50
<170> PatentIn version 3.5
<210> 1
   <211> 455
   <212> DNA
   <213> Homo sapiens
<400> 1
   tggcccctcc ctcgggttac cccacagcct aggccgattc gacctctctc cgctggggcc      60
   ctcgctggcg tccctgcacc ctgggagcgc gagcggcgcg cgggcgggga agcgcggccc     120
   agacccccgg gtccgcccgg agcagctgcg ctgtcggggc caggccgggc tcccagtgga     180
   ttcgcgggca cagacgccca ggaccgcgct ccccacgtgg cggagggact ggggacccgg     240
   gcacccgtcc tgccccttca ccttccagct ccgcctcctc cgcgcggacc ccgccccgtc     300
   ccgacccctc ccgggtcccc ggcccagccc cctccgggcc ctcccagccc ctccccttcc     360
   tttccgcggc cccgccctct cctcgcggcg cgagtttcag gcagcgctgc gtcctgctgc     420
   gcacgtggga agccctggcc ccggccaccc ccgcg                                455
<210> 2
   <211> 899
   <212> DNA
   <213> Adenovirus
<400> 2
   acaccgggac tgaaaatgag acatattatc tgccacggag gtgttattac cgaagaaatg        60
   gccgccagtc ttttggacca gctgatcgaa gaggtactgg ctgataatct tccacctcct       120
   agccattttg aaccacctac ccttcacgaa ctgtatgatt tagacgtgac ggcccccgaa       180
   gatcccaacg aggaggcggt ttcgcagatt tttcccgact ctgtaatgtt ggcggtgcag       240
   gaagggattg acttactcac ttttccgccg gcgcccggtt ctccggagcc gcctcacctt       300
   tcccggcagc ccgagcagcc ggagcagaga gccttgggtc cggtttctat gccaaacctt       360
   gtaccggagg tgatcgatct tacctgccac gaggctggct ttccacccag tgacgacgag       420
   gatgaagagg gtgaggagtt tgtgttagat tatgtggagc accccgggca cggttgcagg       480
   tcttgtcatt atcaccggag gaatacgggg gacccagata ttatgtgttc gctttgctat       540
   atgaggacct gtggcatgtt tgtctacagt cctgtgtctg aacctgagcc tgagcccgag       600
   ccagaaccgg agcctgcaag acctacccgc cgtcctaaaa tggcgcctgc tatcctgaga       660
   cgcccgacat cacctgtgtc tagagaatgc aatagtagta cggatagctg tgactccggt       720
   ccttctaaca cacctcctga gatacacccg gtggtcccgc tgtgccccat taaaccagtt       780
   gccgtgagag ttggtgggcg tcgccaggct gtggaatgta tcgaggactt gcttaacgag       840
   cctgggcaac ctttggactt gagctgtaaa cgccccaggc cataaggtgt aaacctgtg        899
<210> 3
   <211> 1823
   <212> DNA
   <213> Adenovirus
<400> 3
   ctgacctcat ggaggcttgg gagtgtttgg aagatttttc tgctgtgcgt aacttgctgg      60
   aacagagctc taacagtacc tcttggtttt ggaggtttct gtggggctca tcccaggcaa     120
   agttagtctg cagaattaag gaggattaca agtgggaatt tgaagagctt ttgaaatcct     180
   gtggtgagct gtttgattct ttgaatctgg gtcaccaggc gcttttccaa gagaaggtca     240
   tcaagacttt ggatttttcc acaccggggc gcgctgcggc tgctgttgct tttttgagtt     300
   ttataaagga taaatggagc gaagaaaccc atctgagcgg ggggtacctg ctggattttc     360
   tggccatgca tctgtggaga gcggttgtga gacacaagaa tcgcctgcta ctgttgtctt     420
   ccgtccgccc ggcgataata ccgacggagg agcagcagca gcagcaggag gaagccaggc     480
   ggcggcggca ggagcagagc ccatggaacc cgagagccgg cctggaccct cgggaatgaa     540
   tgttgtacag gtggctgaac tgtatccaga actgagacgc attttgacaa ttacagagga     600
   tgggcagggg ctaaaggggg taaagaggga gcggggggct tgtgaggcta cagaggaggc     660
   taggaatcta gcttttagct taatgaccag acaccgtcct gagtgtatta cttttcaaca     720
   gatcaaggat aattgcgcta atgagcttga tctgctggcg cagaagtatt ccatagagca     780
   gctgaccact tactggctgc agccagggga tgattttgag gaggctatta gggtatatgc     840
   aaaggtggca cttaggccag attgcaagta caagatcagc aaacttgtaa atatcaggaa     900
   ttgttgctac atttctggga acggggccga ggtggagata gatacggagg atagggtggc     960
   ctttagatgt agcatgataa atatgtggcc gggggtgctt ggcatggacg gggtggttat    1020
   tatgaatgta aggtttactg gccccaattt tagcggtacg gttttcctgg ccaataccaa    1080
   ccttatccta cacggtgtaa gcttctatgg gtttaacaat acctgtgtgg aagcctggac    1140
   cgatgtaagg gttcggggct gtgcctttta ctgctgctgg aagggggtgg tgtgtcgccc    1200
   caaaagcagg gcttcaatta agaaatgcct ctttgaaagg tgtaccttgg gtatcctgtc    1260
   tgagggtaac tccagggtgc gccacaatgt ggcctccgac tgtggttgct tcatgctagt    1320
   gaaaagcgtg gctgtgatta agcataacat ggtatgtggc aactgcgagg acagggcctc    1380
   tcagatgctg acctgctcgg acggcaactg tcacctgctg aagaccattc acgtagccag 1440
   ccactctcgc aaggcctggc cagtgtttga gcataacata ctgacccgct gttccttgca 1500
   tttgggtaac aggagggggg tgttcctacc ttaccaatgc aatttgagtc acactaagat 1560
   attgcttgag cccgagagca tgtccaaggt gaacctgaac ggggtgtttg acatgaccat 1620
   gaagatctgg aaggtgctga ggtacgatga gacccgcacc aggtgcagac cctgcgagtg 1680
   tggcggtaaa catattagga accagcctgt gatgctggat gtgaccgagg agctgaggcc 1740
   cgatcacttg gtgctggcct gcacccgcgc tgagtttggc tctagcgatg aagatacaga 1800
   ttgaggtact gaaatgtgtg ggc 1823
<210> 4
   <211> 605
   <212> DNA
   <213> Artificial
<220>
   <223> synthetic DNA
<400> 4
   tgcatctagg gcggccaatt ccgcccctct ccctcccccc cccctaacgt tactggccga 60
   agccgcttgg aataaggccg gtgtgcgttt gtctatatgt gattttccac catattgccg 120
   tcttttggca atgtgagggc ccggaaacct ggccctgtct tcttgacgag cattcctagg 180
   ggtctttccc ctctcgccaa aggaatgcaa ggtctgttga atgtcgtgaa ggaagcagtt 240
   cctctggaag cttcttgaag acaaacaacg tctgtagcga ccctttgcag gcagcggaac 300
   cccccacctg gcgacaggtg cctctgcggc caaaagccac gtgtataaga tacacctgca 360
   aaggcggcac aaccccagtg ccacgttgtg agttggatag ttgtggaaag agtcaaatgg 420
   ctctcctcaa gcgtattcaa caaggggctg aaggatgccc agaaggtacc ccattgtatg 480
   ggatctgatc tggggcctcg gtgcacatgc tttacatgtg tttagtcgag gttaaaaaaa 540
   cgtctaggcc ccccgaacca cggggacgtg gttttccttt gaaaaacacg atgataagct 600
   tgcca 605
<210> 5
   <211> 23
   <212> RNA
   <213> Artificial
<220>
   <223> synthetic RNA
<400> 5
   uguaguguuu ccuacuuuau gga 23
<210> 6
   <211> 21
   <212> RNA
   <213> Artificial
<220>
   <223> synthetic RNA
<400> 6
   cauaaaguag aaagcacuac u 21
<210> 7
   <211> 22
   <212> RNA
   <213> Artificial
<220>
   <223> synthetic RNA
<400> 7
   uagcagcaca uaaugguuug ug 22
<210> 8
   <211> 22
   <212> RNA
   <213> Artificial
<220>
   <223> synthetic RNA
<400> 8
   caggccauau ugugcugccu ca 22
<210> 9
   <211> 22
   <212> RNA
   <213> Artificial
<220>
   <223> synthetic RNA
<400> 9
   uagcagcacg uaaauauugg cg 22
<210> 10
   <211> 22
   <212> RNA
   <213> Artificial
<220>
   <223> synthetic RNA
<400> 10
   ccaguauuaa cugugcugcu ga 22
<210> 11
   <211> 22
   <212> RNA
   <213> Artificial
<220>
   <223> synthetic RNA
<400> 11
   uagcuuauca gacugauguu ga 22
<210> 12
   <211> 21
   <212> RNA
   <213> Artificial
<220>
   <223> synthetic RNA
<400> 12
   caacaccagu cgaugggcug u 21
<210> 13
   <211> 22
   <212> RNA
   <213> Artificial
<220>
   <223> synthetic RNA
<400> 13
   ucguaccgug aguaauaaug cg 22
<210> 14
   <211> 21
   <212> RNA
   <213> Artificial
<220>
   <223> synthetic RNA
<400> 14
   cauuauuacu uuugguacgc g 21
<210> 15
   <211> 23
   <212> RNA
   <213> Artificial
<220>
   <223> synthetic RNA
<400> 15
   aacauucaac gcugucggug agu 23
<210> 16
   <211> 22
   <212> RNA
   <213> Artificial
<220>
   <223> synthetic RNA
<400> 16
   ugucaguuug ucaaauaccc ca 22
<210> 17
   <211> 22
   <212> RNA
   <213> Artificial
<220>
   <223> synthetic RNA
<400> 17
   cguguauuug acaagcugag uu 22
<210> 18
   <211> 22
   <212> RNA
   <213> Artificial
<220>
   <223> synthetic RNA
<400> 18
   gaggguuggg uggaggcucu cc 22
<210> 19
   <211> 21
   <212> RNA
   <213> Artificial
<220>
   <223> synthetic RNA
<400> 19
   agggcccccc cucaauccug u 21
<210> 20
   <211> 24
   <212> RNA
   <213> Artificial
<220>
   <223> synthetic RNA
<400> 20
   ucccugagac ccuuuaaccu guga 24
<210> 21
   <211> 21
   <212> RNA
   <213> Artificial
<220>
   <223> synthetic RNA
<400> 21
   ugagaugaag cacuguagcu c 21
<210> 22
   <211> 22
   <212> RNA
   <213> Artificial
<220>
   <223> synthetic RNA
<400> 22
   ggugcagugc ugcaucucug gu 22
<210> 23
   <211> 23
   <212> RNA
   <213> Artificial
<220>
   <223> synthetic RNA
<400> 23
   guccaguuuu cccaggaauc ccu 23
<210> 24
   <211> 22
   <212> RNA
   <213> Artificial
<220>
   <223> synthetic RNA
<400> 24
   ggauuccugg aaauacuguu cu 22
<210> 25
   <211> 23
   <212> RNA
   <213> Artificial
<220>
   <223> synthetic RNA
<400> 25
   cccaguguuc agacuaccug uuc 23
<210> 26
   <211> 22
   <212> RNA
   <213> Artificial
<220>
   <223> synthetic RNA
<400> 26
   ugagguagua gguuguauag uu 22
<210> 27
   <211> 69
   <212> DNA
   <213> Artificial
<220>
   <223> synthetic DNA
<400> 27
   gcggcctcca taaagtagga aacactacac agctccataa agtaggaaac actacattat        60
   aagcggtac                                                                  69
<210> 28
   <211> 113
   <212> DNA
   <213> Artificial
<220>
   <223> synthetic DNA
<400> 28
   ggcctccata aagtaggaaa cactacacag ctccataaag taggaaacac tacattaatt        60
   ccataaagta ggaaacacta caccactcca taaagtagga aacactacag tac              113
<210> 29
   <211> 1307
   <212> DNA
   <213> Homo sapiens
<400> 29
   accgtccagg gagcaggtag ctgctgggct ccggggacac tttgcgttcg ggctgggagc 60
   gtgctttcca cgacggtgac acgcttccct ggattggcag ccagactgcc ttccgggtca 120
   ctgccatgga ggagccgcag tcagatccta gcgtcgagcc ccctctgagt caggaaacat 180
   tttcagacct atggaaacta cttcctgaaa acaacgttct gtcccccttg ccgtcccaag 240
   caatggatga tttgatgctg tccccggacg atattgaaca atggttcact gaagacccag 300
   gtccagatga agctcccaga atgccagagg ctgctccccg cgtggcccct gcaccagcga 360
   ctcctacacc ggcggcccct gcaccagccc cctcctggcc cctgtcatct tctgtccctt 420
   cccagaaaac ctaccagggc agctacggtt tccgtctggg cttcttgcat tctgggacag 480
   ccaagtctgt gacttgcacg tactcccctg ccctcaacaa gatgttttgc caactggcca 540
   agacctgccc tgtgcagctg tgggttgatt ccacaccccc gcccggcacc cgcgtccgcg 600
   ccatggccat ctacaagcag tcacagcaca tgacggaggt tgtgaggcgc tgcccccacc 660
   atgagcgctg ctcagatagc gatggtctgg cccctcctca gcatcttatc cgagtggaag 720
   gaaatttgcg tgtggagtat ttggatgaca gaaacacttt tcgacatagt gtggtggtgc 780
   cctatgagcc gcctgaggtt ggctctgact gtaccaccat ccactacaac tacatgtgta 840
   acagttcctg catgggcggc atgaaccgga ggcccatcct caccatcatc acactggaag 900
   actccagtgg taatctactg ggacggaaca gctttgaggt gcgtgtttgt gcctgtcctg 960
   ggagagaccg gcgcacagag gaagagaatc tccgcaagaa aggggagcct caccacgagc 1020
   tgcccccagg gagcactaag cgagcactgc ccaacaacac cagctcctct ccccagccaa 1080
   agaagaaacc actggatgga gaatatttca cccttcagat ccgtgggcgt gagcgcttcg 1140
   agatgttccg agagctgaat gaggccttgg aactcaagga tgcccaggct gggaaggagc 1200
   caggggggag cagggctcac tccagccacc tgaagtccaa aaagggtcag tctacctccc 1260
   gccataaaaa actcatgttc aagacagaag ggcctgactc agactga 1307
<210> 30
   <211> 837
   <212> DNA
   <213> Homo sapiens
<400> 30
   gaggactccg cgacggtccg caccctgcgg ccagagcggc tttgagctcg gctgcttccg 60
   cgctaggcgc tttttcccag aagcaatcca ggcgcgcccg ctggttcttg agcgccagga 120
   aaagcccgga gctaacgacc ggccgctcgg cactgcacgg ggccccaagc cgcagaagaa 180
   ggacgacggg agggtaatga agctgagccc aggtctccta ggaaggagag agtgcgccgg 240
   agcagcgtgg gaaagaaggg aagagtgtcg ttaagtttac ggccaacggt ggattatccg 300
   ggccgctgcg cgtctggggg ctgcggaatg cgcgaggaga acaagggcat gcccagtggg 360
   ggcggcagcg atgagggtct ggccacgccg gcgcggggac tagtggagaa ggtgcgacac 420
   tcctgggaag ccggcgcgga tcccaacgga gtcaaccgtt tcgggaggcg cgcgatccag 480
   gtcatgatga tgggcagcgc ccgcgtggcg gagctgctgc tgctccacgg cgcggagccc 540
   aactgcgcag accctgccac tctcacccga ccggtgcatg atgctgcccg ggagggcttc 600
   ctggacacgc tggtggtgct gcaccgggcc ggggcgcggc tggacgtgcg cgatgcctgg 660
   ggtcgtctgc ccgtggactt ggccgaggag cggggccacc gcgacgttgc agggtacctg 720
   cgcacagcca cgggggactg acgccaggtt ccccagccgc ccacaacgac tttattttct 780
   tacccaattt cccaccccca cccacctaat tcgatgaagg ctgccaacgg ggagcgg 837
<210> 31
   <211> 987
   <212> DNA
   <213> Homo sapiens
<400> 31
   cggagagggg gagaacagac aacgggcggc ggggagcagc atggagccgg cggcggggag 60
   cagcatggag ccttcggctg actggctggc cacggccgcg gcccggggtc gggtagagga 120
   ggtgcgggcg ctgctggagg cgggggcgct gcccaacgca ccgaatagtt acggtcggag 180
   gccgatccag gtcatgatga tgggcagcgc ccgagtggcg gagctgctgc tgctccacgg 240
   cgcggagccc aactgcgccg accccgccac tctcacccga cccgtgcacg acgctgcccg      300
   ggagggcttc ctggacacgc tggtggtgct gcaccgggcc ggggcgcggc tggacgtgcg      360
   cgatgcctgg ggccgtctgc ccgtggacct ggctgaggag ctgggccatc gcgatgtcgc      420
   acggtacctg cgcgcggctg cggggggcac cagaggcagt aaccatgccc gcatagatgc       480
   cgcggaaggt ccctcagaca tccccgattg aaagaaccag agaggctctg agaaacctcg      540
   ggaaacttag atcatcagtc accgaaggtc ctacagggcc acaactgccc ccgccacaac      600
   ccaccccgct ttcgtagttt tcatttagaa aatagagctt ttaaaaatgt cctgcctttt     660
   aacgtagata taagccttcc cccactaccg taaatgtcca tttatatcat tttttatata      720
   ttcttataaa aatgtaaaaa agaaaaacac cgcttctgcc ttttcactgt gttggagttt      780
   tctggagtga gcactcacgc cctaagcgca cattcatgtg ggcatttctt gcgagcctcg      840
   cagcctccgg aagctgtcga cttcatgaca agcattttgt gaactaggga agctcagggg      900
   ggttactggc ttctcttgag tcacactgct agcaaatggc agaaccaaag ctcaaataaa      960
   aataaaataa ttttcattca ttcactc                                          987
<210> 32
   <211> 8972
   <212> DNA
   <213> Homo sapiens
<400> 32
   gtccaagggt agccaaggat ggctgcagct tcatatgatc agttgttaaa gcaagttgag      60
   gcactgaaga tggagaactc aaatcttcga caagagctag aagataattc caatcatctt     120
   acaaaactgg aaactgaggc atctaatatg aaggaagtac ttaaacaact acaaggaagt     180
   attgaagatg aagctatggc ttcttctgga cagattgatt tattagagcg tcttaaagag     240
   cttaacttag atagcagtaa tttccctgga gtaaaactgc ggtcaaaaat gtccctccgt     300
   tcttatggaa gccgggaagg atctgtatca agccgttctg gagagtgcag tcctgttcct     360
   atgggttcat ttccaagaag agggtttgta aatggaagca gagaaagtac tggatattta     420
   gaagaacttg agaaagagag gtcattgctt cttgctgatc ttgacaaaga agaaaaggaa     480
   aaagactggt attacgctca acttcagaat ctcactaaaa gaatagatag tcttccttta     540
   actgaaaatt tttccttaca aacagatatg accagaaggc aattggaata tgaagcaagg     600
   caaatcagag ttgcgatgga agaacaacta ggtacctgcc aggatatgga aaaacgagca     660
   cagcgaagaa tagccagaat tcagcaaatc gaaaaggaca tacttcgtat acgacagctt     720
   ttacagtccc aagcaacaga agcagagagg tcatctcaga acaagcatga aaccggctca     780
   catgatgctg agcggcagaa tgaaggtcaa ggagtgggag aaatcaacat ggcaacttct     840
   ggtaatggtc agggttcaac tacacgaatg gaccatgaaa cagccagtgt tttgagttct     900
   agtagcacac actctgcacc tcgaaggctg acaagtcatc tgggaaccaa ggtggaaatg 960
   gtgtattcat tgttgtcaat gcttggtact catgataagg atgatatgtc gcgaactttg 1020
   ctagctatgt ctagctccca agacagctgt atatccatgc gacagtctgg atgtcttcct 1080
   ctcctcatcc agcttttaca tggcaatgac aaagactctg tattgttggg aaattcccgg 1140
   ggcagtaaag aggctcgggc cagggccagt gcagcactcc acaacatcat tcactcacag 1200
   cctgatgaca agagaggcag gcgtgaaatc cgagtccttc atcttttgga acagatacgc 1260
   gcttactgtg aaacctgttg ggagtggcag gaagctcatg aaccaggcat ggaccaggac 1320
   aaaaatccaa tgccagctcc tgttgaacat cagatctgtc ctgctgtgtg tgttctaatg 1380
   aaactttcat ttgatgaaga gcatagacat gcaatgaatg aactaggggg actacaggcc 1440
   attgcagaat tattgcaagt ggactgtgaa atgtacgggc ttactaatga ccactacagt 1500
   attacactaa gacgatatgc tggaatggct ttgacaaact tgacttttgg agatgtagcc 1560
   aacaaggcta cgctatgctc tatgaaaggc tgcatgagag cacttgtggc ccaactaaaa 1620
   tctgaaagtg aagacttaca gcaggttatt gcaagtgttt tgaggaattt gtcttggcga 1680
   gcagatgtaa atagtaaaaa gacgttgcga gaagttggaa gtgtgaaagc attgatggaa 1740
   tgtgctttag aagttaaaaa ggaatcaacc ctcaaaagcg tattgagtgc cttatggaat 1800
   ttgtcagcac attgcactga gaataaagct gatatatgtg ctgtagatgg tgcacttgca 1860
   tttttggttg gcactcttac ttaccggagc cagacaaaca ctttagccat tattgaaagt 1920
   ggaggtggga tattacggaa tgtgtccagc ttgatagcta caaatgagga ccacaggcaa 1980
   atcctaagag agaacaactg tctacaaact ttattacaac acttaaaatc tcatagtttg 2040
   acaatagtca gtaatgcatg tggaactttg tggaatctct cagcaagaaa tcctaaagac 2100
   caggaagcat tatgggacat gggggcagtt agcatgctca agaacctcat tcattcaaag 2160
   cacaaaatga ttgctatggg aagtgctgca gctttaagga atctcatggc aaataggcct 2220
   gcgaagtaca aggatgccaa tattatgtct cctggctcaa gcttgccatc tcttcatgtt 2280
   aggaaacaaa aagccctaga agcagaatta gatgctcagc acttatcaga aacttttgac 2340
   aatatagaca atttaagtcc caaggcatct catcgtagta agcagagaca caagcaaagt 2400
   ctctatggtg attatgtttt tgacaccaat cgacatgatg ataataggtc agacaatttt 2460
   aatactggca acatgactgt cctttcacca tatttgaata ctacagtgtt acccagctcc 2520
   tcttcatcaa gaggaagctt agatagttct cgttctgaaa aagatagaag tttggagaga 2580
   gaacgcggaa ttggtctagg caactaccat ccagcaacag aaaatccagg aacttcttca 2640
   aagcgaggtt tgcagatctc caccactgca gcccagattg ccaaagtcat ggaagaagtg 2700
   tcagccattc atacctctca ggaagacaga agttctgggt ctaccactga attacattgt 2760
   gtgacagatg agagaaatgc acttagaaga agctctgctg cccatacaca ttcaaacact 2820
   tacaatttca ctaagtcgga aaattcaaat aggacatgtt ctatgcctta tgccaaatta 2880
   gaatacaaga gatcttcaaa tgatagttta aatagtgtca gtagtagtga tggttatggt 2940
   aaaagaggtc aaatgaaacc ctcgattgaa tcctattctg aagatgatga aagtaagttt 3000
   tgcagttatg gtcaataccc agccgaccta gcccataaaa tacatagtgc aaatcatatg 3060
   gatgataatg atggagaact agatacacca ataaattata gtcttaaata ttcagatgag 3120
   cagttgaact ctggaaggca aagtccttca cagaatgaaa gatgggcaag acccaaacac 3180
   ataatagaag atgaaataaa acaaagtgag caaagacaat caaggaatca aagtacaact 3240
   tatcctgttt atactgagag cactgatgat aaacacctca agttccaacc acattttgga 3300
   cagcaggaat gtgtttctcc atacaggtca cggggagcca atggttcaga aacaaatcga 3360
   gtgggttcta atcatggaat taatcaaaat gtaagccagt ctttgtgtca agaagatgac 3420
   tatgaagatg ataagcctac caattatagt gaacgttact ctgaagaaga acagcatgaa 3480
   gaagaagaga gaccaacaaa ttatagcata aaatataatg aagagaaacg tcatgtggat 3540
   cagcctattg attatagttt aaaatatgcc acagatattc cttcatcaca gaaacagtca 3600
   ttttcattct caaagagttc atctggacaa agcagtaaaa ccgaacatat gtcttcaagc 3660
   agtgagaata cgtccacacc ttcatctaat gccaagaggc agaatcagct ccatccaagt 3720
   tctgcacaga gtagaagtgg tcagcctcaa aaggctgcca cttgcaaagt ttcttctatt 3780
   aaccaagaaa caatacagac ttattgtgta gaagatactc caatatgttt ttcaagatgt 3840
   agttcattat catctttgtc atcagctgaa gatgaaatag gatgtaatca gacgacacag 3900
   gaagcagatt ctgctaatac cctgcaaata gcagaaataa aagaaaagat tggaactagg 3960
   tcagctgaag atcctgtgag cgaagttcca gcagtgtcac agcaccctag aaccaaatcc 4020
   agcagactgc agggttctag tttatcttca gaatcagcca ggcacaaagc tgttgaattt 4080
   tcttcaggag cgaaatctcc ctccaaaagt ggtgctcaga cacccaaaag tccacctgaa 4140
   cactatgttc aggagacccc actcatgttt agcagatgta cttctgtcag ttcacttgat 4200
   agttttgaga gtcgttcgat tgccagctcc gttcagagtg aaccatgcag tggaatggta 4260
   agtggcatta taagccccag tgatcttcca gatagccctg gacaaaccat gccaccaagc 4320
   agaagtaaaa cacctccacc acctcctcaa acagctcaaa ccaagcgaga agtacctaaa 4380
   aataaagcac ctactgctga aaagagagag agtggaccta agcaagctgc agtaaatgct 4440
   gcagttcaga gggtccaggt tcttccagat gctgatactt tattacattt tgccacggaa 4500
   agtactccag atggattttc ttgttcatcc agcctgagtg ctctgagcct cgatgagcca 4560
   tttatacaga aagatgtgga attaagaata atgcctccag ttcaggaaaa tgacaatggg 4620
   aatgaaacag aatcagagca gcctaaagaa tcaaatgaaa accaagagaa agaggcagaa 4680
   aaaactattg attctgaaaa ggacctatta gatgattcag atgatgatga tattgaaata 4740
   ctagaagaat gtattatttc tgccatgcca acaaagtcat cacgtaaagc aaaaaagcca 4800
   gcccagactg cttcaaaatt acctccacct gtggcaagga aaccaagtca gctgcctgtg 4860
   tacaaacttc taccatcaca aaacaggttg caaccccaaa agcatgttag ttttacaccg 4920
   ggggatgata tgccacgggt gtattgtgtt gaagggacac ctataaactt ttccacagct 4980
   acatctctaa gtgatctaac aatcgaatcc cctccaaatg agttagctgc tggagaagga 5040
   gttagaggag gagcacagtc aggtgaattt gaaaaacgag ataccattcc tacagaaggc 5100
   agaagtacag atgaggctca aggaggaaaa acctcatctg taaccatacc tgaattggat 5160
   gacaataaag cagaggaagg tgatattctt gcagaatgca ttaattctgc tatgcccaaa 5220
   gggaaaagtc acaagccttt ccgtgtgaaa aagataatgg accaggtcca gcaagcatct 5280
   gcgtcgtctt ctgcacccaa caaaaatcag ttagatggta agaaaaagaa accaacttca 5340
   ccagtaaaac ctataccaca aaatactgaa tataggacac gtgtaagaaa aaatgcagac 5400
   tcaaaaaata atttaaatgc tgagagagtt ttctcagaca acaaagattc aaagaaacag 5460
   aatttgaaaa ataattccaa ggacttcaat gataagctcc caaataatga agatagagtc 5520
   agaggaagtt ttgcttttga ttcacctcat cattacacgc ctattgaagg aactccttac 5580
   tgtttttcac gaaatgattc tttgagttct ctagattttg atgatgatga tgttgacctt 5640
   tccagggaaa aggctgaatt aagaaaggca aaagaaaata aggaatcaga ggctaaagtt 5700
   accagccaca cagaactaac ctccaaccaa caatcagcta ataagacaca agctattgca 5760
   aagcagccaa taaatcgagg tcagcctaaa cccatacttc agaaacaatc cacttttccc 5820
   cagtcatcca aagacatacc agacagaggg gcagcaactg atgaaaagtt acagaatttt 5880
   gctattgaaa atactccagt ttgcttttct cataattcct ctctgagttc tctcagtgac 5940
   attgaccaag aaaacaacaa taaagaaaat gaacctatca aagagactga gccccctgac 6000
   tcacagggag aaccaagtaa acctcaagca tcaggctatg ctcctaaatc atttcatgtt 6060
   gaagataccc cagtttgttt ctcaagaaac agttctctca gttctcttag tattgactct 6120
   gaagatgacc tgttgcagga atgtataagc tccgcaatgc caaaaaagaa aaagccttca 6180
   agactcaagg gtgataatga aaaacatagt cccagaaata tgggtggcat attaggtgaa 6240
   gatctgacac ttgatttgaa agatatacag agaccagatt cagaacatgg tctatcccct 6300
   gattcagaaa attttgattg gaaagctatt caggaaggtg caaattccat agtaagtagt 6360
   ttacatcaag ctgctgctgc tgcatgttta tctagacaag cttcgtctga ttcagattcc 6420
   atcctttccc tgaaatcagg aatctctctg ggatcaccat ttcatcttac acctgatcaa 6480
   gaagaaaaac cctttacaag taataaaggc ccacgaattc taaaaccagg ggagaaaagt 6540
   acattggaaa ctaaaaagat agaatctgaa agtaaaggaa tcaaaggagg aaaaaaagtt 6600
   tataaaagtt tgattactgg aaaagttcga tctaattcag aaatttcagg ccaaatgaaa 6660
   cagccccttc aagcaaacat gccttcaatc tctcgaggca ggacaatgat tcatattcca 6720
   ggagttcgaa atagctcctc aagtacaagt cctgtttcta aaaaaggccc accccttaag 6780
   actccagcct ccaaaagccc tagtgaaggt caaacagcca ccacttctcc tagaggagcc 6840
   aagccatctg tgaaatcaga attaagccct gttgccaggc agacatccca aataggtggg 6900
   tcaagtaaag caccttctag atcaggatct agagattcga ccccttcaag acctgcccag 6960
   caaccattaa gtagacctat acagtctcct ggccgaaact caatttcccc tggtagaaat 7020
   ggaataagtc ctcctaacaa attatctcaa cttccaagga catcatcccc tagtactgct 7080
   tcaactaagt cctcaggttc tggaaaaatg tcatatacat ctccaggtag acagatgagc 7140
   caacagaacc ttaccaaaca aacaggttta tccaagaatg ccagtagtat tccaagaagt 7200
   gagtctgcct ccaaaggact aaatcagatg aataatggta atggagccaa taaaaaggta 7260
   gaactttcta gaatgtcttc aactaaatca agtggaagtg aatctgatag atcagaaaga 7320
   cctgtattag tacgccagtc aactttcatc aaagaagctc caagcccaac cttaagaaga 7380
   aaattggagg aatctgcttc atttgaatct ctttctccat catctagacc agcttctccc 7440
   actaggtccc aggcacaaac tccagtttta agtccttccc ttcctgatat gtctctatcc 7500
   acacattcgt ctgttcaggc tggtggatgg cgaaaactcc cacctaatct cagtcccact 7560
   atagagtata atgatggaag accagcaaag cgccatgata ttgcacggtc tcattctgaa 7620
   agtccttcta gacttccaat caataggtca ggaacctgga aacgtgagca cagcaaacat 7680
   tcatcatccc ttcctcgagt aagcacttgg agaagaactg gaagttcatc ttcaattctt 7740
   tctgcttcat cagaatccag tgaaaaagca aaaagtgagg atgaaaaaca tgtgaactct 7800
   atttcaggaa ccaaacaaag taaagaaaac caagtatccg caaaaggaac atggagaaaa 7860
   ataaaagaaa atgaattttc tcccacaaat agtacttctc agaccgtttc ctcaggtgct 7920
   acaaatggtg ctgaatcaaa gactctaatt tatcaaatgg cacctgctgt ttctaaaaca 7980
   gaggatgttt gggtgagaat tgaggactgt cccattaaca atcctagatc tggaagatct 8040
   cccacaggta atactccccc ggtgattgac agtgtttcag aaaaggcaaa tccaaacatt 8100
   aaagattcaa aagataatca ggcaaaacaa aatgtgggta atggcagtgt tcccatgcgt 8160
   accgtgggtt tggaaaatcg cctgaactcc tttattcagg tggatgcccc tgaccaaaaa 8220
   ggaactgaga taaaaccagg acaaaataat cctgtccctg tatcagagac taatgaaagt 8280
   tctatagtgg aacgtacccc attcagttct agcagctcaa gcaaacacag ttcacctagt 8340
   gggactgttg ctgccagagt gactcctttt aattacaacc caagccctag gaaaagcagc 8400
   gcagatagca cttcagctcg gccatctcag atcccaactc cagtgaataa caacacaaag 8460
   aagcgagatt ccaaaactga cagcacagaa tccagtggaa cccaaagtcc taagcgccat     8520
   tctgggtctt accttgtgac atctgtttaa aagagaggaa gaatgaaact aagaaaattc     8580
   tatgttaatt acaactgcta tatagacatt ttgtttcaaa tgaaacttta aaagactgaa     8640
   aaattttgta aataggtttg attcttgtta gagggttttt gttctggaag ccatatttga     8700
   tagtatactt tgtcttcact ggtcttattt tgggaggcac tcttgatggt taggaaaaaa     8760
   atagtaaagc caagtatgtt tgtacagtat gttttacatg tatttaaagt agcacccatc     8820
   ccaacttcct ttaattattg cttgtcttaa aataatgaac actacagata gaaaatatga     8880
   tatattgctg ttatcaatca tttctagatt ataaactgac taaacttaca tcagggaaaa     8940
   attggtattt atgcaaaaaa aaatgttttt gt                                   8972
<210> 33
   <211> 5711
   <212> DNA
   <213> Homo sapiens
<400> 33
   agctcgctga gacttcctgg accccgcacc aggctgtggg gtttctcaga taactgggcc      60
   cctgcgctca ggaggccttc accctctgct ctgggtaaag ttcattggaa cagaaagaaa     120
   tggatttatc tgctcttcgc gttgaagaag tacaaaatgt cattaatgct atgcagaaaa     180
   tcttagagtg tcccatctgt ctggagttga tcaaggaacc tgtctccaca aagtgtgacc     240
   acatattttg caaattttgc atgctgaaac ttctcaacca gaagaaaggg ccttcacagt     300
   gtcctttatg taagaatgat ataaccaaaa ggagcctaca agaaagtacg agatttagtc     360
   aacttgttga agagctattg aaaatcattt gtgcttttca gcttgacaca ggtttggagt     420
   atgcaaacag ctataatttt gcaaaaaagg aaaataactc tcctgaacat ctaaaagatg     480
   aagtttctat catccaaagt atgggctaca gaaaccgtgc caaaagactt ctacagagtg     540
   aacccgaaaa tccttccttg caggaaacca gtctcagtgt ccaactctct aaccttggaa     600
   ctgtgagaac tctgaggaca aagcagcgga tacaacctca aaagacgtct gtctacattg     660
   aattgggatc tgattcttct gaagataccg ttaataaggc aacttattgc agtgtgggag     720
   atcaagaatt gttacaaatc acccctcaag gaaccaggga tgaaatcagt ttggattctg     780
   caaaaaaggc tgcttgtgaa ttttctgaga cggatgtaac aaatactgaa catcatcaac     840
   ccagtaataa tgatttgaac accactgaga agcgtgcagc tgagaggcat ccagaaaagt     900
   atcagggtag ttctgtttca aacttgcatg tggagccatg tggcacaaat actcatgcca     960
   gctcattaca gcatgagaac agcagtttat tactcactaa agacagaatg aatgtagaaa    1020
   aggctgaatt ctgtaataaa agcaaacagc ctggcttagc aaggagccaa cataacagat    1080
   gggctggaag taaggaaaca tgtaatgata ggcggactcc cagcacagaa aaaaaggtag    1140
   atctgaatgc tgatcccctg tgtgagagaa aagaatggaa taagcagaaa ctgccatgct 1200
   cagagaatcc tagagatact gaagatgttc cttggataac actaaatagc agcattcaga 1260
   aagttaatga gtggttttcc agaagtgatg aactgttagg ttctgatgac tcacatgatg 1320
   gggagtctga atcaaatgcc aaagtagctg atgtattgga cgttctaaat gaggtagatg 1380
   aatattctgg ttcttcagag aaaatagact tactggccag tgatcctcat gaggctttaa 1440
   tatgtaaaag tgaaagagtt cactccaaat cagtagagag taatattgaa gacaaaatat 1500
   ttgggaaaac ctatcggaag aaggcaagcc tccccaactt aagccatgta actgaaaatc 1560
   taattatagg agcatttgtt actgagccac agataataca agagcgtccc ctcacaaata 1620
   aattaaagcg taaaaggaga cctacatcag gccttcatcc tgaggatttt atcaagaaag 1680
   cagatttggc agttcaaaag actcctgaaa tgataaatca gggaactaac caaacggagc 1740
   agaatggtca agtgatgaat attactaata gtggtcatga gaataaaaca aaaggtgatt 1800
   ctattcagaa tgagaaaaat cctaacccaa tagaatcact cgaaaaagaa tctgctttca 1860
   aaacgaaagc tgaacctata agcagcagta taagcaatat ggaactcgaa ttaaatatcc 1920
   acaattcaaa agcacctaaa aagaataggc tgaggaggaa gtcttctacc aggcatattc 1980
   atgcgcttga actagtagtc agtagaaatc taagcccacc taattgtact gaattgcaaa 2040
   ttgatagttg ttctagcagt gaagagataa agaaaaaaaa gtacaaccaa atgccagtca 2100
   ggcacagcag aaacctacaa ctcatggaag gtaaagaacc tgcaactgga gccaagaaga 2160
   gtaacaagcc aaatgaacag acaagtaaaa gacatgacag cgatactttc ccagagctga 2220
   agttaacaaa tgcacctggt tcttttacta agtgttcaaa taccagtgaa cttaaagaat 2280
   ttgtcaatcc tagccttcca agagaagaaa aagaagagaa actagaaaca gttaaagtgt 2340
   ctaataatgc tgaagacccc aaagatctca tgttaagtgg agaaagggtt ttgcaaactg 2400
   aaagatctgt agagagtagc agtatttcat tggtacctgg tactgattat ggcactcagg 2460
   aaagtatctc gttactggaa gttagcactc tagggaaggc aaaaacagaa ccaaataaat 2520
   gtgtgagtca gtgtgcagca tttgaaaacc ccaagggact aattcatggt tgttccaaag 2580
   ataatagaaa tgacacagaa ggctttaagt atccattggg acatgaagtt aaccacagtc 2640
   gggaaacaag catagaaatg gaagaaagtg aacttgatgc tcagtatttg cagaatacat 2700
   tcaaggtttc aaagcgccag tcatttgctc cgttttcaaa tccaggaaat gcagaagagg 2760
   aatgtgcaac attctctgcc cactctgggt ccttaaagaa acaaagtcca aaagtcactt 2820
   ttgaatgtga acaaaaggaa gaaaatcaag gaaagaatga gtctaatatc aagcctgtac 2880
   agacagttaa tatcactgca ggctttcctg tggttggtca gaaagataag ccagttgata 2940
   atgccaaatg tagtatcaaa ggaggctcta ggttttgtct atcatctcag ttcagaggca 3000
   acgaaactgg actcattact ccaaataaac atggactttt acaaaaccca tatcgtatac 3060
   caccactttt tcccatcaag tcatttgtta aaactaaatg taagaaaaat ctgctagagg 3120
   aaaactttga ggaacattca atgtcacctg aaagagaaat gggaaatgag aacattccaa 3180
   gtacagtgag cacaattagc cgtaataaca ttagagaaaa tgtttttaaa gaagccagct 3240
   caagcaatat taatgaagta ggttccagta ctaatgaagt gggctccagt attaatgaaa 3300
   taggttccag tgatgaaaac attcaagcag aactaggtag aaacagaggg ccaaaattga 3360
   atgctatgct tagattaggg gttttgcaac ctgaggtcta taaacaaagt cttcctggaa 3420
   gtaattgtaa gcatcctgaa ataaaaaagc aagaatatga agaagtagtt cagactgtta 3480
   atacagattt ctctccatat ctgatttcag ataacttaga acagcctatg ggaagtagtc 3540
   atgcatctca ggtttgttct gagacacctg atgacctgtt agatgatggt gaaataaagg 3600
   aagatactag ttttgctgaa aatgacatta aggaaagttc tgctgttttt agcaaaagcg 3660
   tccagaaagg agagcttagc aggagtccta gccctttcac ccatacacat ttggctcagg 3720
   gttaccgaag aggggccaag aaattagagt cctcagaaga gaacttatct agtgaggatg 3780
   aagagcttcc ctgcttccaa cacttgttat ttggtaaagt aaacaatata ccttctcagt 3840
   ctactaggca tagcaccgtt gctaccgagt gtctgtctaa gaacacagag gagaatttat 3900
   tatcattgaa gaatagctta aatgactgca gtaaccaggt aatattggca aaggcatctc 3960
   aggaacatca ccttagtgag gaaacaaaat gttctgctag cttgttttct tcacagtgca 4020
   gtgaattgga agacttgact gcaaatacaa acacccagga tcctttcttg attggttctt 4080
   ccaaacaaat gaggcatcag tctgaaagcc agggagttgg tctgagtgac aaggaattgg 4140
   tttcagatga tgaagaaaga ggaacgggct tggaagaaaa taatcaagaa gagcaaagca 4200
   tggattcaaa cttaggtgaa gcagcatctg ggtgtgagag tgaaacaagc gtctctgaag 4260
   actgctcagg gctatcctct cagagtgaca ttttaaccac tcagcagagg gataccatgc 4320
   aacataacct gataaagctc cagcaggaaa tggctgaact agaagctgtg ttagaacagc 4380
   atgggagcca gccttctaac agctaccctt ccatcataag tgactcttct gcccttgagg 4440
   acctgcgaaa tccagaacaa agcacatcag aaaaagcagt attaacttca cagaaaagta 4500
   gtgaataccc tataagccag aatccagaag gcctttctgc tgacaagttt gaggtgtctg 4560
   cagatagttc taccagtaaa aataaagaac caggagtgga aaggtcatcc ccttctaaat 4620
   gcccatcatt agatgatagg tggtacatgc acagttgctc tgggagtctt cagaatagaa 4680
   actacccatc tcaagaggag ctcattaagg ttgttgatgt ggaggagcaa cagctggaag 4740
   agtctgggcc acacgatttg acggaaacat cttacttgcc aaggcaagat ctagagggaa 4800
   ccccttacct ggaatctgga atcagcctct tctctgatga ccctgaatct gatccttctg 4860
   aagacagagc cccagagtca gctcgtgttg gcaacatacc atcttcaacc tctgcattga 4920
   aagttcccca attgaaagtt gcagaatctg cccagagtcc agctgctgct catactactg 4980
   atactgctgg gtataatgca atggaagaaa gtgtgagcag ggagaagcca gaattgacag 5040
   cttcaacaga aagggtcaac aaaagaatgt ccatggtggt gtctggcctg accccagaag 5100
   aatttatgct cgtgtacaag tttgccagaa aacaccacat cactttaact aatctaatta 5160
   ctgaagagac tactcatgtt gttatgaaaa cagatgctga gtttgtgtgt gaacggacac 5220
   tgaaatattt tctaggaatt gcgggaggaa aatgggtagt tagctatttc tgggtgaccc 5280
   agtctattaa agaaagaaaa atgctgaatg agcatgattt tgaagtcaga ggagatgtgg 5340
   tcaatggaag aaaccaccaa ggtccaaagc gagcaagaga atcccaggac agaaagatct 5400
   tcaggggcct agaaatctgt tgctatgggc ccttcaccaa catgcccaca gatcaactgg 5460
   aatggatggt acagctgtgt ggtgcttctg tggtgaagga gctttcatca ttcacccttg 5520
   gcacaggtgt ccacccaatt gtggttgtgc agccagatgc ctggacagag gacaatggct 5580
   tccatgcaat tgggcagatg tgtgaggcac ctgtggtgac ccgagagtgg gtgttggaca 5640
   gtgtagcact ctaccagtgc caggagctgg acacctacct gataccccag atcccccaca 5700
   gccactactg a 5711
<210> 34
   <211> 2680
   <212> DNA
   <213> Homo sapiens
<400> 34
   ggttatcctg aatacatgtc taacaatttt ccttgcaacg ttagctgttg tttttcactg      60
   tttccaaagg atcaaaattg cttcagaaat tggagacata tttgatttaa aaggaaaaac     120
   ttgaacaaat ggacaatatg tctattacga atacaccaac aagtaatgat gcctgtctga     180
   gcattgtgca tagtttgatg tgccatagac aaggtggaga gagtgaaaca tttgcaaaaa     240
   gagcaattga aagtttggta aagaagctga aggagaaaaa agatgaattg gattctttaa     300
   taacagctat aactacaaat ggagctcatc ctagtaaatg tgttaccata cagagaacat     360
   tggatgggag gcttcaggtg gctggtcgga aaggatttcc tcatgtgatc tatgcccgtc     420
   tctggaggtg gcctgatctt cacaaaaatg aactaaaaca tgttaaatat tgtcagtatg     480
   cgtttgactt aaaatgtgat agtgtctgtg tgaatccata tcactacgaa cgagttgtat     540
   cacctggaat tgatctctca ggattaacac tgcagagtaa tgctccatca agtatgatgg     600
   tgaaggatga atatgtgcat gactttgagg gacagccatc gttgtccact gaaggacatt     660
   caattcaaac catccagcat ccaccaagta atcgtgcatc gacagagaca tacagcaccc     720
   cagctctgtt agccccatct gagtctaatg ctaccagcac tgccaacttt cccaacattc     780
   ctgtggcttc cacaagtcag cctgccagta tactgggggg cagccatagt gaaggactgt     840
   tgcagatagc atcagggcct cagccaggac agcagcagaa tggatttact ggtcagccag 900
   ctacttacca tcataacagc actaccacct ggactggaag taggactgca ccatacacac 960
   ctaatttgcc tcaccaccaa aacggccatc ttcagcacca cccgcctatg ccgccccatc 1020
   ccggacatta ctggcctgtt cacaatgagc ttgcattcca gcctcccatt tccaatcatc 1080
   ctgctcctga gtattggtgt tccattgctt actttgaaat ggatgttcag gtaggagaga 1140
   catttaaggt tccttcaagc tgccctattg ttactgttga tggatacgtg gacccttctg 1200
   gaggagatcg cttttgtttg ggtcaactct ccaatgtcca caggacagaa gccattgaga 1260
   gagcaaggtt gcacataggc aaaggtgtgc agttggaatg taaaggtgaa ggtgatgttt 1320
   gggtcaggtg ccttagtgac cacgcggtct ttgtacagag ttactactta gacagagaag 1380
   ctgggcgtgc acctggagat gctgttcata agatctaccc aagtgcatat ataaaggtct 1440
   ttgatttgcg tcagtgtcat cgacagatgc agcagcaggc ggctactgca caagctgcag 1500
   cagctgccca ggcagcagcc gtggcaggaa acatccctgg cccaggatca gtaggtggaa 1560
   tagctccagc tatcagtctg tcagctgctg ctggaattgg tgttgatgac cttcgtcgct 1620
   tatgcatact caggatgagt tttgtgaaag gctggggacc ggattaccca agacagagca 1680
   tcaaagaaac accttgctgg attgaaattc acttacaccg ggccctccag ctcctagacg 1740
   aagtacttca taccatgccg attgcagacc cacaaccttt agactgaggt cttttaccgt 1800
   tggggccctt aaccttatca ggatggtgga ctacaaaata caatcctgtt tataatctga 1860
   agatatattt cacttttctt ctgctttatc ttttcataaa gggttgaaaa tgtgtttgct 1920
   gccttgctcc tagcagacag aaactggatt aaaacaattt ttttttcctc ttcagaactt 1980
   gtcaggcatg gctcagagct tgaagattag gagaaacaca ttcttattaa ttcttcacct 2040
   gttatgtatg aaggaatcat tccagtgcta gaaaatttag ccctttaaaa cgtcttagag 2100
   ccttttatct gcagaacatc gatatgtata tcattctaca gaataatcca gtattgctga 2160
   ttttaaaggc agagaagttc tcaaagttaa ttcacctatg ttattttgtg tacaagttgt 2220
   tattgttgaa catacttcaa aaataatgtg ccatgtgggt gagttaattt taccaagagt 2280
   aactttactc tgtgtttaaa aatgaagtta ataatgtatt gtaatctttc atccaaaata 2340
   ttttttgcaa gttatattag tgaagatggt ttcaattcag attgtcttgc aacttcagtt 2400
   ttatttttgc caaggcaaaa aactcttaat ctgtgtgtat attgagaatc ccttaaaatt 2460
   accagacaaa aaaatttaaa attacgtttg ttattcctag tggatgactg ttgatgaagt 2520
   atacttttcc cctgttaaac agtagttgta ttcttctgta tttctaggca caaggttggt 2580
   tgctaagaag cctataagag gaatttcttt tccttcattc atagggaaag gttttgtatt 2640
   ttttaaaaca ctaaaagcag cgtcactcta cctaatgtct 2680
<210> 35
   <211> 1095
   <212> DNA
   <213> Homo sapiens
<400> 35
   tccccgctct gctctgtccg gtcacaggac tttttgccct ctgttcccgg gtccctcagg      60
   cggccaccca gtgggcacac tcccaggcgg cgctccggcc ccgcgctccc tccctctgcc     120
   tttcattccc agctgtcaac atcctggaag ctttgaagct caggaaagaa gagaaatcca     180
   ctgagaacag tctgtaaagg tccgtagtgc tatctacatc cagacggtgg aagggagaga     240
   aagagaaaga aggtatccta ggaatacctg cctgcttaga ccctctataa aagctctgtg     300
   catcctgcca ctgaggactc cgaagaggta gcagtcttct gaaagacttc aactgtgagg     360
   acatgtcgtt cagatttggc caacatctca tcaagccctc tgtagtgttt ctcaaaacag     420
   aactgtcctt cgctcttgtg aataggaaac ctgtggtacc aggacatgtc cttgtgtgcc     480
   cgctgcggcc agtggagcgc ttccatgacc tgcgtcctga tgaagtggcc gatttgtttc     540
   agacgaccca gagagtcggg acagtggtgg aaaaacattt ccatgggacc tctctcacct     600
   tttccatgca ggatggcccc gaagccggac agactgtgaa gcacgttcac gtccatgttc     660
   ttcccaggaa ggctggagac tttcacagga atgacagcat ctatgaggag ctccagaaac     720
   atgacaagga ggactttcct gcctcttgga gatcagagga ggaaatggca gcagaagccg     780
   cagctctgcg ggtctacttt cagtgacaca gatgtttttc agatcctgaa ttccagcaaa     840
   agagctattg ccaaccagtt tgaagaccgc ccccccgcct ctccccaaga ggaactgaat     900
   cagcatgaaa atgcagtttc ttcatctcac catcctgtat tcttcaacca gtgatccccc     960
   acctcggtca ctccaactcc cttaaaatac ctagacctaa acggctcaga caggcagatt    1020
   tgaggtttcc ccctgtctcc ttattcggca gccttatgat taaacttcct tctctgctgc    1080
   aaaaaaaaaa aaaaa                                                     1095
<210> 36
   <211> 2234
   <212> DNA
   <213> Homo sapiens
<400> 36
   aggggacgca gcgaaaccgg ggcccgcgcc aggccagccg ggacggacgc cgatgcccgg       60
   ggctgcgacg gctgcagagc gagctgccct cggaggccgg cgtggggaag atggcccagt      120
   ccaccgccac ctcccctgat gggggcacca cgtttgagca cctctggagc tctctggaac      180
   cagacagcac ctacttcgac cttccccagt caagccgggg gaataatgag gtggtgggcg      240
   gaacggattc cagcatggac gtcttccacc tggagggcat gactacatct gtcatggccc      300
   agttcaatct gctgagcagc accatggacc agatgagcag ccgcgcggcc tcggccagcc      360
   cctacacccc agagcacgcc gccagcgtgc ccacccactc gccctacgca caacccagct      420
   ccaccttcga caccatgtcg ccggcgcctg tcatcccctc caacaccgac taccccggac      480
   cccaccactt tgaggtcact ttccagcagt ccagcacggc caagtcagcc acctggacgt      540
   actccccgct cttgaagaaa ctctactgcc agatcgccaa gacatgcccc atccagatca      600
   aggtgtccac cccgccaccc ccaggcactg ccatccgggc catgcctgtt tacaagaaag      660
   cggagcacgt gaccgacgtc gtgaaacgct gccccaacca cgagctcggg agggacttca      720
   acgaaggaca gtctgctcca gccagccacc tcatccgcgt ggaaggcaat aatctctcgc      780
   agtatgtgga tgaccctgtc accggcaggc agagcgtcgt ggtgccctat gagccaccac      840
   aggtggggac ggaattcacc accatcctgt acaacttcat gtgtaacagc agctgtgtag      900
   ggggcatgaa ccggcggccc atcctcatca tcatcaccct ggagatgcgg gatgggcagg      960
   tgctgggccg ccggtccttt gagggccgca tctgcgcctg tcctggccgc gaccgaaaag     1020
   ctgatgagga ccactaccgg gagcagcagg ccctgaacga gagctccgcc aagaacgggg     1080
   ccgccagcaa gcgtgccttc aagcagagcc cccctgccgt ccccgccctt ggtgccggtg     1140
   tgaagaagcg gcggcatgga gacgaggaca cgtactacct tcaggtgcga ggccgggaga     1200
   actttgagat cctgatgaag ctgaaagaga gcctggagct gatggagttg gtgccgcagc     1260
   cactggtgga ctcctatcgg cagcagcagc agctcctaca gaggccgagt cacctacagc     1320
   ccccgtccta cgggccggtc ctctcgccca tgaacaaggt gcacgggggc atgaacaagc     1380
   tgccctccgt caaccagctg gtgggccagc ctcccccgca cagttcggca gctacaccca     1440
   acctggggcc cgtgggcccc gggatgctca acaaccatgg ccacgcagtg ccagccaacg     1500
   gcgagatgag cagcagccac agcgcccagt ccatggtctc ggggtcccac tgcactccgc     1560
   caccccccta ccacgccgac cccagcctcg tcagtttttt aacaggattg gggtgtccaa     1620
   actgcatcga gtatttcacc tcccaagggt tacagagcat ttaccacctg cagaacctga     1680
   ccattgagga cctgggggcc ctgaagatcc ccgagcagta ccgcatgacc atctggcggg     1740
   gcctgcagga cctgaagcag ggccacgact acagcaccgc gcagcagctg ctccgctcta     1800
   gcaacgcggc caccatctcc atcggcggct caggggaact gcagcgccag cgggtcatgg     1860
   aggccgtgca cttccgcgtg cgccacacca tcaccatccc caaccgcggc ggcccaggcg     1920
   gcggccctga cgagtgggcg gacttcggct tcgacctgcc cgactgcaag gcccgcaagc     1980
   agcccatcaa ggaggagttc acggaggccg agatccactg agggcctcgc ctggctgcag     2040
   cctgcgccac cgcccagaga cccaagctgc ctcccctctc cttcctgtgt gtccaaaact     2100
   gcctcaggag gcaggacctt cgggctgtgc ccggggaaag gcaaggtccg gcccatcccc     2160
   aggcacctca caggccccag gaaaggccca gccaccgaag ccgcctgtgg acagcctgag     2220
   tcacctgcag aacc 2234
<210> 37
   <211> 4344
   <212> DNA
   <213> Homo sapiens
<400> 37
   atggcctcgg ctggtaacgc cgccgagccc caggaccgcg gcggcggcgg cagcggctgt      60
   atcggtgccc cgggacggcc ggctggaggc gggaggcgca gacggacggg ggggctgcgc     120
   cgtgctgccg cgccggaccg ggactatctg caccggccca gctactgcga cgccgccttc     180
   gctctggagc agatttccaa ggggaaggct actggccgga aagcgccact gtggctgaga     240
   gcgaagtttc agagactctt atttaaactg ggttgttaca ttcaaaaaaa ctgcggcaag     300
   ttcttggttg tgggcctcct catatttggg gccttcgcgg tgggattaaa agcagcgaac     360
   ctcgagacca acgtggagga gctgtgggtg gaagttggag gacgagtaag tcgtgaatta     420
   aattatactc gccagaagat tggagaagag gctatgttta atcctcaact catgatacag     480
   acccctaaag aagaaggtgc taatgtcctg accacagaag cgctcctaca acacctggac     540
   tcggcactcc aggccagccg tgtccatgta tacatgtaca acaggcagtg gaaattggaa     600
   catttgtgtt acaaatcagg agagcttatc acagaaacag gttacatgga tcagataata     660
   gaatatcttt acccttgttt gattattaca cctttggact gcttctggga aggggcgaaa     720
   ttacagtctg ggacagcata cctcctaggt aaacctcctt tgcggtggac aaacttcgac     780
   cctttggaat tcctggaaga gttaaagaaa ataaactatc aagtggacag ctgggaggaa     840
   atgctgaata aggctgaggt tggtcatggt tacatggacc gcccctgcct caatccggcc     900
   gatccagact gccccgccac agcccccaac aaaaattcaa ccaaacctct tgatatggcc     960
   cttgttttga atggtggatg tcatggctta tccagaaagt atatgcactg gcaggaggag    1020
   ttgattgtgg gtggcacagt caagaacagc actggaaaac tcgtcagcgc ccatgccctg    1080
   cagaccatgt tccagttaat gactcccaag caaatgtacg agcacttcaa ggggtacgag    1140
   tatgtctcac acatcaactg gaacgaggac aaagcggcag ccatcctgga ggcctggcag    1200
   aggacatatg tggaggtggt tcatcagagt gtcgcacaga actccactca aaaggtgctt    1260
   tccttcacca ccacgaccct ggacgacatc ctgaaatcct tctctgacgt cagtgtcatc    1320
   cgcgtggcca gcggctactt actcatgctc gcctatgcct gtctaaccat gctgcgctgg    1380
   gactgctcca agtcccaggg tgccgtgggg ctggctggcg tcctgctggt tgcactgtca    1440
   gtggctgcag gactgggcct gtgctcattg atcggaattt cctttaacgc tgcaacaact    1500
   caggttttgc catttctcgc tcttggtgtt ggtgtggatg atgtttttct tctggcccac    1560
   gccttcagtg aaacaggaca gaataaaaga atccctttgg aggacaggac cggggagtgc    1620
   ctgaagcgca caggagccag cgtggccctc acgtccatca gcaatgtcac agccttcttc 1680
   atggccgcgt taatcccaat tcccgctctg cgggcgttct ccctccaggc agcggtagta 1740
   gtggtgttca attttgccat ggttctgctc atttttcctg caattctcag catggattta 1800
   tatcgacgcg aggacaggag actggatatt ttctgctgtt ttacaagccc ctgcgtcagc 1860
   agagtgattc aggttgaacc tcaggcctac accgacacac acgacaatac ccgctacagc 1920
   cccccacctc cctacagcag ccacagcttt gcccatgaaa cgcagattac catgcagtcc 1980
   actgtccagc tccgcacgga gtacgacccc cacacgcacg tgtactacac caccgctgag 2040
   ccgcgctccg agatctctgt gcagcccgtc accgtgacac aggacaccct cagctgccag 2100
   agcccagaga gcaccagctc cacaagggac ctgctctccc agttctccga ctccagcctc 2160
   cactgcctcg agcccccctg tacgaagtgg acactctcat cttttgctga gaagcactat 2220
   gctcctttcc tcttgaaacc aaaagccaag gtagtggtga tcttcctttt tctgggcttg 2280
   ctgggggtca gcctttatgg caccacccga gtgagagacg ggctggacct tacggacatt 2340
   gtacctcggg aaaccagaga atatgacttt attgctgcac aattcaaata cttttctttc 2400
   tacaacatgt atatagtcac ccagaaagca gactacccga atatccagca cttactttac 2460
   gacctacaca ggagtttcag taacgtgaag tatgtcatgt tggaagaaaa caaacagctt 2520
   cccaaaatgt ggctgcacta cttcagagac tggcttcagg gacttcagga tgcatttgac 2580
   agtgactggg aaaccgggaa aatcatgcca aacaattaca agaatggatc agacgatgga 2640
   gtccttgcct acaaactcct ggtgcaaacc ggcagccgcg ataagcccat cgacatcagc 2700
   cagttgacta aacagcgtct ggtggatgca gatggcatca ttaatcccag cgctttctac 2760
   atctacctga cggcttgggt cagcaacgac cccgtcgcgt atgctgcctc ccaggccaac 2820
   atccggccac accgaccaga atgggtccac gacaaagccg actacatgcc tgaaacaagg 2880
   ctgagaatcc cggcagcaga gcccatcgag tatgcccagt tccctttcta cctcaacggg 2940
   ttgcgggaca cctcagactt tgtggaggca attgaaaaag taaggaccat ctgcagcaac 3000
   tatacgagcc tggggctgtc cagttacccc aacggctacc ccttcctctt ctgggagcag 3060
   tacatcggcc tccgccactg gctgctgctg ttcatcagcg tggtgttggc ctgcacattc 3120
   ctcgtgtgcg ctgtcttcct tctgaacccc tggacggccg ggatcattgt gatggtcctg 3180
   gcgctgatga cggtcgagct gttcggcatg atgggcctca tcggaatcaa gctcagtgcc 3240
   gtgcccgtgg tcatcctgat cgcttctgtt ggcataggag tggagttcac cgttcacgtt 3300
   gctttggcct ttctgacggc catcggcgac aagaaccgca gggctgtgct tgccctggag 3360
   cacatgtttg cacccgtcct ggatggcgcc gtgtccactc tgctgggagt gctgatgctg 3420
   gcgggatctg agttcgactt cattgtcagg tatttctttg ctgtgctggc gatcctcacc 3480
   atcctcggcg ttctcaatgg gctggttttg cttcccgtgc ttttgtcttt ctttggacca 3540
   tatcctgagg tgtctccagc caacggcttg aaccgcctgc ccacaccctc ccctgagcca      3600
   ccccccagcg tggtccgctt cgccatgccg cccggccaca cgcacagcgg gtctgattcc      3660
   tccgactcgg agtatagttc ccagacgaca gtgtcaggcc tcagcgagga gcttcggcac      3720
   tacgaggccc agcagggcgc gggaggccct gcccaccaag tgatcgtgga agccacagaa      3780
   aaccccgtct tcgcccactc cactgtggtc catcccgaat ccaggcatca cccaccctcg      3840
   aacccgagac agcagcccca cctggactca gggtccctgc ctcccggacg gcaaggccag      3900
   cagccccgca gggacccccc cagagaaggc ttgtggccac ccctctacag accgcgcaga      3960
   gacgcttttg aaatttctac tgaagggcat tctggcccta gcaatagggc ccgctggggc      4020
   cctcgcgggg cccgttctca caaccctcgg aacccagcgt ccactgccat gggcagctcc      4080
   gtgcccggct actgccagcc catcaccact gtgacggctt ctgcctccgt gactgtcgcc      4140
   gtgcacccgc cgcctgtccc tgggcctggg cggaaccccc gagggggact ctgcccaggc      4200
   taccctgaga ctgaccacgg cctgtttgag gacccccacg tgcctttcca cgtccggtgt      4260
   gagaggaggg attcgaaggt ggaagtcatt gagctgcagg acgtggaatg cgaggagagg      4320
   ccccggggaa gcagctccaa ctga                                              4344
<210> 38
   <211> 4740
   <212> DNA
   <213> Homo sapiens
<400> 38
   ttccggtttt tctcagggga cgttgaaatt atttttgtaa cgggagtcgg gagaggacgg      60
   ggcgtgcccc gcgtgcgcgc gcgtcgtcct ccccggcgct cctccacagc tcgctggctc     120
   ccgccgcgga aaggcgtcat gccgcccaaa accccccgaa aaacggccgc caccgccgcc     180
   gctgccgccg cggaaccccc ggcaccgccg ccgccgcccc ctcctgagga ggacccagag     240
   caggacagcg gcccggagga cctgcctctc gtcaggcttg agtttgaaga aacagaagaa     300
   cctgatttta ctgcattatg tcagaaatta aagataccag atcatgtcag agagagagct     360
   tggttaactt gggagaaagt ttcatctgtg gatggagtat tgggaggtta tattcaaaag     420
   aaaaaggaac tgtggggaat ctgtatcttt attgcacgag ttgacctaga tgagatgtcg     480
   ttcactttac tgagctacag aaaaacatac gaaatcagtg tccataaatt ctttaactta     540
   ctaaaagaaa ttgataccag taccaaagtt gataatgcta tgtcaagact gttgaagaag     600
   tatgatgtat tgtttgcact cttcagcaaa ttggaaagga catgtgaact tatatatttg     660
   acacaaccca gcagttcgat atctactgaa ataaattctg cattggtgct aaaagtttct     720
   tggatcacat ttttattagc taaaggggaa gtattacaaa tggaagatga tctggtgatt     780
   tcatttcagt taatgctatg tgtccttgac tattttatta aactctcacc tcccatgttg     840
   ctcaaagaac catataaaac agctgttata cccattaatg gttcacctcg aacacccagg 900
   cgaggtcaga acaggagtgc acggatagca aaacaactag aaaatgatac aagaattatt 960
   gaagttctct gtaaagaaca tgaatgtaat atagatgagg tgaaaaatgt ttatttcaaa 1020
   aattttatac cttttatgaa ttctcttgga cttgtaacat ctaatggact tccagaggtt 1080
   gaaaatcttt ctaaacgata cgaagaaatt tatcttaaaa ataaagatct agatcgaaga 1140
   ttatttttgg atcatgataa aactcttcag actgattcta tagacagttt tgaaacacag 1200
   agaacaccac gaaaaagtaa ccttgatgaa gaggtgaata taattcctcc acacactcca 1260
   gttaggactg ttatgaacac tatccaacaa ttaatgatga ttttaaattc tgcaagtgat 1320
   caaccttcag aaaatctgat ttcctatttt aacaactgca cagtgaatcc aaaagaaagt 1380
   atactgaaaa gagtgaagga tataggatac atctttaaag agaaatttgc taaagctgtg 1440
   ggacagggtt gtgtcgaaat tggatcacag cgatacaaac ttggagttcg cttgtattac 1500
   cgagtaatgg aatccatgct taaatcagaa gaagaacgat tatccattca aaattttagc 1560
   aaacttctga atgacaacat ttttcatatg tctttattgg cgtgcgctct tgaggttgta 1620
   atggccacat atagcagaag tacatctcag aatcttgatt ctggaacaga tttgtctttc 1680
   ccatggattc tgaatgtgct taatttaaaa gcctttgatt tttacaaagt gatcgaaagt 1740
   tttatcaaag cagaaggcaa cttgacaaga gaaatgataa aacatttaga acgatgtgaa 1800
   catcgaatca tggaatccct tgcatggctc tcagattcac ctttatttga tcttattaaa 1860
   caatcaaagg accgagaagg accaactgat caccttgaat ctgcttgtcc tcttaatctt 1920
   cctctccaga ataatcacac tgcagcagat atgtatcttt ctcctgtaag atctccaaag 1980
   aaaaaaggtt caactacgcg tgtaaattct actgcaaatg cagagacaca agcaacctca 2040
   gccttccaga cccagaagcc attgaaatct acctctcttt cactgtttta taaaaaagtg 2100
   tatcggctag cctatctccg gctaaataca ctttgtgaac gccttctgtc tgagcaccca 2160
   gaattagaac atatcatctg gacccttttc cagcacaccc tgcagaatga gtatgaactc 2220
   atgagagaca ggcatttgga ccaaattatg atgtgttcca tgtatggcat atgcaaagtg 2280
   aagaatatag accttaaatt caaaatcatt gtaacagcat acaaggatct tcctcatgct 2340
   gttcaggaga cattcaaacg tgttttgatc aaagaagagg agtatgattc tattatagta 2400
   ttctataact cggtcttcat gcagagactg aaaacaaata ttttgcagta tgcttccacc 2460
   aggcccccta ccttgtcacc aatacctcac attcctcgaa gcccttacaa gtttcctagt 2520
   tcacccttac ggattcctgg agggaacatc tatatttcac ccctgaagag tccatataaa 2580
   atttcagaag gtctgccaac accaacaaaa atgactccaa gatcaagaat cttagtatca 2640
   attggtgaat cattcgggac ttctgagaag ttccagaaaa taaatcagat ggtatgtaac 2700
   agcgaccgtg tgctcaaaag aagtgctgaa ggaagcaacc ctcctaaacc actgaaaaaa 2760
   ctacgctttg atattgaagg atcagatgaa gcagatggaa gtaaacatct cccaggagag 2820
   tccaaatttc agcagaaact ggcagaaatg acttctactc gaacacgaat gcaaaagcag 2880
   aaaatgaatg atagcatgga tacctcaaac aaggaagaga aatgaggatc tcaggacctt 2940
   ggtggacact gtgtacacct ctggattcat tgtctctcac agatgtgact gtataacttt 3000
   cccaggttct gtttatggcc acatttaata tcttcagctc tttttgtgga tataaaatgt 3060
   gcagatgcaa ttgtttgggt gagtcctaag ccacttgaaa tgttagtcat tgttatttat 3120
   acaagattga aaatcttgtg taaatcctgc catttaaaaa gttgtagcag attgtttcct 3180
   cttccaaagt aaaattgctg tgctttatgg atagtaagaa tggccctaga gtgggagtcc 3240
   tgataaccca ggcctgtctg actactttgc cttcttttgt agcatatagg tgatgtttgc 3300
   tcttgttttt attaatttat atgtatattt ttttaattta acatgaacac ccttagaaaa 3360
   tgtgtcctat ctatcttcca aatgcaattt gattgactgc ccattcacca aaattatcct 3420
   gaactcttct gcaaaaatgg atattattag aaattagaaa aaaattacta attttacaca 3480
   ttagatttta ttttactatt ggaatctgat atactgtgtg cttgttttat aaaattttgc 3540
   ttttaattaa ataaaagctg gaagcaaagt ataaccatat gatactatca tactactgaa 3600
   acagatttca tacctcagaa tgtaaaagaa cttactgatt attttcttca tccaacttat 3660
   gtttttaaat gaggattatt gatagtactc ttggttttta taccattcag atcactgaat 3720
   ttataaagta cccatctagt acttgaaaaa gtaaagtgtt ctgccagatc ttaggtatag 3780
   aggaccctaa cacagtatat cccaagtgca ctttctaatg tttctgggtc ctgaagaatt 3840
   aagatacaaa ttaattttac tccataaaca gactgttaat tataggagcc ttaatttttt 3900
   tttcatagag atttgtctaa ttgcatctca aaattattct gccctcctta atttgggaag 3960
   gtttgtgttt tctctggaat ggtacatgtc ttccatgtat cttttgaact ggcaattgtc 4020
   tatttatctt ttattttttt aagtcagtat ggtctaacac tggcatgttc aaagccacat 4080
   tatttctagt ccaaaattac aagtaatcaa gggtcattat gggttaggca ttaatgtttc 4140
   tatctgattt tgtgcaaaag cttcaaatta aaacagctgc attagaaaaa gaggcgcttc 4200
   tcccctcccc tacacctaaa ggtgtattta aactatcttg tgtgattaac ttatttagag 4260
   atgctgtaac ttaaaatagg ggatatttaa ggtagcttca gctagctttt aggaaaatca 4320
   ctttgtctaa ctcagaatta tttttaaaaa gaaatctggt cttgttagaa aacaaaattt 4380
   tattttgtgc tcatttaagt ttcaaactta ctattttgac agttattttg ataacaatga 4440
   cactagaaaa cttgactcca tttcatcatt gtttctgcat gaatatcata caaatcagtt 4500
   agtttttagg tcaagggctt actatttctg ggtcttttgc tactaagttc acattagaat 4560
   tagtgccaga attttaggaa cttcagagat cgtgtattga gatttcttaa ataatgcttc 4620
   agatattatt gctttattgc ttttttgtat tggttaaaac tgtacattta aaattgctat 4680
   gttactattt tctacaatta atagtttgtc tattttaaaa taaattagtt gttaagagtc 4740
<210> 39
   <211> 4608
   <212> DNA
   <213> Homo sapiensl
<400> 39
   atggagaata gtcttagatg tgtttgggta cccaagctgg cttttgtact cttcggagct 60
   tccttgctca gcgcgcatct tcaagtaacc ggttttcaaa ttaaagcttt cacagcactg 120
   cgcttcctct cagaaccttc tgatgccgtc acaatgcggg gaggaaatgt cctcctcgac 180
   tgctccgcgg agtccgaccg aggagttcca gtgatcaagt ggaagaaaga tggcattcat 240
   ctggccttgg gaatggatga aaggaagcag caactttcaa atgggtctct gctgatacaa 300
   aacatacttc attccagaca ccacaagcca gatgagggac tttaccaatg tgaggcatct 360
   ttaggagatt ctggctcaat tattagtcgg acagcaaaag ttgcagtagc aggaccactg 420
   aggttccttt cacagacaga atctgtcaca gccttcatgg gagacacagt gctactcaag 480
   tgtgaagtca ttggggagcc catgccaaca atccactggc agaagaacca acaagacctg 540
   actccaatcc caggtgactc ccgagtggtg gtcttgccct ctggagcatt gcagatcagc 600
   cgactccaac cgggggacat tggaatttac cgatgctcag ctcgaaatcc agccagctca 660
   agaacaggaa atgaagcaga agtcagaatt ttatcagatc caggactgca tagacagctg 720
   tattttctgc aaagaccatc caatgtagta gccattgaag gaaaagatgc tgtcctggaa 780
   tgttgtgttt ctggctatcc tccaccaagt tttacctggt tacgaggcga ggaagtcatc 840
   caactcaggt ctaaaaagta ttctttattg ggtggaagca acttgcttat ctccaatgtg 900
   acagatgatg acagtggaat gtatacctgt gttgtcacat ataaaaatga gaatattagt 960
   gcctctgcag agctcacagt cttggttccg ccatggtttt taaatcatcc ttccaacctg 1020
   tatgcctatg aaagcatgga tattgagttt gaatgtacag tctctggaaa gcctgtgccc 1080
   actgtgaatt ggatgaagaa tggagatgtg gtcattccta gtgattattt tcagatagtg 1140
   ggaggaagca acttacggat acttggggtg gtgaagtcag atgaaggctt ttatcaatgt 1200
   gtggctgaaa atgaggctgg aaatgcccag accagtgcac agctcattgt ccctaagcct 1260
   gcaatcccaa gctccagtgt cctcccttcg gctcccagag atgtggtccc tgtcttggtt 1320
   tccagccgat ttgtccgtct cagctggcgc ccacctgcag aagcgaaagg gaacattcaa 1380
   actttcacgg tctttttctc cagagaaggt gacaacaggg aacgagcatt gaatacaaca 1440
   cagcctgggt cccttcagct cactgtggga aacctgaagc cagaagccat gtacaccttt 1500
   cgagttgtgg cttacaatga atggggaccg ggagagagtt ctcaacccat caaggtggcc 1560
   acacagcctg agttgcaagt tccagggcca gtagaaaacc tgcaagctgt atctacctca 1620
   cctacctcaa ttcttattac ctgggaaccc cctgcctatg caaacggtcc agtccaaggt 1680
   tacagattgt tctgcactga ggtgtccaca ggaaaagaac agaatataga ggttgatgga 1740
   ctatcttata aactggaagg cctgaaaaaa ttcaccgaat atagtcttcg attcttagct 1800
   tataatcgct atggtccggg cgtctctact gatgatataa cagtggttac actttctgac 1860
   gtgccaagtg ccccgcctca gaacgtctcc ctggaagtgg tcaattcaag aagtatcaaa 1920
   gttagctggc tgcctcctcc atcaggaaca caaaatggat ttattaccgg ctataaaatt 1980
   cgacacagaa agacgacccg caggggtgag atggaaacac tggagccaaa caacctctgg 2040
   tacctattca caggactgga gaaaggaagt cagtacagtt tccaggtgtc agccatgaca 2100
   gtcaatggta ctggaccacc ttccaactgg tatactgcag agactccaga gaatgatcta 2160
   gatgaatctc aagttcctga tcaaccaagc tctcttcatg tgaggcccca gactaactgc 2220
   atcatcatga gttggactcc tcccttgaac ccaaacatcg tggtgcgagg ttatattatc 2280
   ggttatggcg ttgggagccc ttacgctgag acagtgcgtg tggacagcaa gcagcgatat 2340
   tattccattg agaggttaga gtcaagttcc cattatgtaa tctccctaaa agcttttaac 2400
   aatgccggag aaggagttcc tctttatgaa agtgccacca ccaggtctat aaccgatccc 2460
   actgacccag ttgattatta tcctttgctt gatgatttcc ccacctcggt cccagatctc     2520
   tccaccccca tgctcccacc agtaggtgta caggctgtgg ctcttaccca tgatgctgtg     2580
   agggtcagct gggcagacaa ctctgtccct aagaaccaaa agacgtctga ggtgcgactt 2640
   tacaccgtcc ggtggagaac cagcttttct gcaagtgcaa aatacaagtc agaagacaca 2700
   acatctctaa gttacacagc aacaggcctc aaaccaaaca caatgtatga attctcggtc 2760
   atggtaacaa aaaacagaag gtccagtact tggagcatga ctgcacatgc caccacgtat 2820
   gaagcagccc ccacctctgc tcccaaggac tttacagtca ttactaggga agggaagcct 2880
   cgtgccgtca ttgtgagttg gcagcctccc ttggaagcca atgggaaaat tactgcttac 2940
   atcttatttt ataccttgga caagaacatc ccaattgatg actggattat ggaaacaatc 3000
   agtggtgata ggcttactca tcaaatcatg gatctcaacc ttgatactat gtattacttt 3060
   cgaattcaag cacgaaattc aaaaggagtg gggccactct ctgatcccat cctcttcagg 3120
   actctgaaag tggaacaccc tgacaaaatg gctaatgacc aaggtcgtca tggagatgga 3180
   ggttattggc cagttgatac taatttgatt gatagaagca ccctaaatga gccgccaatt     3240
   ggacaaatgc accccccgca tggcagtgtc actcctcaga agaacagcaa cctgcttgtg     3300
   atcattgtgg tcaccgttgg tgtcatcaca gtgctggtag tggtcatcgt ggctgtgatt 3360
   tgcacccgac gctcttcagc ccagcagaga aagaaacggg ccacccacag tgctggcaaa 3420
   aggaagggca gccagaagga cctccgaccc cctgatcttt ggatccatca tgaagaaatg 3480
   gagatgaaaa atattgaaaa gccatctggc actgaccctg caggaaggga ctctcccatc 3540
   caaagttgcc aagacctcac accagtcagc cacagccagt cagaaaccca actgggaagc 3600
   aaaagcacct ctcattcagg tcaagacact gaggaagcag ggagctctat gtccactctg 3660
   gagaggtcgc tggctgcacg ccgagccccc cgggccaagc tcatgattcc catggatgcc 3720
   cagtccaaca atcctgctgt cgtgagcgcc atcccggtgc caacgctaga aagtgcccag 3780
   tacccaggaa tcctcccgtc tcccacctgt ggatatcccc acccgcagtt cactctccgg 3840
   cctgtgccat tcccaacact ctcagtggac cgaggtttcg gagcaggaag aagtcagtca 3900
   gtgagtgaag gaccaactac ccaacaacca cctatgctgc ccccatctca gcctgagcat 3960
   tctagcagcg aggaggcacc aagcagaacc atccccacag cttgtgttcg accaactcac 4020
   ccactccgca gctttgctaa tcctttgcta cctccaccaa tgagtgcaat agaaccgaaa 4080
   gtcccttaca caccactttt gtctcagcca gggcccactc ttcctaagac ccatgtgaaa 4140
   acagcctccc ttgggttggc tggaaaagca agatcccctt tgcttcctgt gtctgtgcca 4200
   acagcccctg aagtgtctga ggagagccac aaaccaacag aggattcagc caatgtgtat 4260
   gaacaggatg atctgagtga acaaatggca agtttggaag gactcatgaa gcagcttaat 4320
   gccatcacag gctcagcctt ttaacatgta tttctgaatg gatgaggtga attttccggg 4380
   aactttgcag cataccaatt acccataaac agcacacctg tgtccaagaa ctctaaccag 4440
   tgtacaggtc acccatcagg accactcagt taaggaagat cctgaagcag ttcagaagga 4500
   ataagcattc cttctttcac aggcatcagg aattgtcaaa tgatgattat gagttcccta 4560
   aacaaaagca aagatgcatt ttcactgcaa tgtcaaagtt tagctgct 4608
<210> 40
   <211> 8959
   <212> DNA
   <213> Homo sapiens
<400> 40
   ccccagcctc cttgccaacg ccccctttcc ctctccccct cccgctcggc gctgaccccc      60
   catccccacc cccgtgggaa cactgggagc ctgcactcca cagaccctct ccttgcctct     120
   tccctcacct cagcctccgc tccccgccct cttcccggcc cagggcgccg gcccaccctt     180
   ccctccgccg ccccccggcc gcggggagga catggccgcg cacaggccgg tggaatgggt     240
   ccaggccgtg gtcagccgct tcgacgagca gcttccaata aaaacaggac agcagaacac     300
   acataccaaa gtcagtactg agcacaacaa ggaatgtcta atcaatattt ccaaatacaa     360
   gttttctttg gttataagcg gcctcactac tattttaaag aatgttaaca atatgagaat     420
   atttggagaa gctgctgaaa aaaatttata tctctctcag ttgattatat tggatacact     480
   ggaaaaatgt cttgctgggc aaccaaagga cacaatgaga ttagatgaaa cgatgctggt     540
   caaacagttg ctgccagaaa tctgccattt tcttcacacc tgtcgtgaag gaaaccagca     600
   tgcagctgaa cttcggaatt ctgcctctgg ggttttattt tctctcagct gcaacaactt     660
   caatgcagtc tttagtcgca tttctaccag gttacaggaa ttaactgttt gttcagaaga     720
   caatgttgat gttcatgata tagaattgtt acagtatatc aatgtggatt gtgcaaaatt     780
   aaaacgactc ctgaaggaaa cagcatttaa atttaaagcc ctaaagaagg ttgcgcagtt     840
   agcagttata aatagcctgg aaaaggcatt ttggaactgg gtagaaaatt atccagatga     900
   atttacaaaa ctgtaccaga tcccacagac tgatatggct gaatgtgcag aaaagctatt     960
   tgacttggtg gatggttttg ctgaaagcac caaacgtaaa gcagcagttt ggccactaca    1020
   aatcattctc cttatcttgt gtccagaaat aatccaggat atatccaaag acgtggttga    1080
   tgaaaacaac atgaataaga agttatttct ggacagtcta cgaaaagctc ttgctggcca    1140
   tggaggaagt aggcagctga cagaaagtgc tgcaattgcc tgtgtcaaac tgtgtaaagc    1200
   aagtacttac atcaattggg aagataactc tgtcattttc ctacttgttc agtccatggt    1260
   ggttgatctt aagaacctgc tttttaatcc aagtaagcca ttctcaagag gcagtcagcc    1320
   tgcagatgtg gatctaatga ttgactgcct tgtttcttgc tttcgtataa gccctcacaa    1380
   caaccaacac tttaagatct gcctggctca gaattcacct tctacatttc actatgtgct    1440
   ggtaaattca ctccatcgaa tcatcaccaa ttccgcattg gattggtggc ctaagattga    1500
   tgctgtgtat tgtcactcgg ttgaacttcg aaatatgttt ggtgaaacac ttcataaagc    1560
   agtgcaaggt tgtggagcac acccagcaat acgaatggca ccgagtctta catttaaaga    1620
   aaaagtaaca agccttaaat ttaaagaaaa acctacagac ctggagacaa gaagctataa    1680
   gtatcttctc ttgtccatgg tgaaactaat tcatgcagat ccaaagctct tgctttgtaa    1740
   tccaagaaaa caggggcccg aaacccaagg cagtacagca gaattaatta cagggctcgt    1800
   ccaactggtc cctcagtcac acatgccaga gattgctcag gaagcaatgg aggctctgct    1860
   ggttcttcat cagttagata gcattgattt gtggaatcct gatgctcctg tagaaacatt    1920
   ttgggagatt agctcacaaa tgctttttta catctgcaag aaattaacta gtcatcaaat    1980
   gcttagtagc acagaaattc tcaagtggtt gcgggaaata ttgatctgca ggaataaatt    2040
   tcttcttaaa aataagcagg cagatagaag ttcctgtcac tttctccttt tttacggggt    2100
   aggatgtgat attccttcta gtggaaatac cagtcaaatg tccatggatc atgaagaatt    2160
   actacgtact cctggagcct ctctccggaa gggaaaaggg aactcctcta tggatagtgc    2220
   agcaggatgc agcggaaccc ccccaatttg ccgacaagcc cagaccaaac tagaagtggc    2280
   cctgtacatg tttctgtgga accctgacac tgaagctgtt ctggttgcca tgtcctgttt    2340
   ccgccacctc tgtgaggaag cagatatccg gtgtggggtg gatgaagtgt cagtgcataa    2400
   cctcttgccc aactataaca cattcatgga gtttgcctct gtcagcaata tgatgtcaac 2460
   aggaagagca gcacttcaga aaagagtgat ggcactgctg aggcgcattg agcatcccac 2520
   tgcaggaaac actgaggctt gggaagatac acatgcaaaa tgggaacaag caacaaagct 2580
   aatccttaac tatccaaaag ccaaaatgga agatggccag gctgctgaaa gccttcacaa 2640
   gaccattgtt aagaggcgaa tgtcccatgt gagtggagga ggatccatag atttgtctga 2700
   cacagactcc ctacaggaat ggatcaacat gactggcttc ctttgtgccc ttggaggagt 2760
   gtgcctccag cagagaagca attctggcct ggcaacctat agcccaccca tgggtccagt 2820
   cagtgaacgt aagggttcta tgatttcagt gatgtcttca gagggaaacg cagatacacc 2880
   tgtcagcaaa tttatggatc ggctgttgtc cttaatggtg tgtaaccatg agaaagtggg 2940
   acttcaaata cggaccaatg ttaaggatct ggtgggtcta gaattgagtc ctgctctgta 3000
   tccaatgcta tttaacaaat tgaagaatac catcagcaag ttttttgact cccaaggaca 3060
   ggttttattg actgatacca atactcaatt tgtagaacaa accatagcta taatgaagaa 3120
   cttgctagat aatcatactg aaggcagctc tgaacatcta gggcaagcta gcattgaaac 3180
   aatgatgtta aatctggtca ggtatgttcg tgtgcttggg aatatggtcc atgcaattca 3240
   aataaaaacg aaactgtgtc aattagttga agtaatgatg gcaaggagag atgacctctc 3300
   attttgccaa gagatgaaat ttaggaataa gatggtagaa tacctgacag actgggttat 3360
   gggaacatca aaccaagcag cagatgatga tgtaaaatgt cttacaagag atttggacca 3420
   ggcaagcatg gaagcagtag tttcacttct agctggtctc cctctgcagc ctgaagaagg 3480
   agatggtgtg gaattgatgg aagccaaatc acagttattt cttaaatact tcacattatt 3540
   tatgaacctt ttgaatgact gcagtgaagt tgaagatgaa agtgcgcaaa caggtggcag 3600
   gaaacgtggc atgtctcgga ggctggcatc actgaggcac tgtacggtcc ttgcaatgtc 3660
   aaacttactc aatgccaacg tagacagtgg tctcatgcac tccataggct taggttacca 3720
   caaggatctc cagacaagag ctacatttat ggaagttctg acaaaaatcc ttcaacaagg 3780
   cacagaattt gacacacttg cagaaacagt attggctgat cggtttgaga gattggtgga 3840
   actggtcaca atgatgggtg atcaaggaga actccctata gcgatggctc tggccaatgt 3900
   ggttccttgt tctcagtggg atgaactagc tcgagttctg gttactctgt ttgattctcg 3960
   gcatttactc taccaactgc tctggaacat gttttctaaa gaagtagaat tggcagactc 4020
   catgcagact ctcttccgag gcaacagctt ggccagtaaa ataatgacat tctgtttcaa 4080
   ggtatatggt gctacctatc tacaaaaact cctggatcct ttattacgaa ttgtgatcac 4140
   atcctctgat tggcaacatg ttagctttga agtggatcct accaggttag aaccatcaga 4200
   gagccttgag gaaaaccagc ggaacctcct tcagatgact gaaaagttct tccatgccat 4260
   catcagttcc tcctcagaat tcccccctca acttcgaagt gtgtgccact gtttatacca 4320
   ggtggttagc cagcgtttcc ctcagaacag catcggtgca gtaggaagtg ccatgttcct 4380
   cagatttatc aatcctgcca ttgtctcacc gtatgaagca gggattttag ataaaaagcc 4440
   accacctaga atcgaaaggg gcttgaagtt aatgtcaaag atacttcaga gtattgccaa 4500
   tcatgttctc ttcacaaaag aagaacatat gcggcctttc aatgattttg tgaaaagcaa 4560
   ctttgatgca gcacgcaggt tttccttgat tatagcatct gattgtccta caagtgatgc 4620
   agtaaatcat agtctttcct tcataagtga cggcaatgtg cttgctttac atcgtctact 4680
   ctggaacaat caggagaaaa ttgggcagta tctttccagc aacagggatc ataaagctgt 4740
   tggaagacga ccttttgata agatggcaac acttcttgca tacctgggtc ctccagagca 4800
   caaacctgtg gcagatacac actggtccag ccttaacctt accagttcaa agtttgagga 4860
   atttatgact aggcatcagg tacatgaaaa agaagaattc aaggctttga aaacgttaag 4920
   tattttctac caagctggga cttccaaagc tgggaatcct attttttatt atgttgcacg 4980
   gaggttcaaa actggtcaaa tcaatggtga tttgctgata taccatgtct tactgacttt 5040
   aaagccatat tatgcaaagc catatgaaat tgtagtggac cttacccata ccgggcctag 5100
   caatcgcttt aaaacagact ttctctctaa gtggtttgtt gtttttcctg gctttgctta 5160
   cgacaacgtc tccgcagtct atatctataa ctgtaactcc tgggtcaggg agtacaccaa 5220
   gtatcatgag cggctgctga ctggcctcaa aggtagcaaa aggcttgttt tcatagactg 5280
   tcctgggaaa ctggctgagc acatagagca tgaacaacag aaactacctg ctgccacctt 5340
   ggctttagaa gaggacctga aggtattcca caatgctctc aagctagctc acaaagacac 5400
   caaagtttct attaaagttg gttctactgc tgtccaagta acttcagcag agcgaacaaa 5460
   agtcctaggg caatcagtct ttctaaatga catttattat gcttcggaaa ttgaagaaat 5520
   ctgcctagta gatgagaacc agttcacctt aaccattgca aaccagggca cgccgctcac 5580
   cttcatgcac caggagtgtg aagccattgt ccagtctatc attcatatcc ggacccgctg 5640
   ggaactgtca cagcccgact ctatccccca acacaccaag attcggccaa aagatgtccc 5700
   tgggacactg ctcaatatcg cattacttaa tttaggcagt tctgacccga gtttacggtc 5760
   agctgcctat aatcttctgt gtgccttaac ttgtaccttt aatttaaaaa tcgagggcca 5820
   gttactagag acatcaggtt tatgtatccc tgccaacaac accctcttta ttgtctctat 5880
   tagtaagaca ctggcagcca atgagccaca cctcacgtta gaatttttgg aagagtgtat 5940
   ttctggattt agcaaatcta gtattgaatt gaaacacctt tgtttggaat acatgactcc 6000
   atggctgtca aatctagttc gtttttgcaa gcataatgat gatgccaaac gacaaagagt 6060
   tactgctatt cttgacaagc tgataacaat gaccatcaat gaaaaacaga tgtacccatc 6120
   tattcaagca aaaatatggg gaagccttgg gcagattaca gatctgcttg atgttgtact 6180
   agacagtttc atcaaaacca gtgcaacagg tggcttggga tcaataaaag ctgaggtgat 6240
   ggcagatact gctgtagctt tggcttctgg aaatgtgaaa ttggtttcaa gcaaggttat 6300
   tggaaggatg tgcaaaataa ttgacaagac atgcttatct ccaactccta ctttagaaca 6360
   acatcttatg tgggatgata ttgctatttt agcacgctac atgctgatgc tgtccttcaa 6420
   caattccctt gatgtggcag ctcatcttcc ctacctcttc cacgttgtta ctttcttagt 6480
   agccacaggt ccgctctccc ttagagcttc cacacatgga ctggtcatta atatcattca 6540
   ctctctgtgt acttgttcac agcttcattt tagtgaagag accaagcaag ttttgagact 6600
   cagtctgaca gagttctcat tacccaaatt ttacttgctg tttggcatta gcaaagtcaa 6660
   gtcagctgct gtcattgcct tccgttccag ttaccgggac aggtcattct ctcctggctc 6720
   ctatgagaga gagacttttg ctttgacatc cttggaaaca gtcacagaag ctttgttgga 6780
   gatcatggag gcatgcatga gagatattcc aacgtgcaag tggctggacc agtggacaga 6840
   actagctcaa agatttgcat tccaatataa tccatccctg caaccaagag ctcttgttgt 6900
   ctttgggtgt attagcaaac gagtgtctca tgggcagata aagcagataa tccgtattct 6960
   tagcaaggca cttgagagtt gcttaaaagg acctgacact tacaacagtc aagttctgat 7020
   agaagctaca gtaatagcac taaccaaatt acagccactt cttaataagg actcgcctct 7080
   gcacaaagcc ctcttttggg tagctgtggc tgtgctgcag cttgatgagg tcaacttgta 7140
   ttcagcaggt accgcacttc ttgaacaaaa cctgcatact ttagatagtc tccgtatatt 7200
   caatgacaag agtccagagg aagtatttat ggcaatccgg aatcctctgg agtggcactg 7260
   caagcaaatg gatcattttg ttggactcaa tttcaactct aactttaact ttgcattggt 7320
   tggacacctt ttaaaagggt acaggcatcc ttcacctgct attgttgcaa gaacagtcag 7380
   aattttacat acactactaa ctctggttaa caaacacaga aattgtgaca aatttgaagt 7440
   gaatacacag agcgtggcct acttagcagc tttacttaca gtgtctgaag aagttcgaag 7500
   tcgctgcagc ctaaaacata gaaagtcact tcttcttact gatatttcaa tggaaaatgt 7560
   tcctatggat acatatccca ttcatcatgg tgacccttcc tataggacac taaaggagac 7620
   tcagccatgg tcctctccca aaggttctga aggatacctt gcagccacct atccaactgt 7680
   cggccagacc agtccccgag ccaggaaatc catgagcctg gacatggggc aaccttctca 7740
   ggccaacact aagaagttgc ttggaacaag gaaaagtttt gatcacttga tatcagacac 7800
   aaaggctcct aaaaggcaag aaatggaatc agggatcaca acacccccca aaatgaggag 7860
   agtagcagaa actgattatg aaatggaaac tcagaggatt tcctcatcac aacagcaccc 7920
   acatttacgt aaagtttcag tgtctgaatc aaatgttctc ttggatgaag aagtacttac 7980
   tgatccgaag atccaggcgc tgcttcttac tgttctagct acactggtaa aatataccac 8040
   agatgagttt gatcaacgaa ttctttatga atacttagca gaggccagtg ttgtgtttcc 8100
   caaagtcttt cctgttgtgc ataatttgtt ggactctaag atcaacaccc tgttatcatt 8160
   gtgccaagat ccaaatttgt taaatccaat ccatggaatt gtgcagagtg tggtgtacca 8220
   tgaagaatcc ccaccacaat accaaacatc ttacctgcaa agttttggtt ttaatggctt 8280
   gtggcggttt gcaggaccgt tttcaaagca aacacaaatt ccagactatg ctgagcttat 8340
   tgttaagttt cttgatgcct tgattgacac gtacctgcct ggaattgatg aagaaaccag 8400
   tgaagaatcc ctcctgactc ccacatctcc ttaccctcct gcactgcaga gccagcttag 8460
   tatcactgcc aaccttaacc tttctaattc catgacctca cttgcaactt cccagcattc 8520
   cccaggaatc gacaaggaga acgttgaact ctcccctacc actggccact gtaacagtgg 8580
   acgaactcgc cacggatccg caagccaagt gcagaagcaa agaagcgctg gcagtttcaa 8640
   acgtaatagc attaagaaga tcgtgtgaag cttgcttgct ttctttttta aaatcaactt 8700
   aacatgggct cttcactagt gaccccttcc ctgtccttgc cctttccccc catgttgtaa 8760
   tgctgcactt cctgttttat aatgaaccca tccggtttgc catgttgcca gatgatcaac 8820
   tcttcgaagc cttgcctaaa tttaatgctg ccttttcttt aacttttttt cttctacttt 8880
   tggcgtgtat ctggtatatg taagtgttca gaacaactgc aaagaaagtg ggaggtcagg 8940
   aaacttttaa ctgagaaat 8959
<210> 41
   <211> 2257
   <212> DNA
   <213> Homo sapiens
<400> 41
   acggcagccg tcagggaccg tcccccaact cccctttccg ctcaggcagg gtcctcgcgg 60
   cccatgctgg ccgctgggga cccgcgcagc ccagaccgtt cccgggccgg ccagccggca 120
   ccatggtggc cctgaggcct gtgcagcaac tccagggggg ctaaagggct cagagtgcag 180
   gccgtggggc gcgagggtcc cgggcctgag ccccgcgcca tggccggggc catcgcttcc 240
   cgcatgagct tcagctctct caagaggaag caacccaaga cgttcaccgt gaggatcgtc 300
   accatggacg ccgagatgga gttcaattgc gagatgaagt ggaaagggaa ggacctcttt 360
   gatttggtgt gccggactct ggggctccga gaaacctggt tctttggact gcagtacaca 420
   atcaaggaca cagtggcctg gctcaaaatg gacaagaagg tactggatca tgatgtttca 480
   aaggaagaac cagtcacctt tcacttcttg gccaaatttt atcctgagaa tgctgaagag 540
   gagctggttc aggagatcac acaacattta ttcttcttac aggtaaagaa gcagatttta 600
   gatgaaaaga tctactgccc tcctgaggct tctgtgctcc tggcttctta cgccgtccag 660
   gccaagtatg gtgactacga ccccagtgtt cacaagcggg gattttggcc ccaagaggaa 720
   ttgcttccaa aaagggtaat aaatctgtat cagatgactc cggaaatgtg ggaggagaga 780
   attactgctt ggtacgcaga gcaccgaggc cgagccaggg atgaagctga aatggaatat 840
   ctgaagatag ctcaggacct ggagatgtac ggtgtgaact actttgcaat ccggaataaa 900
   aagggcacag agctgctgct tggagtggat gccctggggc ttcacattta tgaccctgag 960
   aacagactga cccccaagat ctccttcccg tggaatgaaa tccgaaacat ctcgtacagt 1020
   gacaaggagt ttactattaa accactggat aagaaaattg atgtcttcaa gtttaactcc 1080
   tcaaagcttc gtgttaataa gctgattctc cagctatgta tcgggaacca tgatctattt 1140
   atgaggagaa ggaaagccga ttctttggaa gttcagcaga tgaaagccca ggccagggag 1200
   gagaaggcta gaaagcagat ggagcggcag cgcctcgctc gagagaagca gatgagggag 1260
   gaggctgaac gcacgaggga tgagttggag aggaggctgc tgcagatgaa agaagaagca 1320
   acaatggcca acgaagcact gatgcggtct gaggagacag ctgacctgtt ggctgaaaag 1380
   gcccagatca ccgaggagga ggcaaaactt ctggcccaga aggccgcaga ggctgagcag 1440
   gaaatgcagc gcatcaaggc cacagcgatt cgcacggagg aggagaagcg cctgatggag 1500
   cagaaggtgc tggaagccga ggtgctggca ctgaagatgg ctgaggagtc agagaggagg 1560
   gccaaagagg cagatcagct gaagcaggac ctgcaggaag cacgcgaggc ggagcgaaga 1620
   gccaagcaga agctcctgga gattgccacc aagcccacgt acccgcccat gaacccaatt 1680
   ccagcaccgt tgcctcctga cataccaagc ttcaacctca ttggtgacag cctgtctttc 1740
   gacttcaaag atactgacat gaagcggctt tccatggaga tagagaaaga aaaagtggaa 1800
   tacatggaaa agagcaagca tctgcaggag cagctcaatg aactcaagac agaaatcgag 1860
   gccttgaaac tgaaagagag ggagacagct ctggatattc tgcacaatga gaactccgac 1920
   aggggtggca gcagcaagca caataccatt aaaaagctca ccttgcagag cgccaagtcc 1980
   cgagtggcct tctttgaaga gctctagcag gtgacccagc caccccagga cctgccactt 2040
   ctcctgctac cgggaccgcg ggatggacca gatatcaaga gagccatcca tagggagctg 2100
   gctgggggtt tccgtgggag ctccagaact ttccccagct gagtgaagag cccagcccct 2160
   cttatgtgca attgccttga actacgaccc tgtagagatt tctctcatgg cgttctagtt 2220
   ctctgacctg agtctttgtt ttaagaagta tttgtct 2257
<210> 42
   <211> 2969
   <212> DNA
   <213> Homo sapiens
<400> 42
   ccaggcagct ggggtaagga gttcaaggca gcgcccacac ccgggggctc tccgcaaccc 60
   gaccgcctgt ccgctccccc acttcccgcc ctccctccca cctactcatt cacccaccca 120
   cccacccaga gccgggacgg cagcccaggc gcccgggccc cgccgtctcc tcgccgcgat 180
   cctggacttc ctcttgctgc aggacccggc ttccacgtgt gtcccggagc cggcgtctca 240
   gcacacgctc cgctccgggc ctgggtgcct acagcagcca gagcagcagg gagtccggga 300
   cccgggcggc atctgggcca agttaggcgc cgccgaggcc agcgctgaac gtctccaggg 360
   ccggaggagc cgcggggcgt ccgggtctga gccgcagcaa atgggctccg acgtgcggga 420
   cctgaacgcg ctgctgcccg ccgtcccctc cctgggtggc ggcggcggct gtgccctgcc 480
   tgtgagcggc gcggcgcagt gggcgccggt gctggacttt gcgcccccgg gcgcttcggc 540
   ttacgggtcg ttgggcggcc ccgcgccgcc accggctccg ccgccacccc cgccgccgcc 600
   gcctcactcc ttcatcaaac aggagccgag ctggggcggc gcggagccgc acgaggagca 660
   gtgcctgagc gccttcactg tccacttttc cggccagttc actggcacag ccggagcctg 720
   tcgctacggg cccttcggtc ctcctccgcc cagccaggcg tcatccggcc aggccaggat 780
   gtttcctaac gcgccctacc tgcccagctg cctcgagagc cagcccgcta ttcgcaatca 840
   gggttacagc acggtcacct tcgacgggac gcccagctac ggtcacacgc cctcgcacca 900
   tgcggcgcag ttccccaacc actcattcaa gcatgaggat cccatgggcc agcagggctc 960
   gctgggtgag cagcagtact cggtgccgcc cccggtctat ggctgccaca cccccaccga 1020
   cagctgcacc ggcagccagg ctttgctgct gaggacgccc tacagcagtg acaatttata 1080
   ccaaatgaca tcccagcttg aatgcatgac ctggaatcag atgaacttag gagccacctt 1140
   aaagggccac agcacagggt acgagagcga taaccacaca acgcccatcc tctgcggagc 1200
   ccaatacaga atacacacgc acggtgtctt cagaggcatt caggatgtgc gacgtgtgcc 1260
   tggagtagcc ccgactcttg tacggtcggc atctgagacc agtgagaaac gccccttcat 1320
   gtgtgcttac ccaggctgca ataagagata ttttaagctg tcccacttac agatgcacag 1380
   caggaagcac actggtgaga aaccatacca gtgtgacttc aaggactgtg aacgaaggtt 1440
   ttctcgttca gaccagctca aaagacacca aaggagacat acaggtgtga aaccattcca 1500
   gtgtaaaact tgtcagcgaa agttctcccg gtccgaccac ctgaagaccc acaccaggac 1560
   tcatacaggt gaaaagccct tcagctgtcg gtggccaagt tgtcagaaaa agtttgcccg 1620
   gtcagatgaa ttagtccgcc atcacaacat gcatcagaga aacatgacca aactccagct 1680
   ggcgctttga ggggtctccc tcggggaccg ttcagtgtcc caggcagcac agtgtgtgaa 1740
   ctgctttcaa gtctgactct ccactcctcc tcactaaaaa ggaaacttca gttgatcttc 1800
   ttcatccaac ttccaagaca agataccggt gcttctggaa actaccaggt gtgcctggaa 1860
   gagttggtct ctgccctgcc tacttttagt tgactcacag gccctggaga agcagctaac 1920
   aatgtctggt tagttaaaag cccattgcca tttggtgtgg attttctact gtaagaagag 1980
   ccatagctga tcatgtcccc ctgacccttc ccttcttttt ttatgctcgt tttcgctggg 2040
   gatggaatta ttgtaccatt ttctatcatg gaatatttat aggccagggc atgtgtatgt 2100
   gtctgctaat gtaaactttg tcatggtttc catttactaa cagcaacagc aagaaataaa 2160
   tcagagagca aggcatcggg ggtgaatctt gtctaacatt cccgaggtca gccaggctgc 2220
   taacctggaa agcaggatgt agttctgcca ggcaactttt aaagctcatg catttcaagc 2280
   agctgaagaa aaaatcagaa ctaaccagta cctctgtata gaaatctaaa agaattttac 2340
   cattcagtta attcaatgtg aacactggca cactgctctt aagaaactat gaagatctga 2400
   gatttttttg tgtatgtttt tgactctttt gagtggtaat catatgtgtc tttatagatg 2460
   tacatacctc cttgcacaaa tggaggggaa ttcattttca tcactgggag tgtccttagt 2520
   gtataaaaac catgctggta tatggcttca agttgtaaaa atgaaagtga ctttaaaaga 2580
   aaatagggga tggtccagga tctccactga taagactgtt tttaagtaac ttaaggacct 2640
   ttgggtctac aagtatatgt gaaaaaaatg agacttactg ggtgaggaaa tccattgttt 2700
   aaagatggtc gtgtgtgtgt gtgtgtgtgt gtgtgtgtgt gtgttgtgtt gtgttttgtt 2760
   ttttaaggga gggaatttat tatttaccgt tgcttgaaat tactgtgtaa atatatgtct 2820
   gataatgatt tgctctttga caactaaaat taggactgta taagtactag atgcatcact 2880
   gggtgttgat cttacaagat attgatgata acacttaaaa ttgtaacctg catttttcac 2940
   tttgctctca attaaagtct attcaaaag 2969
<210> 43
   <211> 69
   <212> DNA
   <213> Artificial
<220>
   <223> synthetic DNA
<400> 43
   ggcctccata aagtaggaaa cactacacag ctccataaag taggaaacac tacattaatt 60
   aagcggtac 69
<210> 44
   <211> 61
   <212> DNA
   <213> Artificial
<220>
   <223> synthetic DNA
<400> 44
   cgcttaatta atgtagtgtt tcctacttta tggagctgtg tagtgtttcc tactttatgg 60
   a 61
<210> 45
   <211> 54
   <212> DNA
   <213> Artificial
<220>
   <223> synthetic DNA
<400> 45
   tccataaagt aggaaacact acaggactcc ataaagtagg aaacactaca gtac 54
<210> 46
   <211> 52
   <212> DNA
   <213> Artificial
<220>
   <223> synthetic DNA
<400> 46
   tgtagtgttt cctactttat ggagtcctgt agtgtttcct actttatgga at 52
<210> 47
   <211> 573
   <212> DNA
   <213> Adenovirus
<400> 47
   ttatggactg gaataaaccc tccacctaac tgtcaaattg tggaaaacac taatacaaat 60
   gatggcaaac ttactttagt attagtaaaa aacggagggc ttgttaatgg ctacgtgtct 120
   ctagttggtg tatcagacac tgtgaaccaa atgttcacac aaaagacagc aaacatccaa 180
   ttaagattat attttgactc ttctggaaat ctattaactg atgaatcaga cttaaaaatt 240
   ccacttaaaa ataaatcttc tacagcgacc agtgaaactg tagccagcag caaagccttt 300
   atgccaagta ctacagctta tcccttcaac accactacta gggatagtga aaactacatt 360
   catggaatat gttactacat gactagttat gatagaagtc tatttccctt gaacatttct 420
   ataatgctaa acagccgtat gatttcttcc aatgttgcct atgccataca atttgaatgg 480
   aatctaaatg caagtgaatc tccagaaagc aacatagcta cgctgaccac atcccccttt 540
   ttcttttctt acattacaga agacgacaac taa 573
<210> 48
   <211> 270
   <212> DNA
   <213> Adenovirus
<400> 48
   ggagttctta ctcttaagtg tttaacccca ctaacaacca caggcggatc tctacagcta        60
   aaagtgggag ggggacttac agtggatgac actgatggta ccttacaaga aaacatacgt       120
   gctacagcac ccattactaa aaataatcac tctgtagaac tatccattgg aaatggatta       180
   gaaactcaaa acaataaact atgtgccaaa ttgggaaatg ggttaaaatt taacaacggt       240
   gacatttgta taaaggatag tattaacacc                                       270
<210> 49
   <211> 843
   <212> DNA
   <213> Adenovirus
<400> 49
   ggagttctta ctcttaagtg tttaacccca ctaacaacca caggcggatc tctacagcta        60
   aaagtgggag ggggacttac agtggatgac actgatggta ccttacaaga aaacatacgt       120
   gctacagcac ccattactaa aaataatcac tctgtagaac tatccattgg aaatggatta       180
   gaaactcaaa acaataaact atgtgccaaa ttgggaaatg ggttaaaatt taacaacggt       240
   gacatttgta taaaggatag tattaacacc ttatggactg gaataaaccc tccacctaac       300
   tgtcaaattg tggaaaacac taatacaaat gatggcaaac ttactttagt attagtaaaa       360
   aacggagggc ttgttaatgg ctacgtgtct ctagttggtg tatcagacac tgtgaaccaa       420
   atgttcacac aaaagacagc aaacatccaa ttaagattat attttgactc ttctggaaat       480
   ctattaactg atgaatcaga cttaaaaatt ccacttaaaa ataaatcttc tacagcgacc       540
   agtgaaactg tagccagcag caaagccttt atgccaagta ctacagctta tcccttcaac       600
   accactacta gggatagtga aaactacatt catggaatat gttactacat gactagttat       660
   gatagaagtc tatttccctt gaacatttct ataatgctaa acagccgtat gatttcttcc       720
   aatgttgcct atgccataca atttgaatgg aatctaaatg caagtgaatc tccagaaagc       780
   aacatagcta cgctgaccac atcccccttt ttcttttctt acattacaga agacgacaac       840
   taa                                                                     843
<210> 50
   <211> 975
   <212> DNA
   <213> Artificial
<220>
   <223> synthetic DNA
<400> 50
   atgaagcgcg caagaccgtc tgaagatacc ttcaaccccg tgtatccata tgacacggaa        60
   accggtcctc caactgtgcc ttttcttact cctccctttg tatcccccaa tgggtttcaa       120
   gagagtcccc ctggagttct tactcttaag tgtttaaccc cactaacaac cacaggcgga       180
   tctctacagc taaaagtggg agggggactt acagtggatg acactgatgg taccttacaa       240
   gaaaacatac gtgctacagc acccattact aaaaataatc actctgtaga actatccatt       300
   ggaaatggat tagaaactca aaacaataaa ctatgtgcca aattgggaaa tgggttaaaa       360
   tttaacaacg gtgacatttg tataaaggat agtattaaca ccttatggac tggaataaac       420
   cctccaccta actgtcaaat tgtggaaaac actaatacaa atgatggcaa acttacttta       480
   gtattagtaa aaaacggagg gcttgttaat ggctacgtgt ctctagttgg tgtatcagac       540
   actgtgaacc aaatgttcac acaaaagaca gcaaacatcc aattaagatt atattttgac       600
   tcttctggaa atctattaac tgatgaatca gacttaaaaa ttccacttaa aaataaatct       660
   tctacagcga ccagtgaaac tgtagccagc agcaaagcct ttatgccaag tactacagct       720
   tatcccttca acaccactac tagggatagt gaaaactaca ttcatggaat atgttactac       780
   atgactagtt atgatagaag tctatttccc ttgaacattt ctataatgct aaacagccgt       840
   atgatttctt ccaatgttgc ctatgccata caatttgaat ggaatctaaa tgcaagtgaa     900
   tctccagaaa gcaacatagc tacgctgacc acatccccct ttttcttttc ttacattaca     960
   gaagacgaca actaa                                                        975